# EUROPEAN PATENT SPECIFICATION

(11) **EP 2 951 305 B1**
(45) Date of publication and mention of the grant of the patent: **15.08.2018**
(21) Application number: 14704307.9
(22) Date of filing: 30.01.2014
(51) Int. Cl.: C12N 15/113, A61K 31/713, A61K 47/55

(54) **LNA OLIGONUCLEOTIDE CARBOHYDRATE CONJUGATES**
LNA-OLIGONUKLEOTID-KOHLENHYDRATKONJUGATE
CONJUGUÉS GLUCIDIQUES D'OLIGONUCLÉOTIDES D'ACIDES NUCLÉIQUES BLOQUÉS

(30) Priority: 30.01.2013 EP 13153296; 28.02.2013 EP 13157237; 27.06.2013 EP 13174092; 14.11.2013 EP 13192930; 14.11.2013 WO PCT/EP2013/073858; 14.11.2013 WO PCT/EP2013/073859; 14.11.2013 EP 13192938
(43) Date of publication of application: 09.12.2015
(73) Proprietor: F. Hoffmann-La Roche AG, 4070 Basel (CH)
(72) Inventor: ALBÆK, Nanna, 3460 Birkeroed (DK); HANSEN, Henrik Frydenlund, 4100 Ringsted (DK); KAMMLER, Susanne, 2840 Holte (DK); RAVN, Jacob, 2740 Skovlunde (DK); ØRUM, Henrik, 3500 Værløse (DK); TURNER, Mark, 2970 Hørsholm (DK); KRAMPERT, Monika, 95326 Kulmbach (DE); HADWIGER, Philipp, 95326 Kulmbach (DE); OTTOSEN, Søren, 2600 Glostrup (DK); LINDOW, Morten, 2450 Copenhagen SV (DK)
(74) Representative: Hansen, Marianne Rudolph
(86) International application number: PCT/EP2014/051781
(87) International publication number: WO 2014/118267

(56) References cited:
- WO-A1-2006/078217
- WO-A1-2010/076248
- WO-A1-2011/047312
- WO-A1-2014/076195
- WO-A2-01/25248
- WO-A2-2008/132234
- WO-A2-2009/043353
- WO-A2-2009/068033
- WO-A2-2009/073809
- WO-A2-2012/083046
- US-A1- 2012 122 801
- MAIER M A ET AL: "Synthesis of antisense oligonucleotides conjugated to a multivalent carbohydrate cluster for cellular targeting", BIOCONJUGATE CHEMISTRY, ACS, WASHINGTON, DC, US, vol. 14, 1 January 2003 (2003-01-01), pages 18-29, XP002510288, ISSN: 1043-1802, DOI: 10.1021/BC020028V [retrieved on 2002-12-03]
- LIN ZHU ET AL: "Site-Specific Delivery of Oligonucleotides to Hepatocytes after Systemic Administration", BIOCONJUGATE CHEMISTRY, vol. 19, no. 1, 1 January 2008 (2008-01-01), pages 290-298, XP055101407, ISSN: 1043-1802, DOI: 10.1021/bc070126m
- ERIK A.L. BIESSEN ET AL: "Targeted delivery of oligodeoxynucleotides to parenchymal liver cells in vivo", BIOCHEMICAL JOURNAL, vol. 340, no. 3, 15 June 1999 (1999-06-15) , pages 783-792, XP055101395, ISSN: 0264-6021, DOI: 10.1042/0264-6021:3400783
- S. B. RAJUR ET AL: "Covalent Protein-Oligonucleotide Conjugates for Efficient Delivery of Antisense Molecules", BIOCONJUGATE CHEMISTRY, vol. 8, no. 6, 1 November 1997 (1997-11-01), pages 935-940, XP055095373, ISSN: 1043-1802, DOI: 10.1021/bc970172u
- ZHENG SU-JUN ET AL: "Distribution and anti-HBV effects of antisense oligodeoxynucleotides conjugated to galactosylated poly-L-lysine", WORLD JOURNAL OF GASTROENTEROLOGY, WJG PRESS, CN, [Online] vol. 9, no. 6, 1 January 2003 (2003-01-01) , pages 1251-1255, XP002510287, ISSN: 1007-9327
- MAKINO NAOKI ET AL: "Intravenous injection with antisense oligodeoxynucleotides against angiotensin decreases blood pressure in spontaneously hypertensive rats", 19980501; 19980500, vol. 31, no. 5, 1 May 1998 (1998-05-01), pages 1166-1170, XP002336133,
- T. P. PRAKASH ET AL: "Targeted delivery of antisense oligonucleotides to hepatocytes using triantennary N-acetyl galactosamine improves potency 10-fold in mice", NUCLEIC ACIDS RESEARCH, vol. 42, no. 13, 3 July 2014 (2014-07-03), pages 8796-8807, XP055148318, ISSN: 0305-1048, DOI: 10.1093/nar/gku531
- E. Koller ET AL: "Mechanisms of single-stranded phosphorothioate modified antisense oligonucleotide accumulation in hepatocytes", Nucleic Acids Research, vol. 39, no. 11, 23 February 2011 (2011-02-23), pages 4795-4807, XP055154734, ISSN: 0305-1048, DOI: 10.1093/nar/gkr089
- SEHGAL ALFICA ET AL: "Liver as a target for oligonucleotide therapeutics", JOURNAL OF HEPATOLOGY, vol. 59, no. 6, 12 June 2013 (2013-06-12), pages 1354-1359, XP028775443, ISSN: 0168-8278, DOI: 10.1016/J.JHEP.2013.05.045
- MANOHARAN MUTHIAH: "Oligonucleotide conjugates as potential antisense drugs with improved uptake, biodistribution, targeted delivery, and mechanisms of action", ANTISENSE & NUCLEIC ACID DRUG DEVELOPMENT, MARY ANN LIEBERT, INC., NEW YORK, US, vol. 12, no. 2, 1 April 2002 (2002-04-01), pages 103-128, XP002557015, ISSN: 1087-2906, DOI: 10.1089/108729002760070849 [retrieved on 2004-06-08]
- YU BO ET AL: "Targeted Delivery Systems for Oligonucleotide Therapeutics", THE AAPS JOURNAL, SPRINGER US, BOSTON, vol. 11, no. 1, 19 March 2009 (2009-03-19) , pages 195-203, XP035719012, DOI: 10.1208/S12248-009-9096-1 [retrieved on 2009-03-19]
- R. L. Juliano ET AL: "Receptors, endocytosis, and trafficking: the biological basis of targeted delivery of antisense and siRNA oligonucleotides", Journal of Drug Targeting, vol. 21, no. 1, 19 November 2012 (2012-11-19), pages 27-43, XP055340424, ISSN: 1061-186X, DOI: 10.3109/1061186X.2012.740674
- MCKEE T D ET AL: "PREPARATION OF ASIALOOROSOMUCOID-POLYLYSINE CONJUGATES", BIOCONJUGATE CHEMISTRY,, vol. 5, no. 4, 1 July 1994 (1994-07-01), pages 306-311, XP000455280, ISSN: 1043-1802, DOI: 10.1021/BC00028A004
- BUNNELL B A ET AL: "TARGETED DELIVERY OF ANTISENSE OLIGONUCLEOTIDES BY MOLECULAR CONJUGATES", SOMATIC CELL AND MOLECULAR GENE, NEW YORK, NY, US, vol. 18, no. 6, 1 December 1992 (1992-12-01), pages 559-569, XP009000683, DOI: 10.1007/BF01232652

### SEQUENCE LISTING

<110> Santaris Pharma A/S
<120> LNA OLIGONUCLEOTIDE CARBOHYDRATE CONJUGATES
<130> 1139WO
<150> EP13153296.2
   <151> 2013-01-30
<150> EP13157237.2
   <151> 2013-02-28
<150> EP13174092.0
   <151> 2013-06-27
<150> EP13192938.2
   <151> 2013-11-14
<150> EP13192931.7
   <151> 2013-11-14
<150> EP13192930.9
   <151> 2013-11-14
<150> PCT/EP2013/073859
   <151> 2013-11-14
<160> 62
<170> PatentIn version 3.5
<210> 1
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(13)
   <223> n is a, c, g, or t
<220>
   <221> misc_feature
   <222> (11)..(13)
   <223> LNA nucleosides, LNA C are 5-methyl C
<400> 1
   nnnnnnnnnn nnn 13
<210> 2
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(13)
   <223> n is a, c, g, or t
<220>
   <221> misc_feature
   <222> (1)..(1)
   <223> 5 C6 amino linkage group
<220>
   <221> misc_feature
   <222> (11)..(13)
   <223> LNA nucleosides, LNA C are 5-methyl C
<400> 2
   nnnnnnnnnn nnn 13
<210> 3
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(13)
   <223> n is a, c, g, or t
<220>
   <221> misc_feature
   <222> (1)..(1)
   <223> 5 GalNac cluster
<220>
   <221> misc_feature
   <222> (11)..(13)
   <223> LNA nucleosides, LNA C are 5-methyl C
<400> 3
   nnnnnnnnnn nnn 13
<210> 4
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(13)
   <223> n is a, c, g, or t
<220>
   <221> misc_feature
   <222> (1)..(1)
   <223> 5 disulphide linked cholesterol conjugate
<220>
   <221> misc_feature
   <222> (11)..(13)
   <223> LNA nucleosides, LNA C are 5-methyl C
<400> 4
   nnnnnnnnnn nnn 13
<210> 5
   <211> 13
   <212> DNA
   <213> artificial
<220>
   <223> LNA antisense gapmer oligonucleotide
<220>
   <221> misc_feature
   <222> (1)..(10)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(13)
   <223> n is a, c, g, or t
<220>
   <221> misc_feature
   <222> (1)..(1)
   <223> disulphide liked cholesterol conjugate
<220>
   <221> misc_feature
   <222> (11)..(13)
   <223> LNA nucleosides, LNA C are 5-methyl C
<400> 5
   nnnnnnnnnn nnn 13
<210> 6
   <211> 15
   <212> DNA
   <213> artificial
<220>
   <223> LNA antisense gapmer oligonucleotide
<220>
   <221> misc_feature
   <222> (1)..(15)
   <223> phosphorothioate lnternucleoside linkages
<220>
   <221> misc_feature
   <222> (1)..(1)
   <223> 5 3xGalNac cluster Conj1a
<220>
   <221> misc_feature
   <222> (3)..(5)
   <223> LNA nucleosides, LNA C are 5-methyl C
<220>
   <221> misc_feature
   <222> (3)..(15)
   <223> n is a, c, g, or t
<220>
   <221> misc_feature
   <222> (13)..(15)
   <223> LNA nucleosides, LNA C are 5-methyl C
<400> 6
   cannnnnnnn nnnnn 15
<210> 7
   <211> 15
   <212> DNA
   <213> artificial
<220>
   <223> LNA antisense gapmer oligonucleotide
<220>
   <221> misc_feature
   <222> (1)..(15)
   <223> phosphorothioate lnternucleoside linkages
<220>
   <221> misc_feature
   <222> (1)..(1)
   <223> cholesterol conjugate
<220>
   <221> misc_feature
   <222> (3)..(5)
   <223> LNA nucleosides, LNA C are 5-methyl C
<220>
   <221> misc_feature
   <222> (3)..(15)
   <223> n is a, c, g, or t
<220>
   <221> misc_feature
   <222> (13)..(15)
   <223> LNA nucleosides, LNA C are 5-methyl C
<400> 7
   cannnnnnnn nnnnn 15
<210> 8
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 monoGalNAc
<220>
   <221> misc_feature
   <222> (11)..(13)
   <223> LNA nucleosides, LNA C are 5-methyl C
<400> 8
   gcattggtat tca 13
<210> 9
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 disulphide linked monoGalNAc
<220>
   <221> misc_feature
   <222> (11)..(13)
   <223> LNA nucleosides, LNA C are 5-methyl C
<400> 9
   gcattggtat tca 13
<210> 10
   <211> 15
   <212> DNA
   <213> artificial
<220>
   <223> LNA antisense gapmer oligonucleotide conjugate
<220>
   <221> misc_feature
   <222> (1)..(3)
   <223> phosphodiester lnternucleoside linkages
<220>
   <221> misc_feature
   <222> (1)..(1)
   <223> 5 GalNAc conjugate
<220>
   <221> misc_feature
   <222> (3)..(15)
   <223> phosphorothioate lnternucleoside linkages
<220>
   <221> misc_feature
   <222> (3)..(4)
   <223> LNA nucleosides, LNA C are 5-methyl C
<220>
   <221> misc_feature
   <222> (13)..(15)
   <223> LNA nucleosides, LNA C are 5-methyl C
<400> 10
   cagcattggt attca 15
<210> 11
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 3xGalNAc cluster Conj2a
<220>
   <221> misc_feature
   <222> (11)..(13)
   <223> LNA nucleosides, LNA C are 5-methyl C
<400> 11
   gcattggtat tca 13
<210> 12
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (11)..(13)
   <223> LNA nucleosides, LNA C are 5-methyl C
<400> 12
   gcattggtat tca 13
<210> 13
   <211> 13
   <212> DNA
   <213> artificial
<220>
   <223> LNA antisense gapmer oligonucleotide
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 3xGalNAc cluster Conj1a
<220>
   <221> misc_feature
   <222> (11)..(13)
   <223> LNA nucleosides, LNA C are 5-methyl C
<400> 13
   gcattggtat tca 13
<210> 14
   <211> 15
   <212> DNA
   <213> artificial
<220>
   <223> LNA antisense gapmer oligonucleotide
<220>
   <221> misc_feature
   <222> (1)..(3)
   <223> phosphodiester lnternucleoside linkages
<220>
   <221> misc_feature
   <222> (1)..(1)
   <223> 5 cholesterol conjugate
<220>
   <221> misc_feature
   <222> (3)..(15)
   <223> phosphorothioate lnternucleoside linkages
<220>
   <221> misc_feature
   <222> (3)..(4)
   <223> LNA nucleosides, LNA C are 5-methyl C
<220>
   <221> misc_feature
   <222> (13)..(15)
   <223> LNA nucleosides, LNA C are 5-methyl C
<400> 14
   cagcattggt attca 15
<210> 15
   <211> 16
   <212> DNA
   <213> artificial
<220>
   <223> LNA antisense gapmer oligonucleotide
<220>
   <221> misc_feature
   <222> (1)..(16)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (1)..(1)
   <223> 5 3xGalNAc cluster Conj2a
<220>
   <221> misc_feature
   <222> (14)..(16)
   <223> LNA nucleosides, LNA C are 5-methyl C
<400> 15
   aatgctacaa aaccca 16
<210> 16
   <211> 16
   <212> DNA
   <213> artificial
<220>
   <223> LNA antisense gapmer oligonucleotide
<220>
   <221> misc_feature
   <222> (1)..(16)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 3xGalNAc cluster Conj2a
<220>
   <221> misc_feature
   <222> (14)..(16)
   <223> LNA nucleosides, LNA C are 5-methyl C
<400> 16
   tgctgtgtga gcttgg 16
<210> 17
   <211> 12
   <212> DNA
   <213> artificial
<220>
   <223> LNA antisense gapmer oligonucleotide
<220>
   <221> misc_feature
   <222> (1)..(12)
   <223> phosphorothioate lnternucleoside linkages
<220>
   <221> misc_feature
   <222> (1)..(2)
   <223> LNA nucleosides
<220>
   <221> misc_feature
   <222> (1)..(1)
   <223> 5 3xGalNAc cluster Conj2a
<220>
   <221> misc_feature
   <222> (11)..(12)
   <223> LNA nucleosides, LNA C are 5-methyl C
<400> 17
   gttgacactg tc 12
<210> 18
   <211> 14
   <212> DNA
   <213> artificial
<220>
   <223> LNA antisense gapmer oligonucleotide conjugate
<220>
   <221> misc_feature
   <222> (1)..(2)
   <223> phosphodiester lnternucleoside linkages
<220>
   <221> misc_feature
   <222> (1)..(1)
   <223> 5 FAM conjugate
<220>
   <221> misc_feature
   <222> (2)..(14)
   <223> phosphorothioate lnternucleoside linkages
<220>
   <221> misc_feature
   <222> (2)..(3)
   <223> LNA nucleosides, LNA C are 5-methyl C
<220>
   <221> misc_feature
   <222> (12)..(14)
   <223> LNA nucleosides, LNA C are 5-methyl C
<400> 18
   agcattggta ttca 14
<210> 19
   <211> 16
   <212> DNA
   <213> artificial
<220>
   <223> LNA antisense gapmer oligonucleotide conjugate
<220>
   <221> misc_feature
   <222> (1)..(4)
   <223> phosphodiester lnternucleoside linkages
<220>
   <221> misc_feature
   <222> (1)..(1)
   <223> 5 FAM conjugate
<220>
   <221> misc_feature
   <222> (4)..(16)
   <223> phosphorothioate lnternucleoside linkages
<220>
   <221> misc_feature
   <222> (4)..(5)
   <223> LNA nucleosides, LNA C are 5-methyl C
<220>
   <221> misc_feature
   <222> (14)..(16)
   <223> LNA nucleosides, LNA C are 5-methyl C
<400> 19
   gacgcattgg tattca 16
<210> 20
   <211> 13
   <212> DNA
   <213> artificial
<220>
   <223> LNA antisense gapmer oligonucleotide conjugate
<220>
   <221> misc_feature
   <222> (1)..(13)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 FAM conjugate
<220>
   <221> misc_feature
   <222> (11)..(13)
   <223> LNA nucleosides, LNA C are 5-methyl C
<400> 20
   gcattggtat tca 13
<210> 21
   <211> 16
   <212> DNA
   <213> artificial
<220>
   <223> LNA antisense gapmer oligonucleotide conjugate
<220>
   <221> misc_feature
   <222> (1)..(4)
   <223> phosphodiester lnternucleoside linkages
<220>
   <221> misc_feature
   <222> (1)..(1)
   <223> 5 FAM conjugate
<220>
   <221> misc_feature
   <222> (4)..(16)
   <223> phosphorothioate lnternucleoside linkages
<220>
   <221> misc_feature
   <222> (4)..(5)
   <223> LNA nucleosides, LNA C are 5-methyl C
<220>
   <221> misc_feature
   <222> (14)..(16)
   <223> LNA nucleosides, LNA C are 5-methyl C
<400> 21
   tcagcattgg tattca 16
<210> 22
   <211> 14
   <212> DNA
   <213> artificial
<220>
   <223> LNA antisense gapmer oligonucleotide conjugate
<220>
   <221> misc_feature
   <222> (1)..(14)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (12)..(14)
   <223> LNA nucleosides, LNA C are 5-methyl C
<400> 22
   tgctacaaaa ccca 14
<210> 23
   <211> 16
   <212> DNA
   <213> artificial
<220>
   <223> LNA antisense gapmer oligonucleotide conjugate
<220>
   <221> misc_feature
   <222> (1)..(16)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (13)..(16)
   <223> LNA nucleosides, LNA C are 5-methyl C
<400> 23
   aatgctacaa aaccca 16
<210> 24
   <211> 16
   <212> DNA
   <213> artificial
<220>
   <223> LNA antisense gapmer oligonucleotide conjugate
<220>
   <221> misc_feature
   <222> (1)..(16)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (14)..(16)
   <223> LNA nucleosides, LNA C are 5-methyl C
<400> 24
   aatgctacaa aaccca 16
<210> 25
   <211> 15
   <212> DNA
   <213> artificial
<220>
   <223> LNA antisense gapmer oligonucleotide conjugate
<220>
   <221> misc_feature
   <222> (1)..(15)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (14)..(15)
   <223> LNA nucleosides, LNA C are 5-methyl C
<400> 25
   gctgtgtgag cttgg 15
<210> 26
   <211> 16
   <212> DNA
   <213> artificial
<220>
   <223> LNA antisense gapmer oligonucleotide conjugate
<220>
   <221> misc_feature
   <222> (1)..(16)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (14)..(16)
   <223> LNA nucleosides, LNA C are 5-methyl C
<400> 26
   tgctgtgtga gcttgg 16
<210> 27
   <211> 14
   <212> DNA
   <213> artificial
<220>
   <223> LNA antisense gapmer oligonucleotide conjugate
<220>
   <221> misc_feature
   <222> (1)..(14)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (1)..(1)
   <223> 5 3xGalNAc cluster Conj2a
<220>
   <221> misc_feature
   <222> (12)..(14)
   <223> LNA nucleosides, LNA C are 5-methyl C
<400> 27
   tgctacaaaa ccca 14
<210> 28
   <211> 16
   <212> DNA
   <213> artificial
<220>
   <223> LNA antisense gapmer oligonucleotide conjugate
<220>
   <221> misc_feature
   <222> (1)..(16)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (1)..(1)
   <223> 5 3xGalNAc cluster Conj2a
<220>
   <221> misc_feature
   <222> (13)..(16)
   <223> LNA nucleosides, LNA C are 5-methyl C
<400> 28
   aatgctacaa aaccca 16
<210> 29
   <211> 15
   <212> DNA
   <213> artificial
<220>
   <223> LNA antisense gapmer oligonucleotide conjugate
<220>
   <221> misc_feature
   <222> (1)..(15)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 3xGalNAc cluster Conj2a
<220>
   <221> misc_feature
   <222> (14)..(15)
   <223> LNA nucleosides, LNA C are 5-methyl C
<400> 29
   gctgtgtgag cttgg 15
<210> 30
   <211> 18
   <212> DNA
   <213> artificial
<220>
   <223> LNA antisense gapmer oligonucleotide conjugate
<220>
   <221> misc_feature
   <222> (1)..(1)
   <223> 5 cholesterol conjugate
<220>
   <221> misc_feature
   <222> (3)..(18)
   <223> phosphorothioate lnternucleoside linkages
<220>
   <221> misc_feature
   <222> (3)..(5)
   <223> LNA nucleosides, LNA C are 5-methyl C
<220>
   <221> misc_feature
   <222> (15)..(18)
   <223> LNA nucleosides, LNA C are 5-methyl C
<400> 30
   caaatgctac aaaaccca 18
<210> 31
   <211> 18
   <212> DNA
   <213> artificial
<220>
   <223> LNA antisense gapmer oligonucleotide conjugate
<220>
   <221> misc_feature
   <222> (1)..(1)
   <223> 5 cholesterol conjugate
<220>
   <221> misc_feature
   <222> (3)..(18)
   <223> phosphorothioate lnternucleoside linkages
<220>
   <221> misc_feature
   <222> (3)..(4)
   <223> LNA nucleosides, LNA C are 5-methyl C
<220>
   <221> misc_feature
   <222> (16)..(18)
   <223> LNA nucleosides, LNA C are 5-methyl C
<400> 31
   catgctgtgt gagcttgg 18
<210> 32
   <211> 14
   <212> DNA
   <213> artificial
<220>
   <223> LNA antisense gapmer oligonucleotide conjugate
<220>
   <221> misc_feature
   <222> (1)..(1)
   <223> 5 cholesterol conjugate
<220>
   <221> misc_feature
   <222> (3)..(14)
   <223> phosphorothioate lnternucleoside linkages
<220>
   <221> misc_feature
   <222> (3)..(4)
   <223> LNA nucleosides, LNA C are 5-methyl C
<220>
   <221> misc_feature
   <222> (13)..(14)
   <223> LNA nucleosides, LNA C are 5-methyl C
<400> 32
   cagttgacac tgtc 14
<210> 33
   <211> 9
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA nucleosides, LNA C optionally 5-methyl C
<400> 33
   tcacactcc 9
<210> 34
   <211> 23
   <212> RNA
   <213> H.sapiens
<220>
   <221> misc_feature
   <223> Mature microRNA-122
<400> 34
   uggaguguga caaugguguu ugu 23
<210> 35
   <211> 9
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA nucleosides, LNA C optionally 5-methyl C
<400> 35
   tacaatgca 9
<210> 36
   <211> 9
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA nucleosides, LNA C optionally 5-methyl C
<400> 36
   acaatgcac 9
<210> 37
   <211> 8
   <212> DNA
   <213> artificial
<220>
   <223> LNA antisense oligonucleotide conjugate
<220>
   <221> misc_feature
   <222> (1)..(8)
   <223> phosphorothioate lnternucleoside linkages
<220>
   <221> misc_feature
   <222> (1)..(8)
   <223> LNA nucleosides, LNA C optionally 5-methyl C
<400> 37
   acaatgca 8
<210> 38
   <211> 7
   <212> DNA
   <213> artificial
<220>
   <223> LNA antisense oligonucleotide conjugate
<220>
   <221> misc_feature
   <222> (1)..(7)
   <223> phosphorothioate lnternucleoside linkages
<220>
   <221> misc_feature
   <222> (1)..(7)
   <223> LNA nucleosides, LNA C optionally 5-methyl C
<400> 38
   caatgca 7
<210> 39
   <211> 21
   <212> RNA
   <213> H.sapiens
<220>
   <221> misc_feature
   <223> Mature microRNA-33a
<400> 39
   gugcauugua guugcauugc a 21
<210> 40
   <211> 20
   <212> RNA
   <213> H.sapiens
<220>
   <221> misc_feature
   <223> Mature microRNA-33a
<400> 40
   gugcauugcu guugcauugc 20
<210> 41
   <211> 9
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA nucleosides, LNA C optionally 5-methyl C
<400> 41
   tgataagct 9
<210> 42
   <211> 8
   <212> DNA
   <213> artificial
<220>
   <223> LNA antisense oligonucleotide conjugate
<220>
   <221> misc_feature
   <222> (1)..(8)
   <223> phosphorothioate lnternucleoside linkages
<220>
   <221> misc_feature
   <222> (1)..(8)
   <223> LNA nucleosides, LNA C optionally 5-methyl C
<400> 42
   gataagct 8
<210> 43
   <211> 7
   <212> DNA
   <213> artificial
<220>
   <223> LNA antisense oligonucleotide conjugate
<220>
   <221> misc_feature
   <222> (1)..(7)
   <223> phosphorothioate lnternucleoside linkages
<220>
   <221> misc_feature
   <222> (1)..(7)
   <223> LNA nucleosides, LNA C optionally 5-methyl C
<400> 43
   ataagct 7
<210> 44
   <211> 15
   <212> DNA
   <213> artificial
<220>
   <223> LNA antisense oligonucleotide conjugate
<220>
   <221> misc_feature
   <222> (1)..(15)
   <223> phosphorothioate lnternucleoside linkages
<220>
   <221> misc_feature
   <222> (1)..(15)
   <223> LNA nucleosides at positions 1, 3, 4, 6, 7, 8, 10, 12, 14, 15 & LNA C optionally 5-methyl C
<400> 44
   tcagtctgat aagct 15
<210> 45
   <211> 22
   <212> RNA
   <213> H.sapiens
<220>
   <221> misc_feature
   <223> Mature microRNA-21
<400> 45
   uagcuuauca gacugauguu ga 22
<210> 46
   <211> 9
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA nucleosides, LNA C optionally 5-methyl C
<400> 46
   caatgtagc 9
<210> 47
   <211> 8
   <212> DNA
   <213> artificial
<220>
   <223> LNA antisense oligonucleotide conjugate
<220>
   <221> misc_feature
   <222> (1)..(8)
   <223> phosphorothioate lnternucleoside linkages
<220>
   <221> misc_feature
   <222> (1)..(8)
   <223> LNA nucleosides, LNA C optionally 5-methyl C
<400> 47
   aatgtagc 8
<210> 48
   <211> 7
   <212> DNA
   <213> artificial
<220>
   <223> LNA antisense oligonucleotide conjugate
<220>
   <221> misc_feature
   <222> (1)..(7)
   <223> phosphorothioate lnternucleoside linkages
<220>
   <221> misc_feature
   <222> (1)..(7)
   <223> LNA nucleosides, LNA C optionally 5-methyl C
<400> 48
   atgtagc 7
<210> 49
   <211> 23
   <212> RNA
   <213> H.sapiens
<220>
   <221> misc_feature
   <223> Mature microRNA-221
<400> 49
   agcuacauug ucugcugggu uuc 23
<210> 50
   <211> 12
   <212> DNA
   <213> artificial
<220>
   <223> LNA antisense gapmer oligonucleotide
<220>
   <221> misc_feature
   <222> (1)..(12)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (11)..(12)
   <223> LNA nucleosides, LNA C are 5-methyl C
<400> 50
   gttgacactg tc 12
<210> 51
   <211> 8
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA beta-D-oxy-nucleosides, LNA C are 5-methyl C
<400> 51
   cacactcc 8
<210> 52
   <211> 8
   <212> DNA
   <213> artificial
<220>
   <223> LNA antisense oligonucleotide conjugate
<220>
   <221> misc_feature
   <222> (1)..(9)
   <223> phosphorothioate lnternucleoside linkages
<220>
   <221> misc_feature
   <222> (1)..(9)
   <223> LNA beta-D-oxy-nucleosides, LNA C are 5-methyl C
<220>
   <221> misc_feature
   <222> (1)..(1)
   <223> 5 3xGalNAc conjugate, e.g Conj1a or conj2a.
<400> 52
   cacactcc 8
<210> 53
   <211> 18
   <212> DNA
   <213> artificial
<220>
   <223> LNA antisense oligonucleotide conjugate
<220>
   <221> misc_feature
   <222> (1)..(8)
   <223> phosphorothioate lnternucleoside linkages
<220>
   <221> misc_feature
   <222> (1)..(9)
   <223> LNA beta-D-oxy-nucleosides, LNA C are 5-methyl C
<220>
   <221> misc_feature
   <222> (11)..(18)
   <223> phosphorothioate lnternucleoside linkages
<220>
   <221> misc_feature
   <222> (11)..(18)
   <223> LNA beta-D-oxy-nucleosides, LNA C are 5-methyl C
<400> 53
   cacactccca cacactcc 18
<210> 54
   <211> 18
   <212> DNA
   <213> artificial
<220>
   <223> LNA antisense oligonucleotide conjugate
<220>
   <221> misc_feature
   <222> (1)..(8)
   <223> phosphorothioate lnternucleoside linkages
<220>
   <221> misc_feature
   <222> (1)..(9)
   <223> LNA beta-D-oxy-nucleosides, LNA C are 5-methyl C
<220>
   <221> misc_feature
   <222> (1)..(1)
   <223> 5 3xGalNAc conjugate, e.g Conj1a or conj2a.
<220>
   <221> misc_feature
   <222> (11)..(18)
   <223> phosphorothioate lnternucleoside linkages
<220>
   <221> misc_feature
   <222> (11)..(18)
   <223> LNA beta-D-oxy-nucleosides, LNA C are 5-methyl C
<400> 54
   cacactccca cacactcc 18
<210> 55
   <211> 16
   <212> DNA
   <213> artificial
<220>
   <223> LNA antisense gapmer oligonucleotide conjugate
<220>
   <221> misc_feature
   <222> (1)..(16)
   <223> phosphorothioate lnternucleoside linkages
<220>
   <221> misc_feature
   <222> (1)..(4)
   <223> LNA nucleosides, LNA C are 5-methyl C
<220>
   <221> misc_feature
   <222> (13)..(16)
   <223> LNA nucleosides, LNA C are 5-methyl C
<400> 55
   gaggcatagc agcagg 16
<210> 56
   <211> 16
   <212> DNA
   <213> artificial
<220>
   <223> LNA antisense gapmer oligonucleotide conjugate
<220>
   <221> misc_feature
   <222> (1)..(16)
   <223> phosphorothioate lnternucleoside linkages
<220>
   <221> misc_feature
   <222> (1)..(4)
   <223> LNA nucleosides, LNA C are 5-methyl C
<220>
   <221> misc_feature
   <222> (1)..(1)
   <223> 5GalNAc conjugate, e.g. conj1a or Conj2a
<220>
   <221> misc_feature
   <222> (13)..(16)
   <223> LNA nucleosides, LNA C are 5-methyl C
<400> 56
   gaggcatagc agcagg 16
<210> 57
   <211> 15
   <212> DNA
   <213> artificial
<220>
   <223> LNA antisense oligonucleotide conjugate
<220>
   <221> misc_feature
   <222> (1)..(15)
   <223> phosphorothioate lnternucleoside linkages
<220>
   <221> misc_feature
   <222> (1)..(15)
   <223> LNA nucleosides at position 1, 3, 6, 7, 10, 12, 14 & 15, LNA C are 5-methyl C
<400> 57
   ccattgtcac actcc 15
<210> 58
   <211> 7
   <212> DNA
   <213> artificial
<220>
   <223> LNA antisense oligonucleotide conjugate
<220>
   <221> misc_feature
   <222> (1)..(7)
   <223> phosphorothioate lnternucleoside linkages
<220>
   <221> misc_feature
   <222> (1)..(7)
   <223> LNA nucleosides, LNA C are optionally 5-methyl C
<400> 58
   acactcc 7
<210> 59
   <211> 8
   <212> DNA
   <213> artificial
<220>
   <223> LNA antisense oligonucleotide conjugate
<220>
   <221> misc_feature
   <222> (1)..(7)
   <223> phosphorothioate lnternucleoside linkages
<220>
   <221> misc_feature
   <222> (1)..(7)
   <223> LNA nucleosides, LNA C are optionally 5-methyl C
<400> 59
   cacactcc 8
<210> 60
   <211> 14
   <212> DNA
   <213> artificial
<220>
   <223> LNA antisense gapmer oligonucleotide conjugate
<220>
   <221> misc_feature
   <222> (1)..(14)
   <223> phosphorothioate lnternucleoside linkages
<220>
   <221> misc_feature
   <222> (1)..(3)
   <223> LNA nucleosides, LNA C are 5-methyl C
<220>
   <221> misc_feature
   <222> (1)..(1)
   <223> optional 5 conjugate e.g 5 3xGalNAc conjugate, e.g. conj1a or Conj2a
<220>
   <221> misc_feature
   <222> (12)..(14)
   <223> LNA nucleosides, LNA C are 5-methyl C
<400> 60
   attccctgcc tgtg 14
<210> 61
   <211> 28
   <212> DNA
   <213> artificial
<220>
   <223> LNA antisense gapmer poly-oligonucleotide
<220>
   <221> misc_feature
   <222> (1)..(12)
   <223> phosphorothioate lnternucleoside linkages
<220>
   <221> misc_feature
   <222> (1)..(2)
   <223> LNA nucleosides, LNA C are 5-methyl C
<220>
   <221> misc_feature
   <222> (1)..(1)
   <223> 5 conjugate e.g 5 3xGalNAc conjugate, e.g. conj1a or Conj2a
<220>
   <221> misc_feature
   <222> (11)..(12)
   <223> LNA nucleosides, LNA C are 5-methyl C
<220>
   <221> misc_feature
   <222> (15)..(28)
   <223> phosphorothioate lnternucleoside linkages
<220>
   <221> misc_feature
   <222> (15)..(17)
   <223> LNA nucleosides, LNA C are 5-methyl C
<220>
   <221> misc_feature
   <222> (26).. (28)
   <223> LNA nucleosides, LNA C are 5-methyl C
<400> 61
   gttgacactg tccaattccc tgcctgtg 28
<210> 62
   <211> 15
   <212> DNA
   <213> artificial
<220>
   <223> LNA antisense gapmer oligonucleotide conjugate
<220>
   <221> misc_feature
   <222> (1)..(3)
   <223> phosphodiester lnternucleoside linkages
<220>
   <221> misc_feature
   <222> (1)..(1)
   <223> 5 FAM conjugate
<220>
   <221> misc_feature
   <222> (3)..(15)
   <223> phosphorothioate lnternucleoside linkages
<220>
   <221> misc_feature
   <222> (3)..(4)
   <223> LNA nucleosides, LNA C are 5-methyl C
<220>
   <221> misc_feature
   <222> (13)..(15)
   <223> LNA nucleosides, LNA C are 5-methyl C
<400> 62
   cagcattggt attca 15

## Description

### FIELD OF INVENTION

The invention relates to the field of LNA therapeutic single stranded antisense oligonucleotide conjugates. The invention provides LNA therapeutics oligonucleotide carbohydrate conjugates with considerably enhanced potency, extended therapeutic index and reduced toxicity.

### BACKGROUND

Oligonucleotide conjugates have been extensively evaluated for use in siRNAs, where they are considered essential in order to obtain sufficient *in vivo* potency. For example, see WO2004/044141 refers to modified oligomeric compounds that modulate gene expression via an RNA interference pathway. The oligomeric compounds include one or more conjugate moieties that can modify or enhance the pharmacokinetic and pharmacodynamic properties of the attached oligomeric compound.

WO2012/083046 reports on a galactose cluster-pharmacokinetic modulator targeting moiety for siRNAs.

In contrast, single stranded antisense oligonucleotides are typically administered therapeutically without conjugation or formulation. The main target tissues for antisense oligonucleotides are the liver and the kidney, although a wide range of other tissues are also accessible by the antisense modality, including lymph node, spleen, bone marrow.

According to van Poelgeest et al., (American Journal of Kidney Disease, In Press), the administration of an LNA antisense oligonucleotide in human clinical trials may have resulted in acute kidney injury. According to Swayze et al, NAR, Dec. 2006, advanced online publication antisense oligonucleotides containing locked nucleic acid improve potency but cause significant hepatotoxicity in animals.

WO2004/087931 refers to oligonucleotides comprising an acid cleavable hydrophilic polymer (PEG) conjugate.

WO 2005/086775 refers to targeted delivery of therapeutic agents to specific organs using a therapeutic chemical moiety, a cleavable linker and a labeling domain. The cleavable linker may be, for example, a disulfide group, a peptide or a restriction enzyme cleavable oligonucleotide domain.

WO 2011/126937 refers to targeted intracellular delivery of oligonucleotides via conjugation with small molecule ligands.

WO2009/025669 refers to polymeric (polyethylene glycol) linkers containing pyridyl disulphide moieties. See also Zhao et al., Bioconjugate Chem. 2005 16 758 - 766 and Zhu et al 2008 Bioconjugate Chemistry, vol. 19, pages 290-298.

Chaltin et al., Bioconjugate Chem. 2005 16 827 - 836 reports on cholesterol modified mono- di- and tetrameric oligonucleotides used to incorporate antisense oligonucleotides into cationic liposomes, to produce a dendrimeric delivery system. Cholesterol is conjugated to the oligonucleotides via a lysine linker.

Other non-cleavable cholesterol conjugates have been used to target siRNAs and antagomirs to the liver - see for example, Soutscheck et al., Nature 2004 vol. 432 173 - 178 and Krützfeldt et al., Nature 2005 vol 438, 685 - 689. For the partially phosphorothiolated siRNAs and antagomirs, the use of cholesterol as a liver targeting entity was found to be essential for *in vivo* activity.

Bhat et al., AASLD November 7 - 11th 2013 (poster) disclosed data from the use of a GalNac conjugated anti-miR, RG-101 targeting miR-122 for reduction of HCV in preclinical studies. The identity of RG-101 was not disclosed.

Biessen et al 1999 Biochem. J. vol 340, p 783-792 describes targeting of phosphodiester oligonucleotides to parenchymal liver cells using a lysine trimer with 4 galactose residues attached (L3G4).

Zheng et al 2003 World Journal of Gastroenterology, vol. 9, pages 1251-1255 describe a phosphtioate anti-HBV oligonucleotide conjugated to a galactosylated poly-L-lysine.

Maier et al 2003 Bioconj. Chem Vol 14, p. 18-29 describe the synthesis of a cholane scaffold with three -aminocapramide linked galactosyl residues conjugated to a phosporothiate or MOE modified oligonucleotide.

The present invention is based upon the discovery that the potency, bio-distribution and therapeutic index of single stranded LNA antisense oligonucleotides can be vastly improved by the conjugation of the LNA oligonucleotide to a carbohydrate conjugate, such as a GalNAc conjugate.

### RELATED APPLICATIONS

This application claims priority from EP13153296.2 (filed 2013-01-30), EP13157237.2 (filed 2013-02-28), EP13174092.0, (filed 2013-06-27), EP13192938.2, (filed 2013-11-14), EP13192931.7 (filed 2013-11-14), EP13192930.9 (filed 2013-11-14), WO2014/076195 (filed 2013-11-14), and WO2014/076196 (filed 2013-11-14):

### SUMMARY OF INVENTION

The invention provides for an LNA antisense oligomer conjugate comprising an antisense oligomer for use in modulating a nucleic acid and conjugate moiety which comprises an asialoglycoprotein receptor targeting moiety, such as a GalNAc moiety, and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20 acyl, wherein the conjugate moiety (referred to as region C) is covalently bound to the LNA antisense oligomer (referred to as region A).

The invention provides for an LNA antisense oligomer conjugate comprising an antisense oligomer and a GalNAc moiety, such as a trivalent GalNAc moiety, and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20 acyl.

The invention provides for an LNA antisense oligomer conjugate comprising an LNA antisense gapmer oligomer and an asialoglycoprotein receptor targeting conjugate moiety, such as a GalNAc moiety, and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20. The LNA antisense oligomer gapmer part of the conjugate may, for example, target a mRNA or viral RNA target.

The invention provides for an LNA antisense oligomer conjugate comprising an LNA antisense mixmer oligomer and an asialoglycoprotein receptor targeting conjugate moiety, such as a GalNAc moiety, and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20 acyl. The LNA antisense oligomer mixmer part of the conjugate may, for example target a mRNA, such as a mRNA splice site, or a microRNA target.

The invention provides for an LNA antisense oligomer conjugate comprising an LNA antisense totalmer oligomer and an asialoglycoprotein receptor targeting conjugate moiety, such as a GalNAc moiety, and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20 acyl. The LNA antisense oligomer mixmer part of the conjugate may, for example target a microRNA target.

The LNA antisense oligomer part of the conjugate may be 7 - 30, such as 8 - 26, or in some embodiments 8 - 18, nucleosides in length and it comprises at least one LNA unit (nucleoside).

The invention provides for an LNA antisense oligomer covalently joined to (e.g. linked to) a asialoglycoprotein receptor targeting moiety comprising multi-valent, such as, for example 2, 3, 4 or 4 identical or non-identical carbohydrate moieties and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20 acyl. The ASGPR targeting may be covalently joined to the oligomer, optionally via a linker or linkers.

The application describes an LNA antisense oligomer (conjugate) comprising an antisense oligomer and a conjugate moiety which comprises a carbohydrate, such as a carbohydrate conjugate moiety.

The invention provides for a pharmaceutical composition comprising the LNA oligomeric compound of the invention and a pharmaceutically acceptable diluent, carrier, salt or adjuvant.

The invention provides for the oligomeric conjugate according to the invention for use in the inhibition of a nucleic acid target in a cell. In some embodiments the use is in vitro. In some embodiments the use is in vivo.

The invention provides for the oligomeric conjugate of the invention for use in medicine, such as for use as a medicament.

The invention provides for the oligomeric conjugate of the invention for use in the treatment of a medical disease or disorder.

The invention provides for the use of the oligomeric conjugate of the invention for the preparation of a medicament for the treatment of a disease or disorder, such as a metabolic disease or disorder.

In one embodiment, the oligomer part of the conjugate is an LNA oligomer, comprising a contiguous region of 8 - 24 phosphorothioate linked nucleosides, and further comprising between 1 and 6 DNA nucleosides which are contiguous with the LNA oligomer, wherein the internucleoside linkages between the DNA, and/or adjacent to the DNA nucleoside(s), is physiologically labile, such as is / are phosphodiester linkages. Such an LNA oligomer may be in the form of a conjugate, as described herein. When conjugated, the conjugate may, for example a carbohydrate, such as a GalNAc conjugate, such as a GalNAc cluster, e.g. triGalNAc, or another conjugate as described herein.

### BRIEF DESCRIPTION OF FIGURES

**Figure 1****:** LNA Oligonucleotide GalNAc conjugation Step
**Figure 2****:** FVII protein levels
**Figure 3****:** FVII mRNA levels
**Figure 4a** **- d:** Example 3 - FVII levels in serum
**Figure 5a** **-d:** Example 3 - FVII mRNA levels in liver
**Figure 6****:** Example 3 - Oligonucleotide content in liver and kidney
**Figure 7a** **&b:** *In vivo* silencing of ApoB mRNA with different monoGalNAc conjugates. Mice were treated with with different ApoB monoGalNAc conjugates either without biocleavable linker, with Dithio-linker (SS) or with DNA/PO-linker (PO) (A) and MonoGalNAc with DNA/PO-linker was compared to GalNAc cluster (B). RNA was isolated from liver (A)and kidney samples (B) and analysed for ApoB mRNA knock down. Data is shown compared to Saline (=1).
**Figure 8****:** Example 5 - ApoB mRNA expression
**Figure 9****:** Example 5 - Total cholesterol in serum
**Figure 10****:** Example 5 - Oligonucleotide content in liver and kidney
**Figure 11****:** Example 6 - FVII levels in serum
**Figure 12****:** Example 6 - FVII mRNA levels in liver

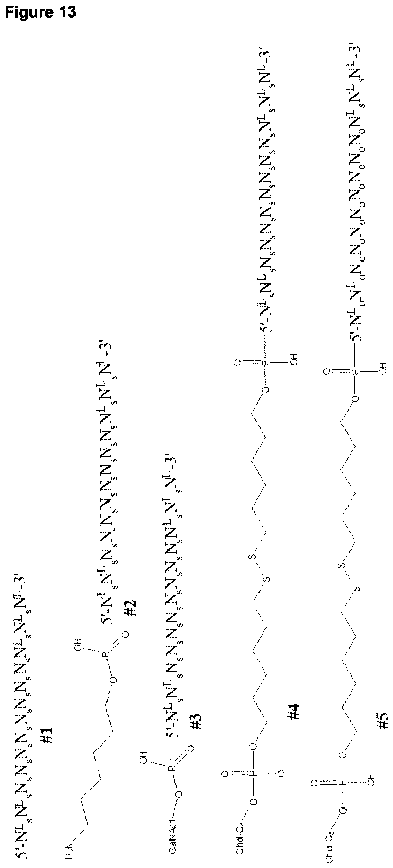

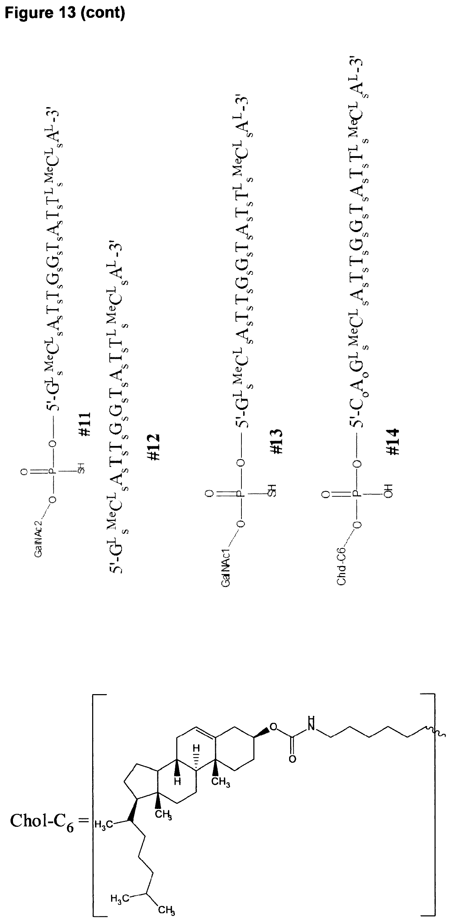

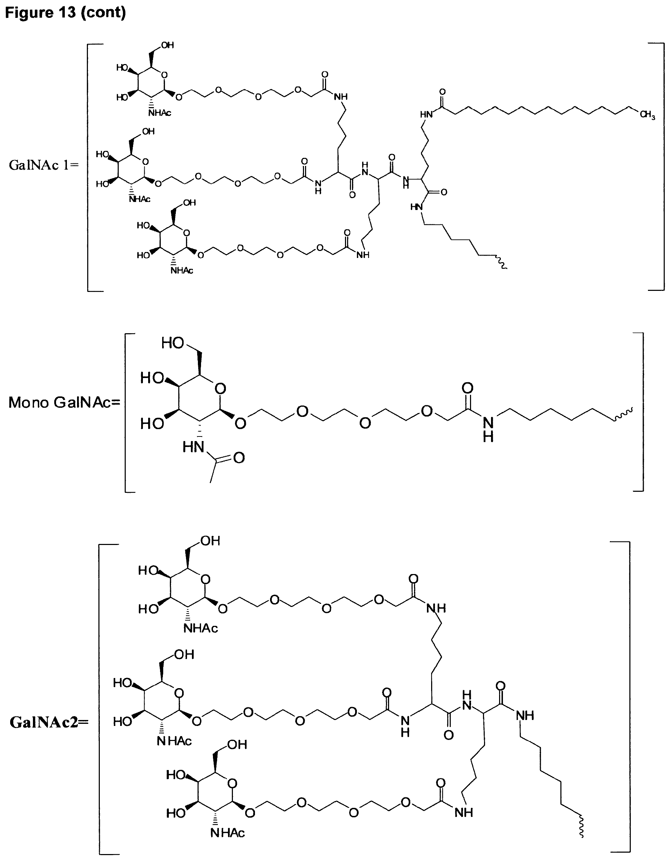

**Figure 13****:** Examples of the compounds and the conjugate moieties used in the examples

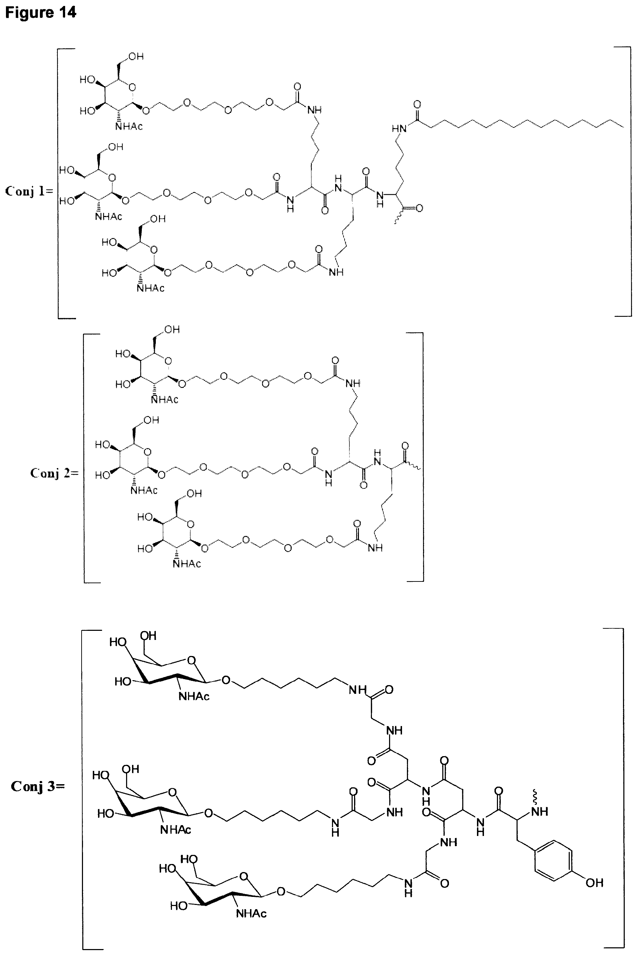

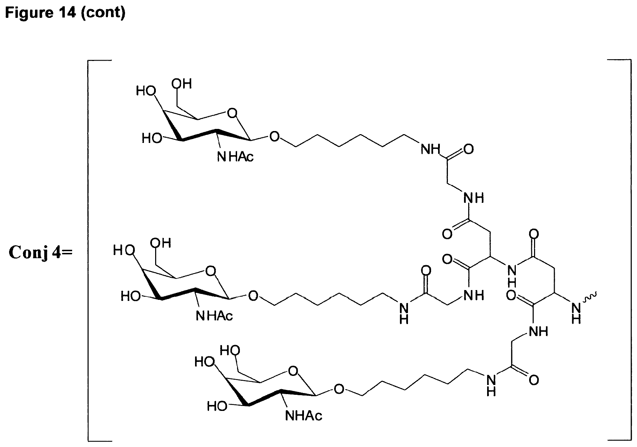

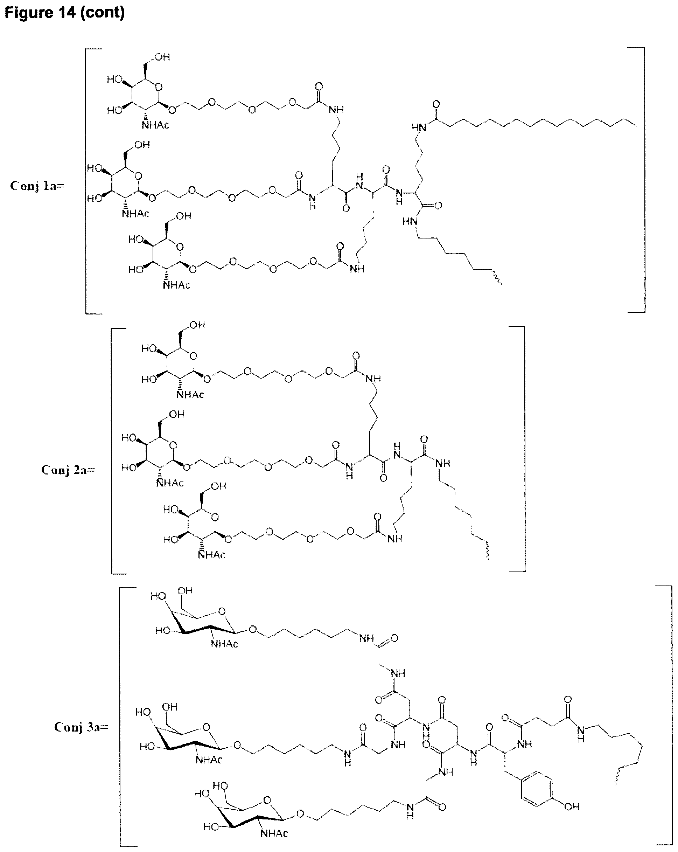

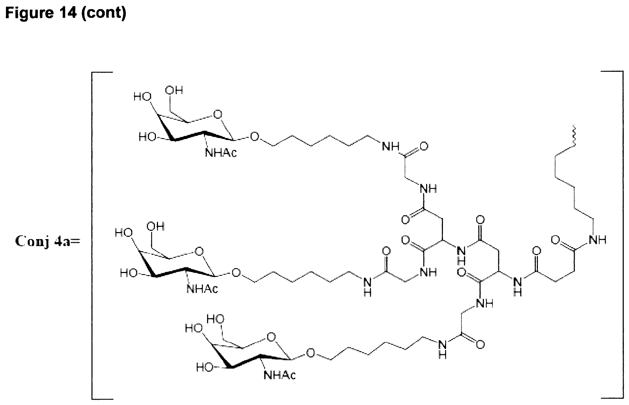

**Figure 14****:** Examples of tri-GalNac conjugates which may be used. Conjuagtes 1 - 4 illustrate 4 suitable GalNac conjugate moieties, and conjugates 1a - 4a refer to the same conjugates with an additional linker moiety (Y) which is used to link the conjugate to the oligomer (region A or to a biocleavable linker, such as region B). The wavy line represents the covalent link to the oligomer.
**Figure 15****:** Kim-1 expression from rat safety study (see Example 10)
**Figure 16****:** Serum ApoB and LDL-C levels in mice treated with a single dose of SEQ ID NO 32 or 17 at 1 or 2.5mg/kg.
**Figure 17****:** Silencing of miR-122 in the mouse liver by seed-targeting tiny LNA. (a) RNA blot analysis of liver RNAs from mice after treatment with three intravenous doses of 20 mg/kg tiny antimiR-122, 15-mer antimiR-122 or LNA scramble control or with saline.
**Figure 18****:** Total Cholesterol analysis at pre-dose, day 4 and day7. Cholesterol is upregulated due to decreased miR122.
**Figure19****:** Expression of Aldo A and Bckdk was measured by standard TaqMan Q-PCR assays. The mRNA levels of these genes are upregulated due to decreased miR122.
**Figure 20****:** ALT was measured from final serum (day 7) to assess tolerability of the compounds.
**Figure 21****:** Viral replication in the livers of Balb/C mice, was determined 7 days after hydrodynamic tailvein injection of pAAV2/HBV to determine antiviral effect of LNA compounds SEQ ID 55 and SEQ ID 56. The compounds were given iv in a single dose 24 hours before the hydrodynamic injection, at the dose level indicated and compared to entecavir, given daily p.o., starting 24 h after hydrodynamic injection. HBV DNA was quantified by qPCR, and reported as genome equivalents per 100ng liver DNA.

### DESCRIPTION OF THE INVENTION

The invention relates to LNA oligomeric compounds, such as LNA antisense oligonucleotides, which are covalently linked to a non-nucleotide carbohydrate conjugate group.

### The Oligomer

The application describes a LNA antisense oligomer conjugate, comprising a LNA antisense oligomer and a conjugate moiety which comprises a carbohydrate, such as a carbohydrate conjugate moiety, covalently bound to the LNA antisense oligomer.

The LNA antisense oligomer conjugate of the present invention employs LNA oligomeric compounds (also referred herein as LNA oligomers or LNA oligonucleotides) for use in modulating, such as inhibiting a target nucleic acid in a cell. An LNA oligomer comprises at least one "Locked Nucleic Acid" (LNA) nucleoside, such as a nucleoside which comprises a covalent bridge (also referred to a radical) between the 2' and 4' position (a 2' - 4' bridge). LNA nucleosides are also referred to as "bicyclic nucleosides". The LNA oligomer is typically a single stranded antisense oligonucleotide.

In some embodiments the LNA oligomer comprises or is a gapmer. In some embodiments the LNA oligomer comprises or is a mixmer. In some embodiments the LNA oligomer comprises or is a totalmer.

In some embodiments, the nucleoside analogues present in the oligomer are all LNA, and the oligomer may, optionally further comprise RNA or DNA, such as DNA nucleosides (e.g. in a gapmer or mixmer).

In various embodiments, the compound of the invention does not comprise RNA (units). In some embodiments, the oligomer has a single contiguous sequence which is a linear molecule or is synthesized as a linear molecule. The oligomer may therefore be single stranded molecule. In some embodiments, the oligomer does not comprise short regions of, for example, at least 3, 4 or 5 contiguous nucleotides, which are complementary to equivalent regions within the same oligomer (*i.e.* duplexes). The oligomer, in some embodiments, may be not (essentially) double stranded. The oligomer is essentially not double stranded, such as is not a siRNA. In some embodiments, the oligomeric compound is not in the form of a duplex with a (substantially) complementary oligonucleotide - e.g. is not an siRNA.

### Length

The term "oligomer" in the context of the present invention, refers to a molecule formed by covalent linkage of two or more nucleotides (*i.e.* an oligonucleotide). Herein, a single nucleotide (unit) may also be referred to as a monomer or unit. In some embodiments, the terms "nucleoside", "nucleotide", "unit" and "monomer" are used interchangeably. It will be recognized that when referring to a sequence of nucleotides or monomers, what is referred to is the sequence of bases, such as A, T, G, C or U.

The oligomer may consists or comprises of a contiguous nucleotide sequence of from 7 - 30, such as 7 - 26 or 8 - 25, such as 9, 10, 11, 12, 13, 14, 15, 16, 17, 18, 19, 20, 21, 22, 23, 24, 25 nucleotides in length, such as 10 - 20 nucleotides in length. In some embodiments, the length of the LNA oligomer is 10 - 16 nucleotides, such as 12, 13 or 14 nucleosides. In some embodiments, the LNA oligomer is 7, 8, 9 nucleosides in length, such as a "Tiny" LNA.

In some embodiments, the oligomers comprise or consist of a contiguous nucleotide sequence of a total of from 10 - 22, such as 12 - 18, such as 13 - 17 or 12 - 16, such as 13, 14, 15, 16 contiguous nucleotides in length.

In some embodiments, the oligomers comprise or consist of a contiguous nucleotide sequence of a total of 10, 11, 12, 13, or 14 contiguous nucleotides in length.

In some embodiments, the oligomer according to the invention consists of no more than 22 nucleotides, such as no more than 20 nucleotides, such as no more than 18 nucleotides, such as 15, 16 or 17 nucleotides. In some embodiments the oligomer of the invention comprises less than 20 nucleotides. It should be understood that when a range is given for an oligomer, or contiguous nucleotide sequence length it includes the lower an upper lengths provided in the range, for example from (or between) 10 - 30, includes both 10 and 30.

Described in the application is the use of the carbohydrate conjugates which have been found to be particularly suitable for short LNA oligomers, including short gapmers, mixmers or totalmers (tinys) such as LNA oligomers of less than 20, such as less than 18, such as 16nts or less or 15 or 14nts or less.

### Internucleoside Linkages

In some embodiments, the internucleoside linkages of the LNA oligomer comprise at least one internucleoside linkage other than phosphodiester, such as at least one, such as at least 50%, such as at least 75%, such as at least 90%, such as 100% of the internucleoside linkages in region A are other than phosphodiester. In some embodiments, the internucleoside linkages other than phosphodiester are sulphur containing internucleoside linkages, such as phosphorothioate, phosphorodithioate such as phosphorothioate.

The LNA oligomer may comprise at least one phosphorothioate internucleoside linkage, such as at least two, three or four phosphorothioate linkages, and in some embodiments at least 50% of the internucleoside linkages may be phosphorothioate, such as at least 75%, at least 90% or all internucleoside linkages (other than those, optionally present in a cleavable linker) may be phosphorothioate. In some embodiments the internucleoside linkages between the two terminal nucleosides at the 5' end, the 3' end or both the 5' and 3' end (other than in region B, when present), are sulphur containing internucleoside linkages, such as phosphorothioate. In some embodiments the oligomer comprises at least one region of consecutive DNA nucleosides, such as a region of 2, 3, 4, 5, 6, 7, 8, 9, 10, 11, 12 consecutive DNA nucleosides, wherein the internucleoside linkage between the DNA nucleosides is a sulphur containing internucleoside linkage such as phosphorothiote. In some embodiments, the central region of a gapmer (Y), which typically comprises a region of consecutive DNA nucleosides has are sulphur containing internucleoside linkages, such as phosphorothioate, between the nucleosides, such as between consecutive DNA nucleosides and/or between DNA nucleosides and nucleic acid analogue nucleosides, such as the sugar modified nucleosides referred to herein, such as LNA.

Other examples of internucleoside linkages which may be used in the oligomer include methylphosphonates (CH₃P=O) and methylthionophosphate (CH₃P=S) and boranophosphate.

### Locked Nucleic Acid Nucleosides (LNA)

Bicyclic nucleoside analogues (LNA nucleosides) include nucleoside analogues typically which comprise a bridge (or biradical) linking the second and forth carbon of the ribose ring, (C4*-C2* bridge or biradical). The presence of the biradical between the 2^{nd} and 4^{th} carbon locks the ribose into a 3' endo- (north) confirmation, and as such bicyclic nucleoside analogues with a C2*-C4* biradical are often referred to as Locked nucleic acid (LNA), or bicyclic nucleic acids (BNA). The terms LNA and BNA are used interchangeably herein.

In some embodiments, some or all of the nucleosides of the LNA oligomer, present in the LNA antisense oligomer conjugate, may be modified nucleosides, also referred to as nucleoside analogues herein, such as sugar modified nucleoside analogues, for example bicyclic nucleoside analogues (e.g. LNA) and/or 2'substituted nucleoside analogues. In some embodiments, the nucleoside analogues present in the oligomer all comprise the same sugar modification, for example are all bicyclic nucleoside analogues, such as they may be (optionally independently) selected from the group consisting of beta-D-X-LNA or alpha-L-X-LNA (wherein X is oxy, amino or thio), or other LNAs disclosed herein including, but not limited to,(R/S) cET, cMOE or 5'-Me-LNA.

In some embodiments, the oligomer may comprise at least one bicyclic nucleoside (LNA) and at least one further nucleoside analogue, such as one or more 2'substituted nucleoside. In some embodiments, some or all of the nucleosides of the oligomer may be modified nucleosides, also referred to as nucleoside analogues herein.

In some embodiments, the first region comprises at least one, such as at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 or 25 nucleoside analogues. In some embodiments the nucleoside analogues are (optionally independently) selected from the group consisting of bicyclic nucleoside analogues (such as LNA), and/or 2' substituted nucleoside analogues, such as (optionally independently) selected from the group consisting of 2'-O-alkyl-RNA units, 2'-OMe-RNA units, 2'-amino-DNA units, 2'-AP, 2'-FANA, 2'-(3-hydroxy)propyl, and 2'-fluoro-DNA units, and/or other (optionally) sugar modified nucleoside analogues such as morpholino, peptide nucleic acid (PNA), CeNA, unlinked nucleic acid (UNA), hexitol nucleoic acid (HNA). bicyclo-HNA (see e.g. WO2009/100320), In some embodiments, the further nucleoside analogues increase the affinity of the first region for its target nucleic acid (or a complementary DNA or RNA sequence). Various nucleoside analogues are disclosed in Freier & Altmann; Nucl. Acid Res., 1997, 25, 4429-4443 and Uhlmann; Curr. Opinion in Drug Development, 2000, 3(2), 293-213.

In some embodiments, the LNA oligomer comprises at least 2, at least 3, at least 4, at least 5, at least 6, at least 7, at least 8, at least 9, at least 10, at least 11, at least 12, at least 13, at least 14, at least 15, at least 16, for example all nucleoside analogues (or in a totalmer all nucleosides) bicyclic nucleoside analogues, such as LNA, e.g. beta-D-X-LNA or alpha-L-X-LNA (wherein X is oxy, amino or thio), or other LNAs disclosed herein including, but not limited to,(R/S) cET, cMOE or 5'-Me-LNA. In some embodiments, the LNA oligomer, comprises of DNA and sugar modified nucleoside analogues, such as bicyclic nucleoside analogues and/or 2'substituted nucleoside analogues. In some embodiments, the oligomer comprises of DNA and LNA nucleoside units.

WO05/013901, WO07/027775, WO07027894 refers to fully 2'substituted oligomers, such as fully 2'-O-MOE. In some embodiments, the first region of the oligomer may comprise of 2' substituted nucleosides. WO07/027775 also refers to MOE, LNA, DNA mixmers for use in targeting microRNAs.

### The Target

The LNA oligomer conjugates of the invention typically are for use in targeting a nucleic acid target, referred to as a target herein. In some embodiments, for a non-limiting example, the oligomer of the invention is for use in modulating a nucleic acid (*i.e.* targets) selected from the group consisting of a mRNA, a microRNA, a IncRNA (long non-coding RNA), a snRNA, snoRNA, and a viral RNA.

In some embodiments, the oligomer targets a liver-expressed RNA, such as a liver-expressed mRNA or microRNA. In some embodiments, the LNA antisense oligomer targets a mRNA, selected from the group consisting of a FactorVII, PCSK9, ApoB, GCGR, CRP, DGAT2, GCCR, PTEN, PTP1B, SGLT2 and SOD1 (see e.g. WO 2007/146511.). We specifically incorporate table 1 of WO'511 which is on page 42 - 43). In some embodiments, the target may be selected from the group consisting of: ApoB Human NM 000384.1, ApoB Mouse XM 137955.5, SGLT2 Human NM 003041.1 , PCSK9 Human NM 174936.2, SODI Human X02317, CRP Human NM 000567.1, GCCR Mouse BC031885.1, GCGR Human NM 000160.1, DGAT2 Human NM 032564.2, PTPIB Human NM 002827.2, PTEN Mouse U92437.1, PTEN Human NM 000314.4 (references are GENBANK Accession No), complement factor VII, complement factor C6, and TTR. Nucleotide sequences that encode Factor VII include, without limitation, the following: GENBANK Accession No. NM 000131.3, GENBANK Accession No. NM 019616.2, nucleotides 1255000 to 5 1273000 of GENBANK Accession No. NT 027140.6, GENBANK Accession NM 010172.3 and nucleotides 10024000 to 10037000 of GENBANK Accession No. NT 039455.6. Complement factor C6 nucleotide sequences include BC035723.1, J05024.1 GI:187824, and J05064.1 GI:179703. See also EP 2320925 A2 which refers to LNA oligos targeting complement factor C6. TTR (transthyretin) nucleotide sequences include, BC020791.1 GI:18089144, and BC005310.1 GI:13529049.

Examples of LNA oligomer targeting PCSK9 are provided in WO2008/043753 and WO2001/009697.

Examples of LNA oligomers targeting ApoB are provided in WO2010014280 & WO2008/113830.

The target may, in some embodiments be a Bcl2 mRNA - please see WO2005/061710 which discloses LNA oligomers targeting Bcl2 (e.g. M13994.1 GI:179366).

The target may be a RNA which is expressed in the liver. The target may be a liver target which is associated with a medical disorder or disease condition. Numerous metabolic diseases are associated with the liver, and may, in some embodiments, be treated using the compounds of the invention. Liver related metabolic disease or disorders targets include, for example Apo-B (high LDL cholesterol, ACS), ApoCIII (high serum Trigluceride), ApoA (cardiovascular disease), FGFR4 (obesity), GCCR (T2 diabetes), GCGR (T2 diabetes), PTP1B (T2 diabetes), DGAT2 (NASH), PCSK9 (hyperlipidaemia and related disorders), MtGPAT, miR-122 (high cholesterol), and miR-33 (metabolic syndrome, atherosclerosis).

The target may be a viral nucleic acid, such as a viral RNA. Examples include hepatitis virus', such as hepatitis B and hepatitis C (HCV). Viral hepatitis includes hepatitis A, B, C, D and E. HCV LNA antisense oligomers are disclosed in for example Laxton et al., Antimicrobial Agents and Chemotherapy 2011 Vol 55 3105 - 3114.

The oligomer may therefore be for use in the treatment of a liver related (or associated) metabolic disease - referred to generally as a metabolic liver disease. Liver related metabolic disease include for examples, metabolic syndrome, obesity, hyperlipidaemia, atherosclerosis, HDL/LDL cholesterol imbalance, dyslipidemias, e.g., familial combined hyperlipidaemia (FCHL), acquired hyperlipidaemia, statin-resistant hypercholesterolemia, cardiovascular disease, coronary artery disease (CAD), and coronary heart disease (CHD)., atherosclerosis, heart disease, diabetes (I and/or II), NASH, acute coronary syndrome (ACS). In some embodiments, a liver related or associated disorder or disease is associated with the expression, such as over-expression of a liver nucleic acid target.

In some embodiments, such as when the target is microRNA-122, the disease may be a viral disease, such as hepatitis, including hepatitis B and hepatitis C, or a metabolic disease related to elevated cholesterol, such as atherosclosis and hyperlipidaemia and related disorders. Oligomers targeting miR-122 may also be used for the improvement of hepatic function (see e.g. WO13068348), for the treatment of treatment of necroinflammation, for improving blood serum biomarkers of liver function, for preventing loss (or reducing the rate of loss) of liver function in a human subject who may or may not be infected with HCV and is at risk of deteriorating liver function, improving liver function in a human subject who is or who is not infected with HCV, and is in need of improved liver function. The liver disease may be a disease or disorder selected from the group consisting of non-alcoholic fatty liver disease and non-alcoholic steatohepatitis; or from the group consisting of a disease or disorder selected from the group consisting of cytomegalovirus infection, schistosomiasis infection and Leptospirosis infection.

In some embodiments, the oligomer, present in the LNA antisense oligomer conjugate of the invention, targets a liver expressed microRNA, such as miR-122. Oligomer's targeting miR-122 are disclosed in WO2007/112754, WO2007/112753, WO2009/043353, and may be mixmers, such as SPC3649, also referred to as miravirsen (which has the sequence 5'-CcAttGTcaCaCtCC-3' (SEQ ID NO 57), where capital letters are beta-D-oxy LNA, small letters are DNA, fully phosphorothioate and LNA C are 5-methyl cyctosine), or a tiny LNA, such as those disclosed in WO2009/043353 (e.g. 5'-ACACTCC-3', 5'-CACACTCC-3', 5'-TCACACTCC-3', SEQ ID NOs 58, 59 & 33) where capital letters are (optionally beta-D_oxy) LNA, fully phosphorothioate and LNA Cs are, optionally5-methyl cyctosine). In some embodiments, the miR-122 targeting oligomers have a length of 8, 9, 10, 11, 12, 13, 14, 15, 16, 17 or 18 nucleotides in length. In some embodiments, the miR-122 targeting oligomers a sequence which is fully complementary to miR-122 as measured accross the length of the oligomer, and preferably include the sequence 5'-CACACTCC-3'. According to miRBase, the mature microRNA-122 sequence is 5' uggagugugacaaugguguuugu 3' (SEQ ID NO 34). In some embodiments, the oligomer targeting a microRNA such as miR-122, is complementary to a corresponding region of the microRNA accorss the length of the oligomer and in some embodiments the 3' nucleoside of the oligomer is compelmentary to (*i.e.* aligns to) the first, second, third or fourth 5' nucleotides of the microRNA, such as miR-122, such as the second 5' nucleotide of the microRNA, such as miR-122.

In some embodiments, the oligomer, present in the LNA antisense oligomer conjugate of the invention, targets a liver expressed microRNA, such as miR-33 (miR-33a and/or miR-33b), which may be used in treating metabolic disorders such as atherosclerosis (see for example WO2010/120508). Oligomer's targeting miR-33a/b may comprise a nucleobase sequence selected from the group consisting of 5'-TACAATGCA-3', 5'-ACAATGCAC-3', 5'-ACAATGCA-3' & 5'-CAATGCA-3' (SEQ ID Nos 35 - 38), specific oligomers targeting miR-33a/b may be 5'-TACAATGCA-3', 5'-ACAATGCA-3' & 5'-CAATGCA-3', where capital letters are (optionally beta-D_oxy) LNA, fully phosphorothioate and LNA Cs are, optionally, 5-methyl cyctosine). According to miRBase, the mature microRNA-33a sequence is 5'-GUGCAUUGUAGUUGCAUUGCA-3' (SEQ ID NO 39), and miR-33b is 5' GUGCAUUGCUGUUGCAUUGC-3' (SEQ ID NO 40).

In some embodiments, the oligomer, present in the LNA antisense oligomer conjugate of the invention, targets a liver expressed microRNA, such as miR-21, which may be used in treating diseases such as liver fibrosis or hepatocellular carcinoma. Oligomer's targeting miR-21 may comprise a nucleobase sequence selected from the group consisting of 5'-TGATAAGCT-3', 5'- GATAAGCT-3', 5'- ATAAGCT-3' (SEQ ID Nos 41 -43), specific oligomers targeting miR-21 may be 5'- TGATAAGCT-3', 5'- GATAAGCT-3', 5'- ATAAGCT-3', or 5' TcAGtCTGaTaAgCT 3' (SEQ ID NO 44) where capital letters are (optionally beta-D_oxy) LNA, lower case letters are DNA, fully phosphorothioate and LNA Cs are, optionally, 5-methyl cyctosine). A fully LNA oligomer phosphorothioate (e.g. beta-D-oxy-LNA) with sequence 5'- GATAAGCT-3' (LNA C are 5-methylcytosine) has been extensively used in vivo for inhibiting miR-21 (SEQ ID NO 51). According to miRBase, the mature microRNA-21 sequence is 5'-UAGCUUAUCAGACUGAUGUUGA -3' (SEQ ID NO 45) .

In some embodiments, the oligomer, present in the LNA antisense oligomer conjugate of the invention, targets a liver expressed microRNA, such as miR-221, which may be used in treating, for example, hepatocellular carcinoma. Oligomer's targeting miR-221 may comprise a nucleobase sequence selected from the group consisting of 5'- CAATGTAGC-3', 5'- AATGTAGC-3', and 5'- ATGTAGC-3' (SEQ ID NO 46 - 48) specific oligomers targeting miR-221 include 5'- CAATGTAGC-3', 5'- AATGTAGC-3', and 5'- ATGTAGC-3', where capital letters are (optionally beta-D_oxy) LNA, fully phosphorothioate and LNA Cs are, optionally, 5-methyl cyctosine). According to miRBase, the mature microRNA-221 sequence is 5' AGCUACAUUGUCUGCUGGGUUUC 3' (SEQ ID NO 49).

Other microRNA targets and compounds targeting them which may be used as the oligomer or the invention, or contiguous nucleotide sequence thereof, or part thereof, are disclosed in WO2007/112754, such as those disclosed in tables 2 and example 29 of WO2007/112754, which are specifically hereby incorporated by reference. Other microRNA targets and compounds targeting them which may be used as the oligomer or the invention, or contiguous nucleotide sequence thereof, or part thereof, are disclosed in WO2009/043353, such as the compounds and targets disclosed in table 1 of WO2009/043353, which are hereby specifically incorporated by reference.

In some embodiments, the oligomer, present in the LNA antisense oligomer conjugate of the invention, is capable of down-regulating (e.g. reducing or removing) expression of the target (e.g. target nucleic acid). In this regards, the oligomer of the invention can affect the inhibition of the target. In some embodiments, the oligomers of the invention bind to the target nucleic acid and affect inhibition of expression of at least 10% or 20% compared to the normal expression level, more preferably at least a 30%, 40%, 50%, 60%, 70%, 80%, 90% or 95% inhibition compared to the normal expression level (such as the expression level in the absence of the oligomer(s) or conjugate(s)). In some embodiments, such modulation is seen when using from 0.04 and 25nM, such as from 0.8 and 20nM concentration of the compound of the invention. In the same or a different embodiment, the inhibition of expression is less than 100%, such as less than 98% inhibition, less than 95% inhibition, less than 90% inhibition, less than 80% inhibition, such as less than 70% inhibition. Modulation of expression level may be determined by measuring protein levels, *e.g.* by the methods such as SDS-PAGE followed by western blotting using suitable antibodies raised against the target protein. Alternatively, modulation of expression levels can be determined by measuring levels of mRNA, e.g. by northern blotting or quantitative RT-PCR. When measuring via mRNA levels, the level of down-regulation when using an appropriate dosage, such as from 0.04 and 25nM, such as from 0.8 and 20nM concentration, is, In some embodiments, typically to a level of from 10-20% the normal levels in the absence of the compound, conjugate or composition of the invention.

The invention therefore provides a method of down-regulating or inhibiting the expression of the target in a cell which is expressing the target, said method comprising administering the oligomer or conjugate according to the invention to said cell to down-regulating or inhibiting the expression of the target in said cell. Suitably the cell is a mammalian cell such as a human cell. The administration may occur, in some embodiments, *in vitro.* The administration may occur, in some embodiments, *in vivo.* Compounds of the invention, such as the oligomers and conjugates thereof, may be targeted to different targets, such as mRNA or microRNA or other nucleic acid targets which are expressed in the liver (references to NCBI Genbank/Gene IDs are given as examples of sequences which may be targeted by the compounds of the invention).

### ApoB

In some embodiments, the first region (or first and second region) form a single contiguous nucleobase sequence which is complementary, to a corresponding region of an ApoB mRNA target (*i.e.* targets) ApoB-100 (NCBI Genbank ID NM_000384.2 GI:105990531). Compounds of the invention which target ApoB may be used in the treatment of acute coronary syndrome (see WO20100076248). The invention therefore provides for the oligomer according to the invention which targets ApoB100 for use in the treatment of acute coronary syndrome. The invention further provides for a method of treatment of acute coronary syndrome, wherein said method comprises the administration of the oligomer of the invention to a subject in need to said treatment.

Compounds of the invention which target ApoB may be used in the treatment atherosclerosis. The invention therefore provides for a oligomer, present in the LNA antisense oligomer conjugate according to the invention, which targets ApoB100 for use in the treatment of atherosclerosis. The application describes a method of treatment of atherosclerosis, wherein said method comprises the administration of the oligomer of the invention to a subject in need to said treatment.

Compounds of the invention which target ApoB may be used in the treatment hypercholesterolemia or hyperlipidaemia. The invention therefore provides for the oligomer according to the invention which targets ApoB100 for use in the treatment of hypercholesterolemia or hyperlipidaemia. The application further describes a method of treatment of hypercholesterolemia or hyperlipidaemia, wherein said method comprises the administration of the oligomer of the invention to a subject in need to said treatment.

The application describes an *in vivo* or *in vitro* method for the inhibition of ApoB in a cell which is expressing ApoB, said method comprising administering an oligomer or conjugate or pharmaceutical composition according to the invention to said cell so as to inhibit ApoB in said cell.

Examples of LNA oligomers which may be used as the first region in the oligomers/conjugates of the invention include, for example those disclosed in WO2007/031081, WO2008/113830, WO2007131238, and WO2010142805. Specific preferred compounds include the following:
5'-Gₛ^{m}CₛaₛtₛtₛgₛgₛtₛaₛtₛTₛ^{m}CₛA -3' (SEQ ID NO 12)
5'- GₛTₛtₛgₛaₛcₛaₛcₛtₛgₛTₛ^{m}C -3' (SEQ ID NO 50)
Wherein capital letters are beta-D-oxy LNA units (nucleosides), lower case letters are DNA units, subscript s is a phosphorothioate linkage, and a superscript m before the capital C illustrates that all LNA cytosines are 5-methyl cytosine. Compounds of the invention targeting ApoB may be conjugated to a conjugate which targets the oligomer to the liver, as disclosed herein, such as a carbohydrate or lipophilic conjugate, such as a GalNac conjugate or a sterol conjugate (e.g. cholesterol or tocohperol). The conjugate may be, for example, at the 5' end or the 3' end of the oligomer compound (in some embodiments via region B)

Other oligomers which target ApoB are disclosed in WO03/011887, WO04/044181, WO2006/020676, WO2007/131238, WO2007/031081, and WO2010142805.

### PCSK9

In some embodiments, the first region (or first and second region) form a single contiguous nucleobase sequence which is complementary, to a corresponding region of a PCSK9 mRNA target (i.e. targets), such as the human PCSK9 mRNA: NCBI Genbank ID NM_174936.3 GI:299523249.

The invention provides for an oligomer, present in the LNA antisense oligomer conjugate according to the invention, which targets PCSK9, for use as a medicament, such as for the treatment of hypercholesterolemia or related disorder, such as a disorder selected from the group consisting of atherosclerosis, hyperlipidaemia, hypercholesterolemia, familiar hypercholesterolemia *e.g.* gain of function mutations in PCSK9, HDL/LDL cholesterol imbalance, dyslipidemias, *e.g.,* familial hyperlipidaemia (FCHL), acquired hyperlipidaemia, statin-resistant hypercholesterolemia, coronary artery disease (CAD), and coronary heart disease (CHD).

The invention provides for the use of an oligomer, present in the LNA antisense oligomer conjugate of the invention, which targets PCSK9, for the manufacture of a medicament for the treatment of hypercholesterolemia or a related disorder, such as a disorder selected from the group consisting of atherosclerosis, hyperlipidaemia, hypercholesterolemia, familiar hypercholesterolemia *e.g.* gain of function mutations in PCSK9, HDL/LDL cholesterol imbalance, dyslipidemias, *e.g.,* familial hyperlipidaemia (FCHL), acquired hyperlipidaemia, statin-resistant hypercholesterolemia, coronary artery disease (CAD), and coronary heart disease (CHD).

The application describes a method of treating hypercholesterolemia or a related disorder, such as a disorder selected from the group consisting atherosclerosis, hyperlipidaemia, hypercholesterolemia, familiar hypercholesterolemia *e.g.* gain of function mutations in PCSK9, HDL/LDL cholesterol imbalance, dyslipidemias, *e.g.,* familial hyperlipidaemia (FCHL), acquired hyperlipidaemia, statin-resistant hypercholesterolemia, coronary artery disease (CAD), and coronary heart disease (CHD), said method comprising administering an effective amount of an oligomer according to the invention which targets PCSK9, to a patient suffering from, or likely to suffer from hypercholesterolemia or a related disorder.

The application describes an *in vivo* or *in vitro* method for the inhibition of PCSK9 in a cell which is expressing PCSK9, said method comprising administering an oligomer according to the invention which targets PCSK9 to said cell so as to inhibit PCSK9 in said cell.

The following is an oligomer which targets the human PCSK9 mRNA, and may be used as region A in the compounds of the invention.
5'- T**ₛ**G**ₛ^{m}**C**ₛ**t**ₛ**a**ₛ**c**ₛ**a**ₛ**a**ₛ**a**ₛ**a**ₛ**c**ₛ^{m}**C**ₛ^{m}**C**ₛ**A-3' (SEQ ID NO 22)
Wherein capital letters are beta-D-oxy LNA units (nucleosides), lower case letters are DNA units, subscript s is a phosphorothioate linkage, and a superscript m before the capital C illustrates that all LNA cytosines are 5-methyl cytosine. Compounds of the invention targeting PCSK9 may be conjugated to a conjugate which targets the oligomer to the liver, as disclosed herein, such as a carbohydrate or lipophilic conjugate, such as a GalNac conjugate or a sterol conjugate (e.g. cholesterol or tocohperol). The conjugate may be, for example, at the 5' end or the 3' end of the oligomer compound (in some embodiments via region B).

Other oligomers which target PCSK9 are disclosed in the examples as well as WO2008/043753, WO2011/009697, WO08/066776, WO07/090071, WO07/146511, WO07/143315, WO09/148605, WO11/123621, and WO11133871. Other compounds, which may be used in the compounds of the invention, which target PCSK9 are illustrated in the examples.

### miR-122

In some embodiments, the first region (or first and second region) form a single contiguous nucleobase sequence which is complementary, to a corresponding region of a microRNA-122 such as miR-122a (i.e. targets), such as the has-miR-122 sequences (miRBase release 20: MI0000442), such as:
>hsa-mir-122 MI0000442 
CCUUAGCAGAGCUGUGGAGUGUGACAAUGGUGUUUGUGUCUAAACUAUCAAACGCCAUUAUCACACUAAAUAGCU
ACUGCUAGGC
>hsa-miR-122-5p MIMAT0000421
   UGGAGUGUGACAAUGGUGUUUG

miR-122 has been indicated in HCV infection, where it is an essential host factor required for maintenance of the infection. Inhibitors of miR-122 may therefore be used in the treatment of hepatitis C infection.

Compounds of the invention which target miR-122 may be used in the treatment of HCV infection. The invention therefore provides for the oligomer according to the invention which targets miR-122 for use in the treatment of HCV infection. The invention further provides for a method of treatment of HCV infection, wherein said method comprises the administration of the oligomer of the invention to a subject in need to said treatment.

The invention provides for the use of an LNA antisense oligomer conjugate of the invention comprising an oligomer which targets miR-122, for the manufacture of a medicament for the treatment of HCV infection.

The application describes a method of treating HCV infection, said method comprising administering an effective amount of an oligomer according to the invention which targets miR-122, to a patient suffering from HCV infection.

The application describes an *in vivo* or *in vitro* method for the inhibition of miR-122 in a cell which is expressing miR-122, such as an HCV infected cell or a HCV replicon expressing cell, said method comprising administering an oligomer or conjugate or pharmaceutical composition according to the invention to said cell so as to inhibit miR-122 in said cell.

miR-122 has also been indicated in cholesterol metabolism, and it has been suggested that inhibition of miR-122 may be used for a treatment to reduce plasma cholesterol levels (Esau, Cell Metab. 2006 Feb;3(2):87-98.)

Inhibitors of miR-122 may therefore be used in a treatment to reduce plasma cholesterol levels, or in the treatment of a metabolic disease associated with elevated levels of cholesterol (related disorders), such as indications selected from the group consisting of liver steatosis,_atherosclerosis, hyperlipidaemia, hypercholesterolemia, familiar hypercholesterolemia, dyslipidemias, coronary artery disease (CAD), and coronary heart disease (CHD)

Compounds of the invention which target miR-122 may be used in the treatment of elevated cholesterol levels or related disorders. The invention therefore provides for the LNA antisense oligomer conjugate of the invention comprising an oligomer which targets miR-122 for use in the treatment of elevated cholesterol levels or related disorders. The application further describes a method of treatment of elevated cholesterol levels or related disorders, wherein said method comprises the administration of the oligomer of the invention to a subject in need to said treatment.

The invention provides for the use of an LNA antisense oligomer conjugate of the invention comprising an oligomer which targets miR-122, for the manufacture of a medicament for the treatment of elevated cholesterol levels or related disorders.

The application describes a method of treating elevated cholesterol levels or related disorders, said method comprising administering an effective amount of an oligomer according to the invention which targets miR-122, to a patient suffering from said disorder.

The application describes an *in vivo* or *in vitro* method for the inhibition of miR-122 in a cell which is expressing miR-122, such as an HCV infected cell or a HCV replicon expressing cell, said method comprising administering an oligomer or conjugate or pharmaceutical composition according to the invention to said cell so as to inhibit miR-122 in said cell.

Oligomer's targeting miR-122 are disclosed in WO2007/112754, WO2007/112753, WO2009/043353, and may be mixmers, such as SPC3649, also referred to as miravirsen see below, or a tiny LNA, such as those disclosed in WO2009/043353 (e.g. 5'-ACACTCC-3', 5'-CACACTCC-3', 5'-TCACACTCC-3', (SEQ ID NOs 58, 64 & 33) where capital letters are beta-D-oxy LNA, fully phosphorothioate and LNA C are 5-methyl cyctosine). In some embodiments, the miR-122 targeting oligomers have a length of 8, 9, 10, 11, 12, 13, 14, 15, 16, 17 or 18 (or 19, 20, 21, 22 or 23 nucleotides) in length. In some embodiments, the miR-122 targeting oligomers a sequence which is fully complementary to miR-122 as measured accross the length of the oligomer, and preferably include the sequence 5'-CACACTCC-3'. In some embodiments, the oligomer targeting a microRNA such as miR-122, is complementary to a corresponding region of the microRNA accorss the length of the oligomer and in some embodiments the 3' nucleoside of the oligomer is compelmentary to (*i.e.* aligns to) the first, second, third or fourth 5' nucleotides of the microRNA, such as miR-122, such as the second 5' nucleotide of the microRNA, such as miR-122. The following is an oligomers which targets the has-miR-122 (human miR-122), and may be used as region A in the compounds of the invention.
Miravirsen: 5'- **^{m}Cₛ**c**ₛAₛ**t**ₛ**tₛ**GₛTₛcₛaₛ^{m}Cₛaₛ^{m}Cₛ**t**ₛ^{m}Cₛ^{m}C** -3' (SEQ ID NO 57)

Other miR-122 targeting compounds which may be used in the context of the present invention (region A) are disclosed in WO2007/027894, WO2007/027775. **MtGPAT:** (NCBI gene ID 57678 - Chromosome: 10;NC_000010.10(113907971 ..113975153, complement) Mitochondrial glycerol-3-phosphate acyltransferase 1 (EC 2.3.1.15, also known as GPAT1, mtGPAT1, GPAM, mtGPAM) playa major role in hepatic triglyceride formation, where high levels of mtGPAT1 activity results in fatty liver (hepatosteatosis) whereas the absence of mtGPAT1 results in low levels of liver triglycerides and stimulated fatty acid oxidation (see WO2010/000656 which discloses oligomers which target mtGPAT).

Compounds of the invention which target MtGPAT may be used to treat conditions such as being overweight, obesity, fatty liver, hepatosteatosis, non alcoholic fatty liver disease (NAFLD), non alcoholic steatohepatitis (NASH), insulin resistance, diabetes such as non insulin dependent diabetes mellitus (NIDDM).

The following oligomer (SEQ ID NO 60) targets human mtGPAT and may be used as the oligomer region (first region) in the compounds of the invention, Capital letters are LNA such as beta-D-oxy-LNA, lower case letters are DNA, subscript s is phosphorothioate linkage. LNA cytosines may be 5-methyl cytosine.
AₛTₛTₛcₛcₛcₛtₛgₛcₛcₛtₛGₛTₛG - 3'

**FactorVII** (NCBI Gene ID 2155, NCBI J02933.1 GI:180333, or EU557239.1 GI:182257998). The oligomer of the conjugate of the invention may target FactorVII, and thereby inhibit the production of Factor VII, a key component of the tissue factor coagulation pathway. Compounds of the invention which target FactorVII may be used for for the treatment or prevention of thrombotic diseases (typically without causing bleeding) and as heart attack, stroke and blood clots, or inflammatory conditions. WO 2013/119979 and WO 2012/174154 disclose oligonucleotide compounds which target FVII which may be incorporated into the conjugates of the present invention.

**Factor XI** (NCBI Genbank BC122863.1 GI:114108211)- Factor XI, a clotting factor that is produced in the liver. High levels of Factor XI are linked to heart attack, stroke and blood clots. WO 2013/070771 discloses oligonucleotide compounds which target XI which may be incorporated into the conjugates of the present invention. Compounds of the invention which target FactorXI may be used for for the treatment or prevention of thrombotic diseases, and as heart attack, stroke and blood clots, or inflammatory conditions such as arthritis and colitis.

**ApoCIII** (NCBI Genbank BC027977.1 GI:20379764) a protein that regulates triglyceride metabolism in blood. High levels of apoC-III are linked to inflammation, high triglycerides, atherosclerosis and metabolic syndrome. Compounds of the invention which target ApoCIII may be used to reduce serum triglyceride levels or in the treatment of e.g. familial chylomicronemia syndrome and severely high triglycerides either as a single agent or in combination with other triglyceride-lowering agents. WO11085271 , discloses oligonucleotide compounds which target ApoCIII which may be incorporated into the conjugates of the present invention.

**Apo(a)** (NCBI Genbank NM_005577.2 GI:116292749) inhibits the production of apo(a) in the liver and is designed to offer a direct approach to reducing Lp(a), an independent risk factor for cardiovascular disease. High levels of Lp(a) are associated with an increased risk of atherosclerosis, coronary heart disease, heart attack and stroke. Lp(a) promotes premature plaque buildup, or atherosclerosis, in arteries. Compounds of the invention which target Apo(a) may be used in the treatment of e.g. atherosclerosis and coronary heart disease. WO05000201 and WO03014307 discloses oligonucleotide compounds which target apolipoprotein (a) which may be incorporated into the conjugates of the present invention.

**Hepatitus B (HBV)** (see for example NCBI D23684.1 GI:560092; D23683.1 GI: 560087; D23682.1 GI: 560082; D23681.1 GI: 560077; D23680.1 GI: 560072; D23679.1 GI: 560067; D23678.1 GI: 560062; D23677.1 GI: 560057.

Oligomers which target HBV are well known in the art, for example see, WO96/03152, WO97/03211, WO2011/052911, WO2012/145674, WO2012/145697, WO2013/003520 and WO2013/159109.

Compounds of the invention which target HBV may be used in the treatment HBV infection. The invention therefore provides for the oligomer according to the invention which targets HBV for use in the treatment of HBV. The application further describes a method of treatment of HBV infection, wherein said method comprises the administration of the oligomer of the invention to a subject in need to said treatment.

The invention provides for an oligomer of the conjugate of the invention which targets hepatitis B (HBV) for use as a medicament, such as for the treatment hepatitis B infection or a related disorder.

The invention provides for the use of an oligomer conjugate or pharmaceutical composition according to the invention which targets hepatitis B (HBV), for the manufacture of a medicament for the treatment of hepatitis B infection or a related disorder.

The invention provides for a method of treating treatment hepatitis B infection or a related disorder, said method comprising administering an effective amount of an oligomer or conjugate of the invention which targets HBV, to a patient infected with Hepatitis B virus.

The invention provides for an in vivo or in vitro method for the inhibition of HBV replication in a cell infected with HBV, said method comprising administering an oligomer or conjugate of the invention which targets HBV to said cell so as to inhibit HBV replication. An example of an LNA oligomer which target's HBV is (as is disclosed in WO2011/47312) which may be used as the oligomer (region A) of the invention 5'- GₛAₛGₛGₛcₛaₛtₛaₛgₛcₛaₛgₛ^{m}CₛAₛGₛG - 3'. Further compounds are disclosed in table 1 of WO2011/47312, and in WO2011/052911, WO2012/145674, WO2012/145697, WO2013/003520 and WO2013/159109.

RG-101 is a compound which targets miR-122 and comprises a GalnNAc conjugate, and is being developed for treatment of HCV by Regulus Therapeutics.

**ANGPTL3,** (e.g. NCBI BC007059.1 GI: 14712025 or BC058287.1 GI: 34849466) ANGIOPOIETIN-LIKE 3 - a protein that regulates lipid, glucose and energy metabolism. Humans with elevated levels of ANGPTL3 have hyperlipidaemia associated with an increased risk of premature heart attacks, increased arterial wall thickness as well as multiple metabolic abnormalities, such as insulin resistance. In contrast, humans with lower levels of ANGPTL3 have lower LDL-C and triglyceride levels and a lower risk of cardiovascular disease. Compounds of the invention which target ANGPTL3 may be used in the treatment of e.g. hyperlipidaemia and related disorders, metabolic disorder, atherosclerosis, coronary heart disease or insulin resistance. WO11085271 discloses oligonucleotide compounds which target ANGPTL3 which may be incorporated into the conjugates of the present invention.

**Glucagon receptor, or GCGR** (BC112041.1 GI: 85567507; L20316.1 GI: 405189): Glucagon is a hormone that opposes the action of insulin and stimulates the liver to produce glucose, particularly in type 2 diabetes. In patients with advanced diabetes, uncontrolled glucagon action leads to a significant increase in blood glucose levels. Therefore, attenuating glucagon action may have a significant glucose lowering effect in patients with severe diabetes. In addition, reducing GCGR produces more active glucagon-like peptide, or GLP-1, a hormone that preserves pancreatic function and enhances insulin secretion. Compounds of the invention which target GCGR may be used in the treatment of e.g. or insulin resistance, hyperglycemia, diabetes, such as type 1 or 2 diabetes, preservation of pancreatic function, and to control of blood glucose levels. WO2007/134014 discloses oligonucleotide compounds which target GCGR.

**Fibroblast growth factor receptor 4, or FGFR4.** (NCBI Gene 2264 - NC_000005.9 (176513906..176525143) FGFR4 is expressed in the liver and fat tissues, and is indicated in decreasing the body's ability to store fat while simultaneously increasing fat burning and energy expenditure. Many anti-obesity drugs act in the brain to suppress appetite, commonly resulting in CNS side effects. Compounds of the invention which target FGFR4 may be used in the treatment of e.g. or insulin resistance, hyperglycemia, diabetes, such as type 1 or 2 diabetes, preservation of obesity (e.g. when used in combination with an appetite-suppressing drug), reducing body weight, and improvement in insulin sensitivity, diabetes, such as type 1 or 2 diabetes and to control of blood glucose levels. WO09046141 and WO12174476 disclose oligonucleotide compounds which target FGFR4 which may be incorporated into the conjugates of the present invention.

**Diacylglycerol acyltransferase-2, or DGAT-2** (NCBI GENE ID 84649): A key component in the synthesis of triglycerides. The inhibition of DGAT may reduce liver fat in patients with Nonalcoholic Steatohepatitis (NASH), and may also be used to treat type 2 diabetes and insulin resistance. Compounds of the invention which target DGAT-2 may be used to treat NASH, to reduce liver fat, to treat diabetes, such as type 2 diabetes, and treat insulin resistance. WO05019418 and WO2007136989 disclose oligonucleotide compounds which target DGAT-2 which may be incorporated into the conjugates of the present invention.

**Glucocorticoid receptor, or GCCR** (BC150257.1 GI: 152013043): Glucocorticoid hormones affect a variety of processes throughout the body, and excessive levels of glucocorticoid hormones can have a detrimental effect on many of the tissues and organs in the body. Cushing's Syndrome is an orphan disease caused by prolonged exposure to high levels of glucocorticoids. If untreated, patients with Cushing's Syndrome can develop hypertension, diabetes and impaired immune functions and have an increased risk of early death. Although there are approved treatments for Cushing's Syndrome, current medicines are associated with significant side effects, such as hypertension and diabetes, and there remains a high unmet medical need for new therapies for these patients. Compounds of the invention which target GCCR-2 may be used to treat Cushing's Syndrome and associated conditions (such as those listed above). WO07035759 and WO2007136988 disclose oligonucleotide compounds which target GCCR which may be incorporated into the conjugates of the present invention.

**Complement component C5** (M57729.1 GI: 179982): The complement system plays a central role in immunity as a protective mechanism for host defense, but its dysregulation results in serious, life-threatening complications in a broad range of human diseases including paroxysmal nocturnal hemoglobinuria (PNH), atypical hemolytic-uremic syndrome (aHUS), myasthenia gravis, neuromyelitis optica, amongst others. Compounds of the invention which target complement component C5 may be used to treat one or more of these disorders. C5 is a genetically and clinically validated target; loss of function human mutations are associated with an attenuated immune defense against certain infections and intravenously administered anti-C5 monoclonal antibody therapy has demonstrated clinical activity and tolerability in a number of complement-mediated diseases. transmembrane protease, serine 6 (Tmprss6) for the treatment of beta-thalassemia and iron-overload disorders.

**Alpha-1 antitrypsin (AAT):** (M11465.1 GI: 177826) Liver disease associated with - WO13142514 disclose oligonucleotide compounds which target AAT which may be incorporated into the oligomers or conjugates of the present invention. Compounds of the invention which target AAT may be used in methods for decreasing AIAT mRNA and protein expression and treating, ameliorating, preventing, slowing progression, or stopping progression of fibrosis, such as, AIATD associated liver disease, and pulmonary disease, such as, AIATD associated pulmonary disease in an individual in need thereof.

**Transthyretin - TTR** (BC005310.1 GI: 13529049) : The oligomers, present in the LNA antisense oligomer conjugate of the invention, which target TTR may be used to treat transthyretin amyloidosis, or TTR amyloidosis, a severe and rare genetic disease in which the patient inherits a mutant gene that produces a misfolded form of TTR, which progressively accumulates in tissues. In patients with TTR amyloidosis, both the mutant and normal forms of TTR can build up as fibrils in tissues, including heart, peripheral nerves, and the gastrointestinal tract. The presence of TTR fibrils interferes with the normal functions of these tissues, and as the TTR protein fibrils enlarge more tissue damage occurs and the disease worsens. TTR is a carrier protein that transports a thyroid hormone and retinol in the blood. In patients with TTR amyloidosis, both the mutant and normal forms of TTR can build up as fibrils in tissue. The compounds of the invention may be used to treat TTR amyloidosis. See Benson et al., Amyloid. 2010 Jun;17(2):43-9, and Ackermann et al., Amyloid. 2012 Jun;19 Suppl 1:43-4.). Antisense compounds targeting TTR which may be used in the oligomers or conjugates of the invention are disclosed in US8101743, WO11139917 and WO10017509.

**Aminolevulinate synthase-1 (ALAS-1)** (BC011798.2 GI: 33877783; AK312566.1 GI: 164690365; NM_199166.2 GI: 362999012; NM_000688.5 GI: 362999011). ALAS1 is a validated target for the treatment of porphyria, such as the treatment of hepatic porphyrias including acute intermittent porphyria (AIP). Compounds of the invention which target ALAS-1 may be used in the treatment of these disorders.

**Vascular endothelial growth factor,** or VEGF (GENE ID 7422, human Sequence: Chromosome: 6; NC_000006.11 (43737946..43754224)). VEGF is indicated in cancers. Compounds of the invention which target VEGF may be used in the treatment of hyperproliferative disorders, such as cancer, such as liver cancer.

Table 3 provides for a group of liver targets which may be targeted by the compounds of the invention, as well as the medical indication / disorder for which such compounds may be used to treat (such as a person suffering from the associated disorder) (See Sehgal et al., Liver as a target for oligonucleotide therapeutics, J. of Hepatology 2013, In Press).

**Table 3**

| The compound of the invention may target a nucleic acid (e.g. mRNA encoding, or miRNA) selected from the groups consisting of | For the treatment of a disease or disorder such as |
|---|---|
| *AAT* | AAT-LivD |
| *ALDH2* | Alcohol dependence |
| HAMP pathway | Anemia or inflammation /CKD |
| *miR-33* | Atherosclerosis |
| *Apo(a)* | Atherosclerosis/high Lp(a) |
| *miR-7* | Liver cancer |
| *miR-378* | Cardiometabolic diseases |
| *miR-21* | Liver cancer |
| *Myc* | Liver cancer |
| *miR-122* | HCV |
| 5'UTR | HCV |
| 5'UTR & NS5B | HCV |
| *NS3* | HCV |
| *TMPRSS6* | Hemochromatosis |
| Antithrombin III | Hemophilia A, B |
| *ApoCIII* | Hypertriglyceridemia |
| *ANGPLT3* | Hyperlipidaemia |
| *MTP* | Hyperlipidaemia |
| *DGAT2* | NASH |
| *ALAS1* | Porphyria |
| Antithrombin III | Rare Bleeding disorders |
| *Serum amyloid A* | SAA-amyloidosis |
| Factor VII | Thrombosis |
| | |
| Growth hormone receptor | Acromegaly |
| *miR-122* | Hepatitis C virus |
| *ApoB-100* | Hypercholesterolemia |
| *ApoCIII* | Hypertriglyceridemia |
| *PCSK9* | Hypercholesterolemia |
| *CRP* | Inflammatory disorders |
| *KSP or VEGF* | Liver cancer |
| *PLK1* | Liver cancer |
| *miR-34* | Liver cancer |
| *FGFR4* | Obesity |
| Factor IXa | Thrombosis |
| Factor XI | Thrombosis |
| *TTR* | TTR amyloidosis |
| *GCCR* | Type 2 diabetes |
| *PTP-1B* | Type 2 diabetes |
| *GCGR* | Cushing's Syndrome |
| Hepatic Glucose 6-Phosphate Transporter-1 | glucose homeostasis, diabetes, type 2 diabetes |

### Sequences

In some embodiments, the oligomers, or first region thereof, comprise a contiguous nucleotide sequence which corresponds to the reverse complement of a nucleotide sequence present in the target nucleic acid (i.e. the sequence which the oligomer targets". Table 3 provides a group of mRNA and miRNA targets which are in pre-clinical or clinical development using oligonucleotide compounds for the associated indication, and are therefore suitable for targeting with the compounds of the present invention.

In some embodiments the target is selected from the group consisting of: miR-122 ,ApoB-100 ,ApoCIII ,PCSK9 ,CRP ,KSP, VEGF ,PLK1 ,miR-34 ,FGFR4 ,Factor IXa ,Factor XI ,TTR ,GCCR ,PTP-1B ,GCGR, AAT ,ALDH2 ,HAMP pathway,miR-33 ,Apo(a) ,miR-7 ,miR-378 ,miR-21 ,Myc ,miR-122 , the HCV genome such as the HCV 5'UTR or HCV NS5B RNA or NS3 RNA ,TMPRSS6 ,Antithrombin III ,ApoCIII ,ANGPLT3 ,MTP ,DGAT2 ,ALAS1 ,Antithrombin III ,Serum amyloid A and Factor VII.

In some embodiments, the contiguous nucleotide sequence comprises no more than a single mismatch when hybridizing to the target sequence. Region B may however be non-complementary and may, optionally, therefore be disregarded when determining the degree of complementarity.

In determining the degree of "complementarity" between oligomers (or regions thereof) and the target region of the nucleic acid, such as those disclosed herein, the degree of "complementarity" (also, "homology" or "identity") is expressed as the percentage identity (or percentage homology) between the sequence of the oligomer (or region thereof) and the sequence of the target region (or the reverse complement of the target region) that best aligns therewith. The percentage is calculated by counting the number of aligned bases that are identical between the 2 sequences, dividing by the total number of contiguous monomers in the oligomer, and multiplying by 100. In such a comparison, if gaps exist, it is preferable that such gaps are merely mismatches rather than areas where the number of monomers within the gap differs between the oligomer of the invention and the target region.

As used herein, the terms "homologous" and "homology" are interchangeable with the terms "identity" and "identical".

The terms "corresponding to" and "corresponds to" refer to the comparison between the nucleotide sequence of the oligomer (*i.e.* the nucleobase or base sequence) or contiguous nucleotide sequence (a first region) and the equivalent contiguous nucleotide sequence of a further sequence selected from either i) a sub-sequence of the reverse complement of the nucleic acid target. Nucleotide analogues are compared directly to their equivalent or corresponding nucleotides. A first sequence which corresponds to a further sequence under i) or ii) typically is identical to that sequence over the length of the first sequence (such as the contiguous nucleotide sequence) or, as described herein may, in some embodiments, is at least 80% homologous to a corresponding sequence, such as at least 85%, at least 90%, at least 91%, at least 92%at least 93%, at least 94%, at least 95%, at least 96% homologous, such as 100% homologous (identical).

The terms "corresponding nucleotide analogue" and "corresponding nucleotide" are intended to indicate that the nucleotide in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

The terms "reverse complement", "reverse complementary" and "reverse complementarity" as used herein are interchangeable with the terms "complement", "complementary" and "complementarity".

The contiguous nucleobase sequence of the oligomer may therefore be complementary to a target, such as those referred to herein.

### Nucleosides and Nucleoside analogues

The term "nucleotide" as used herein, refers to a glycoside comprising a sugar moiety (or analogue thereof), a base moiety and a covalently linked group (linkage group), such as a phosphate or phosphorothioate internucleotide linkage group, and covers both naturally occurring nucleotides, such as DNA or RNA, and non-naturally occurring nucleotides comprising modified sugar and/or base moieties, which are also referred to as "nucleotide analogues" herein. Herein, a single nucleotide (unit) may also be referred to as a monomer or nucleic acid unit.

It will be recognized that in the context of the present invention the term nucleoside and nucleotide are used to refer to both naturally occurring nucleotides/sides, such as DNA and RNA, as well as nucleotide/side analogues.

In field of biochemistry, the term "nucleoside" is commonly used to refer to a glycoside comprising a sugar moiety and a base moiety, and may therefore be used when referring to the nucleotide units, which are covalently linked by the internucleoside linkages between the nucleotides of the oligomer. In the field of biotechnology, the term "nucleotide" is often used to refer to a nucleic acid monomer or unit, and as such in the context of an oligonucleotide may refer to the base - such as the "nucleotide sequence", typically refer to the nucleobase sequence (*i.e.* the presence of the sugar backbone and internucleoside linkages are implicit). Likewise, particularly in the case of oligonucleotides where one or more of the internucleoside linkage groups are modified, the term "nucleotide" may refer to a "nucleoside" for example the term "nucleotide" may be used, even when specifying the presence or nature of the linkages between the nucleosides.

As one of ordinary skill in the art would recognise, the 5' terminal nucleotide of an oligonucleotide does not comprise a 5' internucleoside linkage group, although may or may not comprise a 5' terminal group. Non-naturally occurring nucleotides include nucleotides which have modified sugar moieties, such as bicyclic nucleotides or 2' modified nucleotides, such as 2' substituted nucleotides.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligonucleotide, *i.e.* have no functional effect on the way the oligonucleotide works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell. Specific examples of nucleoside analogues are described by *e.g.* Freier & Altmann; Nucl. Acid Res., 1997, 25, 4429-4443 and Uhlmann; Curr. Opinion in Drug Development, 2000, 3(2), 293-213, and in Scheme 1:

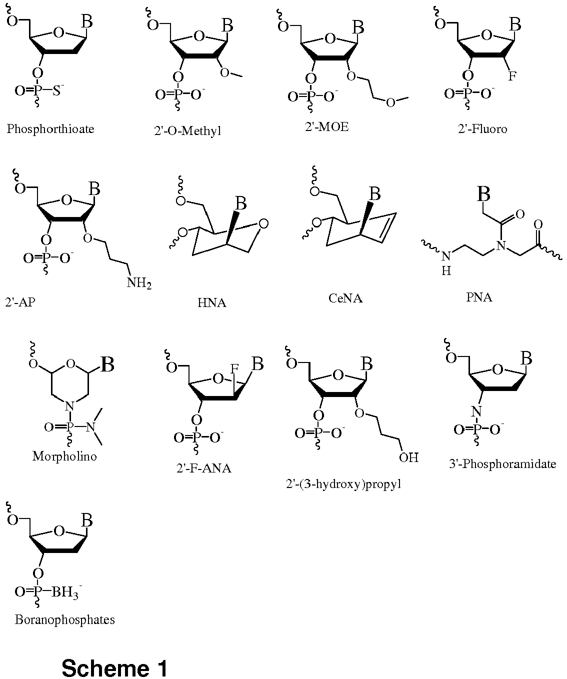

The oligomer may thus comprise or consist of a simple sequence of natural occurring nucleotides - preferably 2'-deoxynucleotides (referred here generally as "DNA"), but also possibly ribonucleotides (referred here generally as "RNA"), or a combination of such naturally occurring nucleotides and one or more non-naturally occurring nucleotides, *i.e.* nucleotide analogues. Such nucleotide analogues may suitably enhance the affinity of the oligomer for the target sequence.

Examples of suitable and preferred nucleotide analogues are provided by WO2007/031091 or are referenced therein. Other nucleotide analogues which may be used in the oligomer of the invention include tricyclic nucleic acids, for example please see WO2013154798 and WO2013154798.

Oligomeric compounds, such as antisense oligonucleotides, such as the compounds referred to herein, including region A, and in some optional embodiments, region B, may contain one or more nucleosides wherein the sugar group has been modified. Such sugar modified nucleosides (nucleoside analogues) may impart enhanced nuclease stability, increased binding affinity, or some other beneficial biological property to the antisense compounds. In some embodiments, nucleosides comprise a chemically modified ribofiiranose ring moiety.

In some embodiments, the oligomer, or first region thereof, comprises at least one , such as at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 or 25 nucleoside analogues, such as sugar modified nucleoside analogues.

In some embodiments the nucleoside analogues are (optionally independently selected from the group consisting of bicyclic nucleoside analogues (such as LNA), and/or 2' substituted nucleoside analogues, such as (optionally independently) selected from the group consisting of 2'-O-alkyl-RNA units, 2'-OMe-RNA units, 2'-amino-DNA units, 2'-AP, 2'-FANA, 2'-(3-hydroxy)propyl, and 2'-fluoro-DNA units, and/or other (optionally) sugar modified nucleoside analogues such as morpholino, peptide nucleic acid (PNA), CeNA, unlinked nucleic acid (UNA), hexitol nucleoic acid (HNA). bicyclo-HNA (see e.g. WO2009/100320), In some embodiments, the nucleoside analogues increase the affinity of the first region for its target nucleic acid (or a complementary DNA or RNA sequence).

In some embodiments, the oligomer comprises at least one bicyclic nucleotide analogue, such as LNA. In some embodiments, the first region comprises of at least one bicyclic nucleoside analogues (e.g. LNA) and/or 2'substituted nucleoside analogues. In some embodiments, the nucleoside analogues present in the oligomer all comprise the same sugar modification. In some embodiments, at least one nucleoside analogue present in the first region is a bicyclic nucleoside analogue, such as at least 2, at least 3, at least 4, at least 5, at least 6, at least 7, at least 8, at least 9, at least 10, at least 11, at least 12, at least 13, at least 14, at least 15, at least 16, for example all nucleoside analogues (except the DNA and or RNA nucleosides of region B) are sugar modified nucleoside analogues, such as such as bicyclic nucleoside analogues, such as LNA, e.g. beta-D-X-LNA or alpha-L-X-LNA (wherein X is oxy, amino or thio), or other LNAs disclosed herein including, but not limited to,(R/S) cET, cMOE or 5'-Me-LNA.

Examples of chemically modified ribofiiranose rings include, without limitation, addition of substituent groups (including 5' and 2' substituent groups); bridging of non-geminal ring atoms to form bicyclic nucleic acids (BNA); replacement of the ribosyl ring oxygen atom with S, N(R), or C(R₁)(R₂) (R = H, C₁ -C₂ alkyl or a protecting group); and combinations thereof. Examples of chemically modified sugars include, 2'-F-5'-methyl substituted nucleoside (see, PCT International Application WO 2008/101157, published on 8/21/08 for other disclosed 5', 2'-bis substituted nucleosides), replacement of the ribosyl ring oxygen atom with S with further substitution at the 2'-position (see, published U.S. Patent Application US2005/0130923, published on June 16, 2005), or, alternatively, 5'-substitution of a BNA (see, PCT International Application WO 2007/134181, published on 11/22/07, wherein LNA is substituted with, for example, a 5'-methyl or a 5'-vinyl group).

Examples of nucleosides having modified sugar moieties include, without limitation, nucleosides comprising 5'-vinyl, 5'-methyl (R or S), 4'-S, 2'-F, 2'-OCH₃, and 2'-O(CH₂)2 O CH₃ substituent groups. The substituent at the 2' position can also be selected from allyl, amino, azido, thio, O-allyl, O-C₁-C₁₀ alkyl, OCF₃, O(CH₂)₂SCH₃, O(CH₂)2-O-N(Rm)(Rn), and O-CH₂-C(=O)-N(Rm)(Rn), where each Rm and Rn is, independently, H or substituted or unsubstituted C₁-C₁₀ alkyl.

As used herein, "bicyclic nucleosides" refer to modified nucleosides comprising a bicyclic sugar moiety. Examples of bicyclic nucleosides include, without limitation, nucleosides comprising a bridge between the 4' and the 2' ribosyl ring atoms. In some embodiments, compounds provided herein include one or more bicyclic nucleosides wherein the bridge comprises a 4' to 2' bicyclic nucleoside. Examples of such 4' to 2' bicyclic nucleosides, include, but are not limited to, one of the formulae: 4'-(CH₂)-O-2' (LNA); 4'-(CH₂)-S-2'; 4-(CH₂)₂-O-2' (ENA); 4'-CH(CH₃)-O-2' and 4'-CH(CH₂OCH₃)-O-2*, and analogs thereof (see, U.S. Patent 7,399,845, issued on July 15, 2008); 4'-C(CH₃)(CH₃)-O-2', and analogs thereof (see, published PCT International Application WO2009/006478, published January 8, 2009); 4'-CH₂-N(OCH₃)-2', and analogs thereof (see, published PCT International Application WO2008/150729, published December 11, 2008); 4'-CH₂-ON(CH₃)-2' (see, published U.S. Patent Application US2004/0171570, published September 2, 2004); 4'-CH₂-N(R)- O -2', wherein R is H, C₁-C₁₀ alkyl, or a protecting group (see, U.S. Patent 7,427,672, issued on September 23, 2008); 4'-CH₂-C(H)(CH₃)-2' (see, Chattopadhyaya, et al, J. Org. Chem.,2009, 74, 118-134); and 4'-CH₂-C(=CH₂)-2', and analogs thereof (see, published PCT International Application WO 2008/154401, published on December 8, 2008). Also see, for example: Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; Singh et al., J. Org. Chem., 1998, 63, 10035-10039; Srivastava et al., J. Am. Chem. Soc, 129(26) 8362-8379 (Jul. 4, 2007); Elayadi et al., Curr. Opinion Invens. Drugs, 2001, 2, 558-561; Braasch et al., Chem. Biol, 2001, 8, 1-7; Oram et al, Curr. Opinion Mol. Ther., 2001, 3, 239-243; U.S. Patent Nos U.S. 6,670,461, 7,053,207, 6,268,490, 6,770,748, 6,794,499, 7,034,133, 6,525,191, 7,399,845; published PCT International applications WO 2004/106356, WO 94/14226, WO 2005/021570, and WO 2007/134181; U.S. Patent Publication Nos. US2004/0171570, US2007/0287831, and US2008/0039618; and U.S. Patent Serial Nos. 12/129,154, 60/989,574, 61/026,995, 61/026,998, 61/056,564, 61/086,231, 61/097,787, and 61/099,844; and PCT International Application Nos. WO08150729, WO08154401, and WO09006478. Each of the foregoing bicyclic nucleosides can be prepared having one or more stereochemical sugar configurations including for example a-L-ribofuranose and beta -D-ribofuranose (see PCT international application PCT DK98/00393, published on March 25, 1999 as WO 99/14226).

In some embodiments, bicyclic sugar moieties of BNA nucleosides include, but are not limited to, compounds having at least one bridge between the 4' and the 2' position of the pentofuranosyl sugar moiety wherein such bridges independently comprises 1 or from 2 to 4 linked groups independently selected from - [CiRₐXR_{b})],,-, -C(Rₐ)=C(R_{b})-, -C(Rₐ)=N-,-C(=NRₐ)-, -C(=O)-, -C(=S)-, - O -, -Si(Rₐ)₂-, -S(=O)ₓ-, and -N(Ra)-; wherein: x is 0, 1, or 2; n is 1, 2, 3, or 4; each Rₐ and R_{b} is, independently, H, a protecting group, hydroxyl, C₁-C₁₂ alkyl, substituted C₁-C₁₂ alkyl, C₂-Ci₂ alkenyl, substituted C₂-C₁₂ alkenyl, C₂-Ci₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)- H), substituted acyl, CN, sulfonyl (S(=O)₂-J₁), or sulfoxyl (S(=O)-J₁); and each J₁ and J₂ is, independently, H, C₁-C₆ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl, or a protecting group.

In some embodiments, the bridge of a bicyclic sugar moiety is, -[C(Rₐ)(Rb)]ₙ-,-[C(Rₐ)(R_{b})]ₙ-O-, -C(RₐR_{b})-N(R)-O- or, -C(RₐR_{b})-O-N(R)-. In some embodiments, the bridge is 4'-CH₂-2', 4'-(CH₂)₂-2', 4'-(CH₂)₃-2', 4'-CH₂-O-2', 4*-(CH₂)2-O-2', 4'-CH₂-ON(R)-2', and 4'-CH₂-N(R)-O-2'-, wherein each R is, independently, H, a protecting group, or C₁-C₁₂ alkyl.

In some embodiments, bicyclic nucleosides are further defined by isomeric configuration. For example, a nucleoside comprising a 4'-2' methylene-oxy bridge, may be in the a-L configuration or in the beta - D configuration. Previously, a-L-methyleneoxy (4'-CH₂-O-2') BNA's have been incorporated into antisense oligonucleotides that showed antisense activity (Frieden et al, Nucleic Acids Research, 2003, 21, 6365- 6372).

In some embodiments, bicyclic nucleosides include, but are not limited to, (A) a-L-Methyleneoxy (4'-CH₂-O-2') BNA, (B) beta -D-Methyleneoxy (4'-CH₂-O-2') BNA, (C) Ethyleneoxy (4'-(CH₂)₂-O-2') BNA, (D) Aminooxy (4'-CH₂-O-N(R)-2') BNA, (E) Oxyamino (4'-CH₂-N(R)-O-2') BNA, (F), Methyl(methyleneoxy) (4'-CH(CH₃)-O-2') BNA, (G) methylene-thio (4'-CH₂-S-2') BNA, (H) methylene- amino (4'-CH₂-N(R)-2') BNA, (I) methyl carbocyclic (4'-CH₂-CH(CH₃)-2') BNA, and (J) propylene carbocyclic (4'-(CH₂)₃-2') BNA as depicted below. 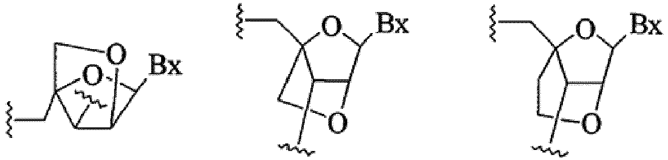 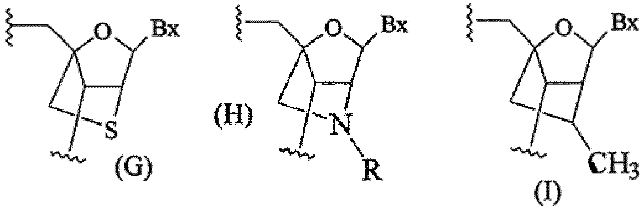 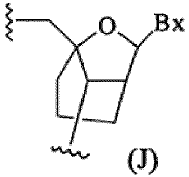 wherein Bx is the base moiety and R is, independently, H, a protecting group or C₁-C₂ alkyl. odiments, bicyclic nucleoside having Formula I: 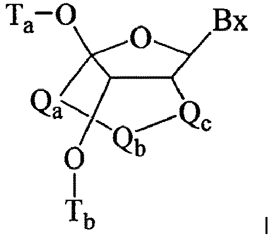 wherein:
Bx is a heterocyclic base moiety;
-Qₐ-Q_{b}-Q_{c}- is -CH₂-N(Rc)-CH₂-, -C(=O)-N(R_{c})-CH₂-, -CH₂-O-N(Rc)-, -CH₂-N(Rc)-O-, or-N(Rc)-O-CH₂;
R_{c} is C₁-C₁₂ alkyl or an amino protecting group; and
Tₐ and T_{b} are each, independently, H, a hydroxyl protecting group, a conjugate group, a reactive phosphorus group, a phosphorus moiety, or a covalent attachment to a support medium.

In some embodiments, bicyclic nucleoside having Formula II: 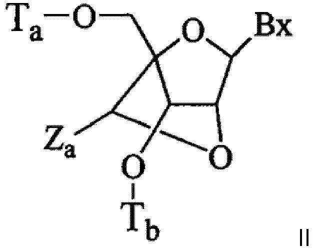 wherein:
Bx is a heterocyclic base moiety;
Tₐ and T_{b} are each, independently, H, a hydroxyl protecting group, a conjugate group, a reactive phosphorus group, a phosphorus moiety, or a covalent attachment to a support medium; Zₐ is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, substituted amide, thiol, or substituted thio.

In some embodiments, each of the substituted groups is, independently, mono or poly substituted with substituent groups independently selected from halogen, oxo, hydroxyl, OJ_{c}, NJ_{d}, SJ_{C}, N₃, OC(=X)J_{c}, and NJₑC(=X)NJ_{c}J_{d}, wherein each J_{c}, J_{d}, and Jₑ is, independently, H, C₁-C₆ alkyl, or substituted C₁-C₆ alkyl and X is O or NJ_{C}.

In some embodiments, bicyclic nucleoside having Formula III: 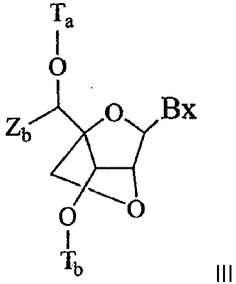 wherein:
Bx is a heterocyclic base moiety;
Tₐ and T_{b} are each, independently, H, a hydroxyl protecting group, a conjugate group, a reactive phosphorus group, a phosphorus moiety, or a covalent attachment to a support medium;
Z_{b} is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, or substituted acyl (C(=O)-).

In some embodiments, bicyclic nucleoside having Formula IV: 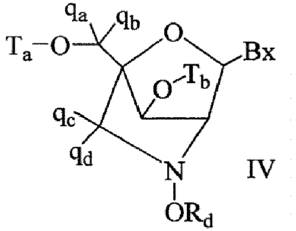 wherein:
Bx is a heterocyclic base moiety;
Tₐ and T_{b} are each, independently H, a hydroxyl protecting group, a conjugate group, a reactive phosphorus group, a phosphorus moiety, or a covalent attachment to a support medium;
R_{d} is C₁-C₆ alkyl, substituted C₁-C₆ alkyl, C₂-C₆ alkenyl, substituted C₂-C₆ alkenyl, C₂-C₆ alkynyl, substituted C₂-C₆ alkynyl; each q_{b}, q_{c} and q_{d} is, independently, H, halogen, C₁-C₆ alkyl, substituted C₁-C₆ alkyl, C₂-Ce alkenyl, substituted C₂-C₆ alkenyl, C₂-C₆ alkynyl, or substituted C₂-C6 alkynyl, C₁-C₆ alkoxyl, substituted Q- C₆ alkoxyl, acyl, substituted acyl, C₁-C₆ aminoalkyl, or substituted C₁-C₆ aminoalkyl;

In some embodiments, bicyclic nucleoside having Formula V: 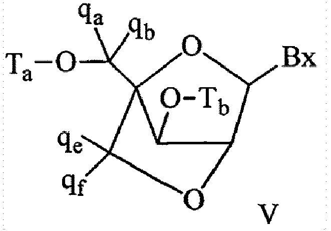 wherein:
Bx is a heterocyclic base moiety;
Tₐ and T_{b} are each, independently, H, a hydroxyl protecting group, a conjugate group, a reactive phosphorus group, a phosphorus moiety, or a covalent attachment to a support medium; qₐ, q_{b}, q_{c} and q_{f} are each, independently, hydrogen, halogen, C₁-C₁₂ alkyl, substituted C₁-C₁₂ alkyl, C₂- C₁₂ alkenyl, substituted C₂-C₁₂ alkenyl, C₂-C₁₂ alkynyl, substituted C₂-C₁₂ alkynyl, C₁-C₁₂ alkoxy, substituted C₁-C₁₂ alkoxy, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ or N(H)C(=S)NJⱼJₖ; or qₑ and q_{f} together are =C(q_{g})(qₕ); q_{g} and qₕ are each, independently, H, halogen, C₁- C₁₂ alkyl, or substituted C₁-C₁₂ alkyl.

The synthesis and preparation of the methyleneoxy (4'-CH₂-O-2') BNA monomers adenine, cytosine, guanine, 5-methyl-cytosine, thymine, and uracil, along with their oligomerization, and nucleic acid recognition properties have been described (see, e.g., Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA, methyleneoxy (4'-CH₂-O-2') BNA, and 2'-thio-BNAs, have also been prepared {see, e.g., Kumar et al., Bioorg. Med. Chem. Lett., 1998, 8, 2219-2222). Preparation of locked nucleoside analogs comprising oligodeoxyribonucleotide duplexes as substrates for nucleic acid polymerases has also been described (see, e.g., Wengel et al., WO 99/14226). Furthermore, synthesis of 2'-amino-BNA, a novel comformationally restricted high-affinity oligonucleotide analog, has been described in the art (see, e.g., Singh et al., J. Org. Chem., 1998, 63, 10035-10039). In addition, 2'-amino- and 2'-methylamino-BNA's have been prepared and the thermal stability of their duplexes with complementary RNA and DNA strands has been previously reported. In some embodiments, the bicyclic nucleoside has Formula VI: 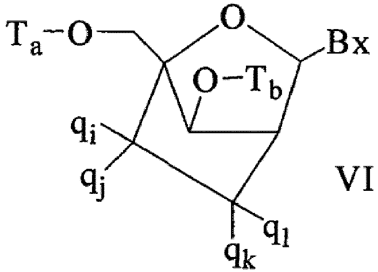 wherein:
Bx is a heterocyclic base moiety;
Tₐ and T_{b} are each, independently, H, a hydroxyl protecting group, a conjugate group, a reactive phosphorus group, a phosphorus moiety, or a covalent attachment to a support medium; each qj, qj, qₖ and ql is, independently, H, halogen, C₁-C₁₂ alkyl, substituted C₁-C₁₂ alkyl, C₂-C12 alkenyl, substituted C₂-C₁₂ alkenyl, C₂-C₁₂ alkynyl, substituted C₂-C₁₂ alkynyl, C₁-C₁₂ alkoxyl, substituted C₂- C₁₂ alkoxyl, OJⱼ, SJⱼ, SOJⱼ, SO₂Jⱼ, NJⱼJₖ, N₃, CN, C(=O)OJⱼ, C(=O)NJⱼJₖ, C(=O)Jⱼ, O-C(=O)NJⱼJₖ, N(H)C(=NH)NJⱼJₖ, N(H)C(=O)NJⱼJₖ, or (H)C(=S)NJⱼJₖ; and qi and qⱼ or ql and qₖ together are =C(q_{g})(qₕ), wherein q_{g} and qₕ are each, independently, H, halogen, C₁-C₁₂ alkyl, or substituted C₁-C₆ alkyl.

One carbocyclic bicyclic nucleoside having a 4'-(CH₂)₃-2' bridge and the alkenyl analog, bridge 4'- CH=CH-CH₂-2', have been described (see, e.g., Freier et al, Nucleic Acids Research, 1997, 25(22), 4429- 4443 and Albaek et al, J. Org. Chem., 2006, 71, 7731-77 '40). The synthesis and preparation of carbocyclic bicyclic nucleosides along with their oligomerization and biochemical studies have also been described (see, e.g., Srivastava et al, J. Am. Chem. Soc. 2007, 129(26), 8362-8379).

As used herein, "4'-2' bicyclic nucleoside" or "4' to 2' bicyclic nucleoside" refers to a bicyclic nucleoside comprising a furanose ring comprising a bridge connecting the 2' carbon atom and the 4' carbon atom.

As used herein, "monocylic nucleosides" refer to nucleosides comprising modified sugar moieties that are not bicyclic sugar moieties. In some embodiments, the sugar moiety, or sugar moiety analogue, of a nucleoside may be modified or substituted at any position. As used herein, "2'-modified sugar" means a furanosyl sugar modified at the 2' position. In some embodiments, such modifications include substituents selected from: a halide, including, but not limited to substituted and unsubstituted alkoxy, substituted and unsubstituted thioalkyl, substituted and unsubstituted amino alkyl, substituted and unsubstituted alkyl, substituted and unsubstituted allyl, and substituted and unsubstituted alkynyl. In some embodiments, 2' modifications are selected from substituents including, but not limited to: O[(CH₂)ₙO]ₘCH₃, O(CH₂),,NH₂, O(CH₂),,CH₃, O(CH₂),,ONH₂, OCH₂C(=O)N(H)CH₃, and O(CH2)ₙON[(CH₂)ₙCH₃]2, where n and m are from 1 to about 10. Other 2'- substituent groups can also be selected from: C₁-C₁₂ alkyl; substituted alkyl; alkenyl; alkynyl; alkaryl; aralkyl; O-alkaryl or O-aralkyl; SH; SCH₃; OCN; CI; Br; CN; CF₃; OCF₃; SOCH₃; SO₂CH₃; ONO₂; NO₂; N₃; NH₂; heterocycloalkyl; heterocycloalkaryl; aminoalkylamino; polyalkylamino; substituted silyl; an R; a cleaving group; a reporter group; an intercalator; a group for improving pharmacokinetic properties; and a group for improving the pharmacodynamic properties of an antisense compound, and other substituents having similar properties. In some embodiments, modified nucleosides comprise a 2'-MOE side chain {see, e.g., Baker et al., J. Biol. Chem., 1997, 272, 1 1944-12000). Such 2'-MOE substitution have been described as having improved binding affinity compared to unmodified nucleosides and to other modified nucleosides, such as 2'- O- methyl, O-propyl, and O-aminopropyl. Oligonucleotides having the 2 -MOE substituent also have been shown to be antisense inhibitors of gene expression with promising features for in vivo use {see, e.g., Martin, P., He/v. Chim. Acta, 1995, 78, 486-504; Altmann et al., Chimia, 1996, 50, 168-176; Altmann et al., Biochem. Soc. Trans., 1996, 24, 630-637; and Altmann et al., Nucleosides Nucleotides, 1997, 16, 917-926).

As used herein, a "modified tetrahydropyran nucleoside" or "modified THP nucleoside" means a nucleoside having a six-membered tetrahydropyran "sugar" substituted in for the pentofuranosyl residue in normal nucleosides (a sugar surrogate). Modified ?THP nucleosides include, but are not limited to, what is referred to in the art as hexitol nucleic acid (HNA), anitol nucleic acid (ANA), manitol nucleic acid (MNA) {see Leumann, CJ. Bioorg. and Med. Chem. (2002) 10:841-854), fluoro HNA (F-HNA), or those compounds having Formula X: 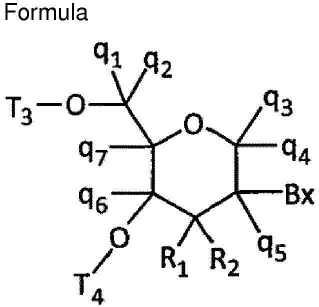 X wherein independently for each of said at least one tetrahydropyran nucleoside analog of Formula X:
Bx is a heterocyclic base moiety;
T₃ and T₄ are each, independently, an internucleoside linking group linking the tetrahydropyran nucleoside analog to the antisense compound or one of T₃ and T₄ is an internucleoside linking group linking the tetrahydropyran nucleoside analog to the antisense compound and the other of T₃ and T4 is H, a hydroxyl protecting group, a linked conjugate group, or a 5' or 3'-terminal group; q₁ q₂ q₃ q₄ q₅, q₆ and q₇ are each, independently, H, C₁-C₆ alkyl, substituted C₁-C₆ alkyl, C₂-C₆ alkenyl, substituted C₂-C₆ alkenyl, C₂-C₆ alkynyl, or substituted C₂-C₆ alkynyl; and one of R₁ and R₂ is hydrogen and the other is selected from halogen, substituted or unsubstituted alkoxy, NJ,J₂, SJ,, N₃, OC(=X)J₁, OC(=X)NJ₁J₂, NJ₃C(=X) NJ₁J₂, and CN, wherein X is O, S, or NJ₁ and each J₁, J₂, and J₃ is, independently, H or C₁-C₆ alkyl.

In some embodiments, the modified THP nucleosides of Formula X are provided wherein qₘ, qₙ, qₚ, qᵣ, qₛ, qₜ, and qᵤ are each H. In some embodiments, at least one of qₘ, qₙ, qₚ, qᵣ, qₛ, qₜ and qᵤ is other than H. In some embodiments, at least one of qₘ, qₙ, qₚ, qᵣ, qₛ, qₜ and qᵤ is methyl. In some embodiments, THP nucleosides of Formula X are provided wherein one of R₁ and R₂ is F. In some embodiments, R₁ is fluoro and R₂ is H, R₁ is methoxy and R₂ is H, and R₁ is methoxyethoxy and R₂ is H.

As used herein, "2'-modified" or "2'-substituted" refers to a nucleoside comprising a sugar comprising a substituent at the 2' position other than H or OH. 2'-modified nucleosides, include, but are not limited to nucleosides with non-bridging 2'substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O- N(Rₘ)(Rₙ), or O-CH₂-C(=O)-N(Rₘ)(Rₙ), where each Rₘ and R,, is, independently, H or substituted or unsubstituted C₁-C₁₀ alkyl. 2'-modifed nucleosides may further comprise other modifications, for example, at other positions of the sugar and/or at the nucleobase.

As used herein, "2'-F" refers to a sugar comprising a fluoro group at the 2' position.

As used herein, "2'-OMe" or "2'-OCH₃" or "2'-O-methyl" each refers to a nucleoside comprising a sugar comprising an -OCH₃ group at the 2' position of the sugar ring.

As used herein, "oligonucleotide" refers to a compound comprising a plurality of linked nucleosides.

In some embodiments, one or more of the plurality of nucleosides is modified. In some embodiments, an oligonucleotide comprises one or more ribonucleosides (RNA) and/or deoxyribonucleosides (DNA).

Many other bicyclo and tricyclo sugar surrogate ring systems are also known in the art that can be used to modify nucleosides for incorporation into antisense compounds {see, e.g., review article: Leumann, J. C, Bioorganic and Medicinal Chemistry, 2002, 10, 841-854). Such ring systems can undergo various additional substitutions to enhance activity. Methods for the preparations of modified sugars are well known to those skilled in the art. In nucleotides having modified sugar moieties, the nucleobase moieties (natural, modified, or a combination thereof) are maintained for hybridization with an appropriate nucleic acid target.

In some embodiments, antisense compounds comprise one or more nucleotides having modified sugar moieties. In some embodiments, the modified sugar moiety is 2'-MOE. In some embodiments, the 2'-MOE modified nucleotides are arranged in a gapmer motif. In some embodiments, the modified sugar moiety is a cEt. In some embodiments, the cEt modified nucleotides are arranged throughout the wings of a gapmer motif.

In some embodiments, in the BNA (LNA), R^{4*} and R^{2*} together designate the biradical -O-CH(CH₂OCH₃)- (2'O-methoxyethyl bicyclic nucleic acid - Seth at al., 2010, J. Org. Chem) - in either the R- or S- configuration.

In some embodiments, in the BNA (LNA), R^{4*} and R^{2*} together designate the biradical -O-CH(CH₂CH₃)- (2'O-ethyl bicyclic nucleic acid - Seth at al., 2010, J. Org. Chem). - in either the R- or S- configuration.

In some embodiments, in the BNA (LNA), R^{4*} and R^{2*} together designate the biradical -O-CH(CH₃)-. - in either the R- or S- configuration. In some embodiments, R^{4*} and R^{2*} together designate the biradical -O-CH₂-O-CH₂- - (Seth at al., 2010, J. Org. Chem).

In some embodiments, in the BNA (LNA), R^{4*} and R^{2*} together designate the biradical -O-NR-CH₃-- (Seth at al., 2010, J. Org. Chem) .

In some embodiments, the LNA units have a structure selected from the following group:

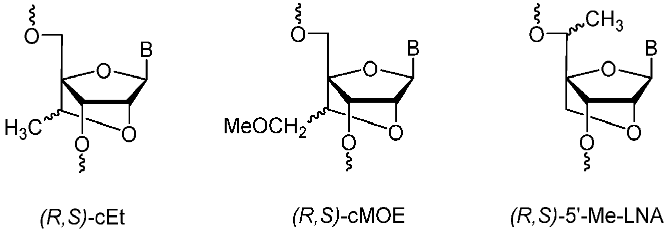

We have evaluated the nephrotoxicity of a cET compound (using (S)-cET, with the sequence (Compound ID 6/411847 of WO2009/12495 and a comparative beta-D-oxy LNA compound (6/392063 of WO2009/12495) and found that the cET compounds elicit surprisingly high nephrotoxicity as compared to the beta-D-oxy LNA control. The study was a single dose study, with sacrifice after 3 days (see EP1984381 example 41 for the methodology, although we used NMRI mice). Nephrotoxicity was confirmed by histological analysis. Notably signs of nephrotoxicity we seen at dosages of the cET compound below those where serum ALT was noted, indicating that for cET compounds, nephrotoxicity may be a particular problem. The use of the conjugates of the present invention, such as trivalent GalNAc conjugates are therefore highly useful in reducing the nephrotoxicity of LNA compounds, such as cET compounds.

The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, *i.e.* nucleotide analogues. Such nucleotide analogues may suitably enhance the affinity of the oligomer for the target sequence.

Incorporation of affinity-enhancing nucleotide analogues in the oligomer, such as BNA, (e.g.) LNA or 2'-substituted sugars, can allow the size of the specifically binding oligomer to be reduced, and may also reduce the upper limit to the size of the oligomer before nonspecific or aberrant binding takes place.

In some embodiments, the oligomer of the LNA antisense oligomer conjugate comprises at least 1 nucleoside analogue. In some embodiments the oligomer comprises at least 2 nucleotide analogues. In some embodiments, the oligomer comprises from 3-8 nucleotide analogues, *e.g.* 6 or 7 nucleotide analogues. In the by far most preferred embodiments, at least one of said nucleotide analogues is a BNA, such as locked nucleic acid (LNA); for example at least 3 or at least 4, or at least 5, or at least 6, or at least 7, or 8, of the nucleotide analogues may be BNA, such as LNA. In some embodiments all the nucleotides analogues may be BNA, such as LNA.

It will be recognised that when referring to a preferred nucleotide sequence motif or nucleotide sequence, which consists of only nucleotides, the oligomers of the invention which are defined by that sequence may comprise a corresponding nucleotide analogue in place of one or more of the nucleotides present in said sequence, such as BNA units or other nucleotide analogues, which raise the duplex stability/Tₘ of the oligomer/target duplex (*i.e.* affinity enhancing nucleotide analogues).

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA).

In some embodiments the further nucleotide analogues present within the oligomer part of the LNA antisense oligomer conjugate of the invention are independently selected from, for example: 2'-O-alkyl-RNA units, 2'-amino-DNA units, 2'-fluoro-DNA units, BNA units, e.g. LNA units, arabino nucleic acid (ANA) units, 2'-fluoro-ANA units, HNA units, INA (intercalating nucleic acid -Christensen, 2002. Nucl. Acids. Res. 2002 30: 4918-4925) units and 2'MOE units. In some embodiments there is only one of the above types of nucleotide analogues present in the oligomer of the invention, such as the first region, or contiguous nucleotide sequence thereof.

In some embodiments the further nucleotide analogues are 2'-O-methoxyethyl-RNA (2'MOE), 2'-fluoro-DNA monomers or LNA nucleotide analogues, and as such the oligonucleotide of the invention may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments at least one of said nucleotide analogues is 2'-fluoro DNA, such as 2, 3, 4, 5, 6, 7, 8, 9 or 10 2'-fluoro-DNA nucleotide units.

The oligomer part of the LNA antisense oligomer conjugate according to the invention comprises at least one BNA, e.g. Locked Nucleic Acid (LNA) unit, such as 1, 2, 3, 4, 5, 6, 7, or 8 BNA/LNA units, such as from 3 - 7 or 4 to 8 BNA/ LNA units, or 3, 4, 5, 6 or 7 BNA/LNA units. In some embodiments, all the nucleotide analogues are BNA, such as LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In some embodiments all BNA, such as LNA, cytosine units are 5'methyl-Cytosine. In some embodiments of the invention, the oligomer (such as the first and optionally second regions) may comprise both BNA and LNA and DNA units. In some embodiments, the combined total of LNA and DNA units is 10-25, such as 10 - 24, preferably 10-20, such as 10 - 18, such as 12-16. In some embodiments of the invention, the nucleotide sequence of the oligomer, of first region thereof, such as the contiguous nucleotide sequence consists of at least one BNA, e.g. LNA and the remaining nucleotide units are DNA units. In some embodiments the oligomer, or first region thereof, comprises only BNA, e.g. LNA, nucleotide analogues and naturally occurring nucleotides (such as RNA or DNA, most preferably DNA nucleotides), optionally with modified internucleotide linkages such as phosphorothioate.

The term "nucleobase" refers to the base moiety of a nucleotide and covers both naturally occurring a well as non-naturally occurring variants. Thus, "nucleobase" covers not only the known purine and pyrimidine heterocycles but also heterocyclic analogues and tautomeres thereof. It will be recognised that the DNA or RNA nucleosides of region B may have a naturally occurring and/or non-naturally occurring nucleobase(s), such as DNA nucleobases independently selected from the group A, C, T and G, or the group C, T and G.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine. In some embodiments the nucleobases may be independently selected from the group consisting of adenine, guanine, cytosine, thymidine, uracil, 5-methylcytosine. In some embodiments the nucleobases may be independently selected from the group consisting of adenine, guanine, cytosine, thymidine, and 5-methylcytosine.

In some embodiments, at least one of the nucleobases present in the oligomer is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleoside analogue which comprises a C2* - C4* biradical (a bridge), and is known as "Locked Nucleic Acid". It may refer to an LNA monomer, or, when used in the context of an "LNA oligonucleotide", LNA refers to an oligonucleotide containing one or more such bicyclic nucleotide analogues. In some aspects bicyclic nucleoside analogues are LNA nucleotides, and these terms may therefore be used interchangeably, and is such embodiments, both are be characterised by the presence of a linker group (such as a bridge) between C2' and C4' of the ribose sugar ring.

In some embodiments the LNA used in the oligonucleotide compounds of the invention preferably has the structure of the general formula II: 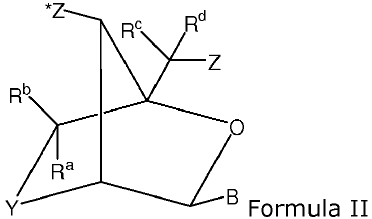 wherein Y is selected from the group consisting of -O-, -CH₂O-, -S-, -NH-, N(R^{e}) and/or-CH₂-; Z and Z* are independently selected among an internucleotide linkage, R^{H}, a terminal group or a protecting group; B constitutes a natural or non-natural nucleotide base moiety (nucleobase), and R^{H} is selected from hydrogen and C₁₋₄-alkyl; R^{a}, R^{b} R^{c}, R^{d} and R^{e} are, optionally independently, selected from the group consisting of hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkoxyalkyl, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxycarbonyl, heteroaryloxy, heteroarylcarbonyl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted and where two geminal substituents R^{a} and R^{b} together may designate optionally substituted methylene (=CH₂); and R^{H} is selected from hydrogen and C₁₋₄-alkyl. In some embodiments R^{a}, R^{b} R^{c}, R^{d} and R^{e} are, optionally independently, selected from the group consisting of hydrogen and C₁₋₆ alkyl, such as methyl. For all chiral centers, asymmetric groups may be found in either *R* or *S* orientation, for example, two exemplary stereochemical isomers include the beta-D and alpha-L isoforms, which may be illustrated as follows:

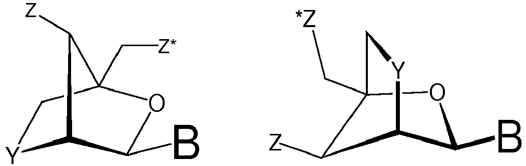

Specific exemplary LNA units are shown below:

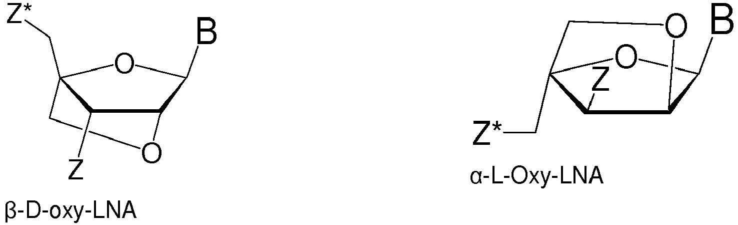

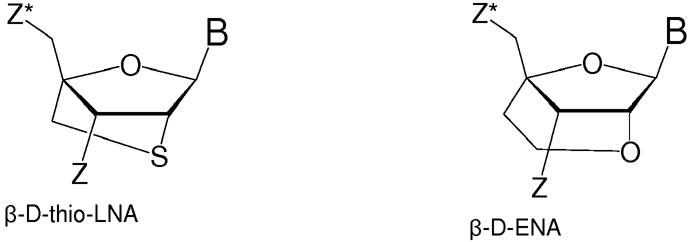

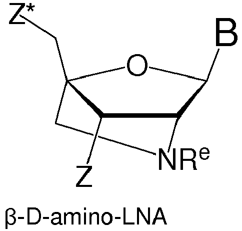

The term "thio-LNA" comprises a locked nucleotide in which Y in the general formula above is selected from S or -CH₂-S-. Thio-LNA can be in both beta-D and alpha-L-configuration.

The term "amino-LNA" comprises a locked nucleotide in which Y in the general formula above is selected from -N(H)-, N(R)-, CH₂-N(H)-, and -CH₂-N(R)- where R is selected from hydrogen and C₁₋₄-alkyl. Amino-LNA can be in both beta-D and alpha-L-configuration.

The term "oxy-LNA" comprises a locked nucleotide in which Y in the general formula above represents -O-. Oxy-LNA can be in both beta-D and alpha-L-configuration.

The term "ENA" comprises a locked nucleotide in which Y in the general formula above is -CH₂-O- (where the oxygen atom of -CH₂-O- is attached to the 2'-position relative to the base B). R^{e} is hydrogen or methyl.

In some exemplary embodiments LNA is selected from beta-D-oxy-LNA, alpha-L-oxy-LNA, beta-D-amino-LNA and beta-D-thio-LNA, in particular beta-D-oxy-LNA.

### RNAse recruitment

It is recognised that an oligomeric compound may function via non RNase mediated degradation of target mRNA, such as by steric hindrance of translation, or other methods, In some embodiments, the oligomers of the invention are capable of recruiting an endoribonuclease (RNase), such as RNase H.

It is preferable such oligomers, such as region A, or contiguous nucleotide sequence, comprises of a region of at least 6, such as at least 7 consecutive nucleotide units, such as at least 8 or at least 9 consecutive nucleotide units (residues), including 7, 8, 9, 10, 11, 12, 13, 14, 15 or 16 consecutive nucleotides, which, when formed in a duplex with the complementary target RNA is capable of recruiting RNase. The contiguous sequence which is capable of recruiting RNAse may be region Y' as referred to in the context of a gapmer as described herein. In some embodiments the size of the contiguous sequence which is capable of recruiting RNAse, such as region Y', may be higher, such as 10, 11, 12, 13, 14, 15, 16, 17, 18, 19 or 20 nucleotide units.

EP 1 222 309 provides *in vitro* methods for determining RNaseH activity, which may be used to determine the ability to recruit RNaseH. A oligomer is deemed capable of recruiting RNase H if, when provided with the complementary RNA target, it has an initial rate, as measured in pmol/l/min, of at least 1 %, such as at least 5%, such as at least 10% or ,more than 20% of the of the initial rate determined using DNA only oligonucleotide, having the same base sequence but containing only DNA monomers, with no 2' substitutions, with phosphorothioate linkage groups between all monomers in the oligonucleotide, using the methodology provided by Example 91 - 95 of EP 1 222 309.

In some embodiments, an oligomer part of the LNA antisense oligomer conjugate is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

In other embodiments, an oligomer part of the LNA antisense oligomer conjugate is deemed capable of recruiting RNaseH if, when provided with the complementary RNA target, and RNaseH, the RNaseH initial rate, as measured in pmol/l/min, is at least 20%, such as at least 40 %, such as at least 60 %, such as at least 80 % of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

Typically the region of the oligomer which forms the consecutive nucleotide units which, when formed in a duplex with the complementary target RNA is capable of recruiting RNase consists of nucleotide units which form a DNA/RNA like duplex with the RNA target. The oligomer of the invention, such as the first region, may comprise a nucleotide sequence which comprises both nucleotides and nucleotide analogues, and may be e.g. in the form of a gapmer, a headmer or a mixmer.

A "headmer" is defined as an oligomer that comprises a region X' and a region Y' that is contiguous thereto, with the 5'-most monomer of region Y' linked to the 3'-most monomer of region X'. Region X' comprises a contiguous stretch of non-RNase recruiting nucleoside analogues and region Y' comprises a contiguous stretch (such as at least 7 contiguous monomers) of DNA monomers or nucleoside analogue monomers recognizable and cleavable by the RNase.

A "tailmer" is defined as an oligomer that comprises a region X' and a region Y' that is contiguous thereto, with the 5'-most monomer of region Y linked to the 3'-most monomer of the region X'. Region X' comprises a contiguous stretch (such as at least 7 contiguous monomers) of DNA monomers or nucleoside analogue monomers recognizable and cleavable by the RNase, and region X' comprises a contiguous stretch of non-RNase recruiting nucleoside analogues.

Other "chimeric" oligomers, called "mixmers", consist of an alternating composition of (i) DNA monomers or nucleoside analogue monomers recognizable and cleavable by RNase, and (ii) non-RNase recruiting nucleoside analogue monomers.

In some embodiments, in addition to enhancing affinity of the oligomer for the target region, some nucleoside analogues also mediate RNase (*e.g.*, RNaseH) binding and cleavage. Since α-L-LNA (BNA) monomers recruit RNaseH activity to a certain extent, in some embodiments, gap regions (*e.g.,* region Y' as referred to herein) of oligomers containing α-L-LNA monomers consist of fewer monomers recognizable and cleavable by the RNaseH, and more flexibility in the mixmer construction is introduced.

### Gapmer Design

In some embodiments, the oligomer part of the LNA antisense oligomer conjugate of the invention, such as the first region, comprises or is a gapmer. A gapmer oligomer is an oligomer which comprises a contiguous stretch of nucleotides which is capable of recruiting an RNAse, such as RNAseH, such as a region of at least 6 or 7 DNA nucleotides, referred to herein in as region Y' (Y'), wherein region Y' is flanked both 5' and 3' by regions of affinity enhancing nucleotide analogues, such as from 1 - 6 nucleotide analogues 5' and 3' to the contiguous stretch of nucleotides which is capable of recruiting RNAse - these regions are referred to as regions X' (X') and Z' (Z') respectively. Examples of gapmers are disclosed in WO2004/046160, WO2008/113832, and WO2007/146511.

In some embodiments, the monomers which are capable of recruiting RNAse are selected from the group consisting of DNA monomers, alpha-L-LNA monomers, C4' alkylayted DNA monomers (see PCT International application WO/2009/090182 and Vester et al., Bioorg. Med. Chem. Lett. 18 (2008) 2296 - 2300), and UNA (unlinked nucleic acid) nucleotides (see Fluiter et al., Mol. Biosyst., 2009, 10, 1039). UNA is unlocked nucleic acid, typically where the C2 - C3 C-C bond of the ribose has been removed, forming an unlocked "sugar" residue. Preferably the gapmer comprises a (poly)nucleotide sequence of formula (5' to 3'), X'-Y'-Z', wherein; region X' (X') (5' region) consists or comprises of at least one nucleotide analogue, such as at least one BNA (e.g. LNA) unit, such as from 1-6 nucleotide analogues, such as BNA (e.g. LNA) units, and; region Y' (Y') consists or comprises of at least five consecutive nucleotides which are capable of recruiting RNAse (when formed in a duplex with a complementary RNA molecule, such as the mRNA target), such as DNA nucleotides, and; region Z' (Z') (3'region) consists or comprises of at least one nucleotide analogue, such as at least one BNA (e.gLNA unit), such as from 1-6 nucleotide analogues, such as BNA (e.g. LNA) units.

In some embodiments, region X' consists of 1, 2, 3, 4, 5 or 6 nucleotide analogues, such as BNA (e.g. LNA) units, such as from 2-5 nucleotide analogues, such as 2-5 LNA units, such as 3 or 4 nucleotide analogues, such as 3 or 4 LNA units; and/or region Z' consists of 1, 2, 3, 4, 5 or 6 nucleotide analogues, such as BNA (e.g. LNA) units, such as from 2-5 nucleotide analogues, such as 2-5 BNA (e.g. LNA units), such as 3 or 4 nucleotide analogues, such as 3 or 4 BNA (e.g. LNA) units.

In some embodiments Y' consists or comprises of 5, 6, 7, 8, 9, 10, 11 or 12 consecutive nucleotides which are capable of recruiting RNAse, or from 6-10, or from 7-9, such as 8 consecutive nucleotides which are capable of recruiting RNAse. In some embodiments region Y' consists or comprises at least one DNA nucleotide unit, such as 1-12 DNA units, preferably from 4-12 DNA units, more preferably from 6-10 DNA units, such as from 7-10 DNA units, most preferably 8, 9 or 10 DNA units.

In some embodiments region X' consist of 3 or 4 nucleotide analogues, such as BNA (e.g. LNA), region X' consists of 7, 8, 9 or 10 DNA units, and region Z' consists of 3 or 4 nucleotide analogues, such as BNA (e.g. LNA). Such designs include (X'-Y'-Z') 3-10-3, 3-10-4, 4-10-3, 3-9-3, 3-9-4, 4-9-3, 3-8-3, 3-8-4, 4-8-3, 3-7-3, 3-7-4, 4-7-3.

Further gapmer designs are disclosed in WO2004/046160. WO2008/113832, which claims priority from US provisional application 60/977,409, refers to 'shortmer' gapmer oligomers. In some embodiments, oligomers presented here may be such shortmer gapmers.

In some embodiments the oligomer, e.g. region X', is consisting of a contiguous nucleotide sequence of a total of 10, 11, 12, 13 or 14 nucleotide units, wherein the contiguous nucleotide sequence comprises or is of formula (5' - 3'), X'-Y'-Z' wherein; X' consists of 1, 2 or 3 nucleotide analogue units, such as BNA (e.g. LNA) units; Y' consists of 7, 8 or 9 contiguous nucleotide units which are capable of recruiting RNAse when formed in a duplex with a complementary RNA molecule (such as a mRNA target); and Z' consists of 1, 2 or 3 nucleotide analogue units, such as BNA (e.g. LNA) units.

In some embodiments X' consists of 1 BNA (e.g. LNA) unit. In some embodiments X' consists of 2 BNA (e.g. LNA) units. In some embodiments X' consists of 3 BNA (e.g. LNA) units. In some embodiments Z' consists of 1 BNA (e.g. LNA) units. In some embodiments Z' consists of 2 BNA (e.g. LNA) units. In some embodiments Z' consists of 3 BNA (e.g. LNA) units. In some embodiments Y' consists of 7 nucleotide units. In some embodiments Y' consists of 8 nucleotide units. In some embodiments Y' consists of 9 nucleotide units.. In certain embodiments, region Y' consists of 10 nucleoside monomers. In certain embodiments, region Y' consists or comprises 1 - 10 DNA monomers. In some embodiments Y' comprises of from 1 - 9 DNA units, such as 2, 3, 4, 5, 6, 7 , 8 or 9 DNA units. In some embodiments Y' consists of DNA units. In some embodiments Y' comprises of at least one BNA unit which is in the alpha-L configuration, such as 2, 3, 4, 5, 6, 7, 8 or 9 LNA units in the alpha-L-configuration. In some embodiments Y' comprises of at least one alpha-L-oxy BNA/LNA unit or wherein all the LNA units in the alpha-L- configuration are alpha-L-oxy LNA units. In some embodiments the number of nucleotides present in X'-Y'-Z' are selected from the group consisting of (nucleotide analogue units - region Y' - nucleotide analogue units): 1-8-1, 1-8-2, 2-8-1, 2-8-2, 3-8-3, 2-8-3, 3-8-2, 4-8-1, 4-8-2, 1-8-4, 2-8-4, or; 1-9-1, 1-9-2, 2-9-1, 2-9-2, 2-9-3, 3-9-2, 1-9-3, 3-9-1, 4-9-1, 1-9-4, or; 1-10-1, 1-10-2, 2-10-1, 2-10-2, 1-10-3, 3-10-1. In some embodiments the number of nucleotides in X'-Y'-Z' are selected from the group consisting of: 2-7-1, 1-7-2, 2-7-2, 3-7-3, 2-7-3, 3-7-2, 3-7-4, and 4-7-3. In certain embodiments, each of regions X' and Y' consists of three BNA (e.g. LNA) monomers, and region Y' consists of 8 or 9 or 10 nucleoside monomers, preferably DNA monomers. In some embodiments both X' and Z' consists of two BNA (e.g. LNA) units each, and Y' consists of 8 or 9 nucleotide units, preferably DNA units. In various embodiments, other gapmer designs include those where regions X' and/or Z' consists of 3, 4, 5 or 6 nucleoside analogues, such as monomers containing a 2'-O-methoxyethyl-ribose sugar (2'-MOE) or monomers containing a 2'-fluoro-deoxyribose sugar, and region Y' consists of 8, 9, 10, 11 or 12 nucleosides, such as DNA monomers, where regions X'-Y'-Z' have 3-9-3, 3-10-3, 5-10-5 or 4-12-4 monomers. Further gapmer designs are disclosed in WO 2007/146511A2.

BNA and LNA Gapmers: The terms BNA and LNA are used interchangeably. A BNA gapmer is a gapmer oligomer (region A) which comprises at least one BNA nucleotide. A LNA gapmer is a gapmer oligomer (region A) which comprises at least one LNA nucleotide.

### Splice switching oligomers

In some embodiments, the antisense oligonucleotide part of the LNA antisense oligomer conjugate is a splice switching oligomer - *i.e.* an oligomer which targets the pre-mRNA causing an alternative splicing of the pre-mRNA.

Targets for the splice switching oligomer may include TNF receptor, for example the SSO may be one or more of the TNFR SSOs disclosed in WO2007/058894, WO08051306 A1 and PCT international application WO/2008/131807.

Splice switching oligomers are typically(essentially) not capable of recruiting RNaseH and as such gapmer, tailmer or headmer designs are generally not desirable. However, mixmer and totalmers designs are suitable designs for SSOs.

Spice switching oligomers have also been used to target dystrophin deficiency in Duchenne muscular dystrophy.

### Mixmers

Most antisense oligonucleotides are compounds which are designed to recruit RNase enzymes (such as RNaseH) to degrade their intended target. Such compounds include DNA phosphorothioate oligonucleotides and gapmer, headmers and tailmers. These compounds typically comprise a region of at least 5 or 6 DNA nucleotides, and in the case of gapmers are flanked on either side by affinity enhancing nucleotide analogues. The oligomers of the present invention may operate via an RNase (such as RNaseH) independent mechanism. Examples of oligomers which operate via a non-RNaseH (or non-RNase) mechanism are mixmers and totalmers.

The term 'mixmer' refers to oligomers which comprise both naturally and non-naturally occurring nucleotides, where, as opposed to gapmers, tailmers, and headmers there is no contiguous sequence of more than 5, and in some embodiments no more than 4 consecutive, such as no more than three consecutive, naturally occurring nucleotides, such as DNA units. In some embodiments, the mixmer does not comprise more than 5 consecutive nucleoside analogues, such as BNA (LNA), and in some embodiments no more than 4 consecutive, such as no more than three consecutive, consecutive nucleoside analogues, such as BNA (LNA). In such mixmers the remaining nucleosides may, for example be DNA nucleosides, and/or in non-bicyclic nucleoside analogues, such as those referred to herein, for example, 2' substituted nucleoside analogues, such as 2'-O-MOE and or 2'fluoro.

The oligomer part of the LNA antisense oligomer conjugate according to the invention may be mixmers - indeed various mixmer designs are highly effective as oligomer or first region thereof, particularly when targeting microRNA (antimiRs), microRNA binding sites on mRNAs (Blockmirs) or as splice switching oligomers (SSOs). See for example WO2007/112754 (LNA-AntimiRs™), WO2008/131807 (LNA splice switching oligos),

In some embodiments, the oligomer or mixmer may comprise of BNA and 2' substituted nucleoside analogues, optionally with DNA nucleosides - see for example see WO07027894 and WO2007/112754. Specific examples include oligomers or first regions which comprise LNA, 2'-O-MOE and DNA, LNA, 2'fluoro and 2'-O-MOE, 2'-O-MOE and 2'fluoro, 2'-O-MOE and 2'fluoro and LNA, or LNA and 2'-O-MOE and LNA and DNA.

In some embodiments, the oligomer or mixmer comprises or consists of a contiguous nucleotide sequence of repeating pattern of nucleotide analogue and naturally occurring nucleotides, or one type of nucleotide analogue and a second type of nucleotide analogues. The repeating pattern, may, for instance be every second or every third nucleotide is a nucleotide analogue, such as BNA (LNA), and the remaining nucleotides are naturally occurring nucleotides, such as DNA, or are a 2'substituted nucleotide analogue such as 2'MOE of 2'fluoro analogues as referred to herein, or, in some embodiments selected form the groups of nucleotide analogues referred to herein. It is recognised that the repeating pattern of nucleotide analogues, such as LNA units, may be combined with nucleotide analogues at fixed positions - e.g. at the 5' or 3' termini.

In some embodiments the first nucleotide of oligomer or mixmer, counting from the 3' end, is a nucleotide analogue, such as an LNA nucleotide.

In some embodiments, which maybe the same or different, the second nucleotide of the oligomer or mixmer, counting from the 3' end, is a nucleotide analogue, such as an LNA nucleotide.

In some embodiments, which maybe the same or different, the seventh and/or eighth nucleotide of the oligomer or mixmer counting from the 3' end, are nucleotide analogues, such as LNA nucleotides. In some embodiments, which maybe the same or different, the ninth and/or the tenth nucleotides of the oligomer or mixmer, counting from the 3' end, are nucleotide analogues, such as LNA nucleotides.

In some embodiments, which maybe the same or different, the 5' terminal of oligomer or mixmer is a nucleotide analogue, such as an LNA nucleotide.

The above design features may, in some embodiments be incorporated into the mixmer design, such as antimiR mixmers.

In some embodiments, the oligomer or mixmer does not comprise a region of more than 4 consecutive DNA nucleotide units or 3 consecutive DNA nucleotide units. In some embodiments, the mixmer does not comprise a region of more than 2 consecutive DNA nucleotide units.

In some embodiments, the oligomer or mixmer comprises at least a region consisting of at least two consecutive nucleotide analogue units, such as at least two consecutive LNA units.

In some embodiments, the oligomer or mixmer comprises at least a region consisting of at least three consecutive nucleotide analogue units, such as at least three consecutive LNA units.

In some embodiments, the oligomer or mixmer of the invention does not comprise a region of more than 7 consecutive nucleotide analogue units, such as LNA units. In some embodiments, the oligomer or mixmer of the invention does not comprise a region of more than 6 consecutive nucleotide analogue units, such as LNA units. In some embodiments, the oligomer or mixmer of the invention does not comprise a region of more than 5 consecutive nucleotide analogue units, such as LNA units. In some embodiments, the oligomer or mixmer of the invention does not comprise a region of more than 4 consecutive nucleotide analogue units, such as LNA units. In some embodiments, the oligomer or mixmer of the invention does not comprise a region of more than 3 consecutive nucleotide analogue units, such as LNA units. In some embodiments, the oligomer or mixmer of the invention does not comprise a region of more than 2 consecutive nucleotide analogue units, such as LNA units. The following embodiments may apply to mixmers or totalmer oligomers (e.g. as region A): The oligomer (e.g. region A) of the invention may, in some embodiments, comprise of at least two alternating regions of LNA and non-LNA nucleotides (such as DNA or 2' substituted nucleotide analogues).

The oligomer part of the LNA antisense oligomer conjugate of the invention may, in some embodiments, comprise a contiguous sequence of formula: 5' ([LNA nucleotides]₁₋₅ and [non-LNA nucleotides]₁₋₄)₂₋₁₂. 3'.

In some embodiments, the 5' nucleotide of the contiguous nucleotide sequence (or the oligomer) is an LNA nucleotide.

In some embodiments, the 3' nucleotide of the contiguous nucleotide sequence is a nucleotide analogue, such as LNA, or the 2, 3, 4, 5 3' nucleotides are nucleotide analogues, such as LNA nucleotides, or other nucleotide analogues which confer enhanced serum stability to the oligomer.

In some embodiments, the contiguous nucleotide sequence of the oligomer has a formula 5' ([LNA nucleotides]₁₋₅ - [non-LNA nucleotides]₁₋₄)₂₋₁₁ - [LNA nucleotides]₁₋₅ 3'.

In some embodiments, the contiguous nucleotide sequence of the oligomer has 2, 3 or 4 contiguous regions of LNA and non-LNA nucleotides - e.g. comprises formula 5' ([LNA nucleotides]₁₋₅ and [non-LNA nucleotides]₁₋₄)₂₋₃, optionally with a further 3' LNA region [LNA nucleotides]₁₋₅.

In some embodiments, the contiguous nucleotide sequence of the oligomer comprises 5' ([LNA nucleotides]₁₋₃ and [non-LNA nucleotides]₁₋₃)₂₋₅, optionally with a further 3' LNA region [LNA nucleotides]₁₋₃.

In some embodiments, the contiguous nucleotide sequence of the oligomer comprises 5' ([LNA nucleotides]₁₋₃ and [non-LNA nucleotides]₁₋₃)₃, optionally with a further 3' LNA region [LNA nucleotides]₁₋₃.

In some embodiments the non-LNA nucleotides are all DNA nucleotides.

In some embodiments, the non-LNA nucleotides are independently or dependently selected from the group consisting of DNA units, RNA units, 2'-O-alkyl-RNA units, 2'-OMe-RNA units, 2'-amino-DNA units, and 2'-fluoro-DNA units.

In some embodiments the non-LNA nucleotides are (optionally independently selected from the group consisting of 2' substituted nucleoside analogues, such as (optionally independently) selected from the group consisting of 2'-O-alkyl-RNA units, 2'-OMe-RNA units, 2'-amino-DNA units, 2'-AP, 2'-FANA, 2'-(3-hydroxy)propyl, and 2'-fluoro-DNA units, and/or other (optionally) sugar modified nucleoside analogues such as morpholino, peptide nucleic acid (PNA), CeNA, unlinked nucleic acid (UNA), hexitol nucleoic acid (HNA). bicyclo-HNA (see e.g. WO2009/100320), In some embodiments, the nucleoside analogues increase the affinity of the first region for its target nucleic acid (or a complementary DNA or RNA sequence). Various nucleoside analogues are disclosed in Freier & Altmann; Nucl. Acid Res., 1997, 25, 4429-4443 and Uhlmann; Curr. Opinion in Drug Development, 2000, 3(2), 293-213.

In some embodiments, the non-LNA nucleotides are DNA nucleotides. In some embodiments, the oligomer or contiguous nucleotide sequence comprises of LNA nucleotides and optionally other nucleotide analogues (such as the nucleotide analogues listed under non-LNA nucleotides) which may be affinity enhancing nucleotide analogues and/or nucleotide analogues which enhance serum stability.

In some embodiments, the oligomer or contiguous nucleotide sequence thereof consists of a contiguous nucleotide sequence of said nucleotide analogues.

In some embodiments, the oligomer or contiguous nucleotide sequence thereof consists of a contiguous nucleotide sequence of LNA nucleotides.

In some embodiments, the oligomer or contiguous nucleotide sequence is 8 - 12, such as 8 - 10, or 10 - 20, such as 12 - 18 or 14 - 16 nts in length.

In some embodiments, the oligomer or contiguous nucleotide sequence is capable of forming a duplex with a complementary single stranded RNA nucleic acid molecule with phosphodiester internucleoside linkages, wherein the duplex has a Tₘ of at least about 60°C, such as at least 65°C.

Example of a Tₘ Assay: The oligonucleotide: Oligonucleotide and RNA target (PO) duplexes are diluted to 3 mM in 500 ml RNase-free water and mixed with 500 ml 2x Tₘ-buffer (200mM NaCl, 0.2mM EDTA, 20mM Naphosphate, pH 7.0). The solution is heated to 95ºC for 3 min and then allowed to anneal in room temperature for 30 min. The duplex melting temperatures (Tₘ) is measured on a Lambda 40 UV/VIS Spectrophotometer equipped with a Peltier temperature programmer PTP6 using PE Templab software (Perkin Elmer). The temperature is ramped up from 20ºC to 95ºC and then down to 25ºC, recording absorption at 260 nm. First derivative and the local maximums of both the melting and annealing are used to assess the duplex Tₘ.

### Totalmers

A totalmer is a single stranded oligomer which only comprises non-naturally occurring nucleosides, such as sugar-modified nucleoside analogues.

The first region according to the invention maybe totalmers - indeed various totalmer designs are highly effective as oligomers or first region thereofs, e.g. particularly when targeting microRNA (antimiRs) or as splice switching oligomers (SSOs). In some embodiments, the totalmer comprises or consists of at least one XYX or YXY sequence motif, such as a repeated sequence XYX or YXY, wherein X is LNA and Y is an alternative (*i.e.* non LNA) nucleotide analogue, such as a 2'-O-MOE RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 7 and 16 nucleotides, such as 9, 10, 11, 12, 13, 14, or 15 nucleotides, such as between 7 and 12 nucleotides.

In some embodiments, the contiguous nucleotide sequence of the totolmer comprises of at least 30%, such as at least 40%, such as at least 50%, such as at least 60%, such as at least 70%, such as at least 80%, such as at least 90%, such as 95%, such as 100% BNA (LNA) units. The remaining units may be selected from the non-LNA nucleotide analogues referred to herein in, such those selected from the group consisting of 2'-O_alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group 2'-OMe RNA unit and 2'-fluoro DNA unit.

In some embodiments the totalmer consist or comprises of a contiguous nucleotide sequence which consists only of LNA units. In some embodiments, the totalmer, such as the LNA totalmer, is between 7 - 12 nucleoside units in length. In some embodiments, the totalmer (as the oligomer or first region thereof) may be targeted against a microRNA *(i.e.* be antimiRs) - as referred to WO2009/043353.

In some embodiments, the oligomer or contiguous nucleotide sequence comprises of LNA nucleotides and optionally other nucleotide analogues which may be affinity enhancing nucleotide analogues and/or nucleotide analogues which enhance serum stability.

In some embodiments, the oligomer or contiguous nucleotide sequence thereof consists of a contiguous nucleotide sequence of said nucleotide analogues.

In some embodiments, the oligomer or contiguous nucleotide sequence thereof consists of a contiguous nucleotide sequence of LNA nucleotides.

### MicroRNA modulation via the oligomer of the invention

In some embodiments, the oligomer or first region thereof is an oligomer, such as an LNA-antimiR® (an LNA mixmer or totalmer), which comprises or consists of a contiguous nucleotide sequence which is corresponds to or is fully complementary to a microRNA sequence, such as a mature microRNA or part thereof. The use of the present invention in controlling the *in vivo* activity of microRNA is considered of primary importance due to the fact that microRNAs typically regulate numerous mRNAs in the subject. The ability to inactivate therapeutic antimiRs is therefore very desirable.

Numerous microRNAs are related to a number of diseases - see WO2009/043353 for example. The oligomer may in some embodiments, target (i.e. comprises or consists of a contiguous nucleotide sequence which is fully complementary to (a corresponding region of) a microRNA. The microRNA may be a liver expressed microRNA, such as microRNA-21, microRNA-221, miR-122 or miR-33 (miR33a & miR-33b).

Hence, some aspects of the invention relates to the treatment of a disease associated with the expression of microRNAs In some embodiments the oligomer or first region thereof according to the invention, consists or comprises of a contiguous nucleotide sequence which corresponds to or is fully complementary to a microRNA sequence, such as a mature microRNA sequence, such as the human microRNAs published in miRBase (http://microrna.sanger.ac.uk/cgi-bin/sequences/mirna summary.pl?org=hsa). In some embodiment the microRNA is a viral microRNA. At the time of writing, in miRbase 19, there are 1600 precursors and 2042 mature human miRNA sequences in miRBase, including the mature microRNA sequence of each human microRNA. In some embodiments the oligomer according to the invention, consists or comprises of a contiguous nucleotide sequence which corresponds to or is fully complementary to hsa-miR122 (NR_029667.1 GI:262205241), such as the mature has-miR-122. In some embodiments the oligomer according to the invention, consists or comprises of a contiguous nucleotide sequence which corresponds to or is fully complementary to hsa-miR122 (NR_029667.1 GI:262205241), such as the mature has-miR-122 across the length of the oligomer.

In some embodiments when the oligomer or first region thereof targets miR-122, the oligomer is for the use in the treatment of hepatitis C infection.

In some embodiments when the oligomer targets has-miR-33, such as has-miR-33a (GUGCAUUGUAGUUGCAUUGCA) or has-miR-33b (GUGCAUUGCUGUUGCAUUGC), for example in use in the treatment of a metabolic disease, such as metabolic syndrome, athersosclerosis, hypercholesterolemia and related disorders. See Najafi-Shoushtar et al, Science 328 1566-1569, Rayner et al., Science 328 (1570-1573), Horie et al., J Am Heart Assoc. 2012, Dec 1(6). Other liver expressed microRNA which are indicated in metabolic diseases, include miR-758, miR-10b, miR-26 and miR-106b, which are known to directly modulate cholesterol efflux (see Dávalos & Fernandez-Hernando, Pharmacol Res. 2013 Feb) The target may therefore be a microRNA selected from the group consisting of miR-122(MIMAT0004590), miR-33(MIMAT0000091, MIMAT0003301), miR-758 (MIMAT0003879), miR-10b (MIPF0000033), miR-26a (MIMAT0000082) and miR-106b (MIMAT0004672). MicroRNA references are miRBase release 19.

### AntimiR oligomers

Preferred oligomer or first region thereof 'antimiR' designs and oligomers are disclosed in WO2007/112754, WO2007/112753, WO09043353 and US provisional applications 60/979217 and 61/028062. In some embodiments, the oligomer or first region thereof is an antimiR which is a mixmer or a totalmer. The term AntimiR may therefore be replaces with the term oligomer.

AntimiR oligomers are oligomers which consist or comprise of a contiguous nucleotide sequence which is fully complementary to, or essentially complementary to (*i.e.* may comprise one or two mismatches), to a microRNA sequence, or a corresponding sub-sequence thereof. In this regards it is considered that the antimiR may be comprise a contiguous nucleotide sequence which is complementary or essentially complementary to the entire mature microRNA, or the antimiR may be comprise a contiguous nucleotide sequence which is complementary or essentially complementary to a sub-sequence of the mature microRNA or pre-microRNA - such a sub-sequence (and therefore the corresponding contiguous nucleotide sequence) is typically at least 8 nucleotides in length, such as between 8 and 25 nucleotides, such as 9, 10, 11, 12, 13, 14, 15, 16, 17, 18, 19, 20, 21, 22, 23, 24 nucleotides in length, such as between 10-17 or 10-16 nucleotides, such as between 12 - 15 nucleotides.

Numerous designs of AnitmiRs have been suggested, and typically antimiRs for therapeutic use, such as the contiguous nucleotide sequence thereof comprise one or more nucleotide analogues units.

In some embodiments the antimiR may have a gapmer structure as herein described. However, as explained in WO2007/112754 and WO2007/112753, other designs may be preferable, such as mixmers, or totalmers.

WO2007/112754 and WO2007/112753, provide antimiR oligomers and antimiR oligomer designs where the oligomers which are complementary to mature microRNA

In some embodiments, a subsequence of the antimiR corresponds to the miRNA seed region. In some embodiments, the first or second 3' nucleobase of the oligomer corresponds to the second 5' nucleotide of the microRNA sequence.

In some antimiR embodiments, nucleobase units 1 to 6 (inclusive) of the oligomer as measured from the 3' end the region of the oligomer are complementary to the microRNA seed region sequence.

In some antimiR embodiments, nucleobase units 1 to 7 (inclusive) of the oligomer as measured from the 3' end the region of the oligomer are complementary to the microRNA seed region sequence.

In some e antimiR embodiments, nucleobase units 2 to 7 (inclusive) of the oligomer as measured from the 3' end the region of the oligomer are complementary to the microRNA seed region sequence.

In some embodiments, the antimiR oligomer comprises at least one nucleotide analogue unit, such as at least one LNA unit, in a position which is within the region complementary to the miRNA seed region. The antimiR oligomer may, in some embodiments comprise at between one and 6 or between 1 and 7 nucleotide analogue units, such as between 1 and 6 and 1 and 7 LNA units, in a position which is within the region complementary to the miRNA seed region.

In some embodiments, the antimiR of the invention is 7, 8 or 9 nucleotides long, and comprises a contiguous nucleotide sequence which is complementary to a seed region of a human or viral microRNA, and wherein at least 80 %, such as 85%, such as 90%, such as 95%, such as 100% of the nucleotides are LNA.

In some embodiments, the antimiR of the invention is 7, 8 or 9 nucleotides long, and comprises a contiguous nucleotide sequence which is complementary to a seed region of a human or viral microRNA, and wherein at least 80 % of the nucleotides are LNA, and wherein at least 80%, such as 85%, such as 90%, such as 95%, such as 100% of the internucleotide bonds are phosphorothioate bonds.

In some embodiments, the antimiR comprises one or two LNA units in positions three to eight, counting from the 3' end. This is considered advantageous for the stability of the A-helix formed by the oligo:microRNA duplex, a duplex resembling an RNA:RNA duplex in structure.

The table on pages 48 line 15 to page 51, line 9 of WO2007/112754 provides examples of anti microRNA oligomers (*i.e.* antimiRs which may be the oligomer or first region thereof) and is hereby specifically incorporated by reference.

### MicroRNA mimics

In some embodiments the oligomer or first region thereof is in the form of a miRNA mimic which can be introduced into a cell to repress the expression of one or more mRNA target(s). miRNA mimics are typically fully complementary to the full length miRNA sequence. miRNA mimics are compounds comprising a contiguous nucleotide sequence which are homologous to a corresponding region of one, or more, of the miRNA sequences provided or referenced to herein. The use of miRNA mimics or antimiRs can be used to (optionally) further repress the mRNA targets, or to silence (down- regulate) the miRNA, thereby inhibiting the function of the endogenous miRNA, causing derepression and increased expression of the mRNA target.

### Aptamers

In some embodiments the oligomer or first region thereof may be a therapeutic aptamer, a spiegelmer. Please note that aptmaers may also be ligands, such as recptor ligands, and may therefore be used as a targeting moiety (i.e. further conjugate). Aptamers (e.g. *Spiegelmers)* in the context of the present invention as nucleic acids of between 20 and 50 nucleotides in length, which have been selected on the basis of their conformational structure rather than the sequence of nucleotides - they elicit their therapeutic effect by binding with a target protein directly *in vivo* and they do not, therefore, comprise of the reverse complement of their target - indeed their target is not a nucleic acid but a protein. Specific aptamers which may be the oligomer or first region thereof include Macugen (OSI Pharmaceuticals) or ARC1779, (Archemix, Cambridge, MA). In some embodiments, the oligomer or first region thereof is not an aptamer. In some embodiments the oligomer or first region thereof is not an aptamer or a spiegelmer.

### Internucleotide Linkages

The nucleoside monomers of the oligomers (e.g. first and second regions) described herein are coupled together via [internucleoside] linkage groups. Suitably, each monomer is linked to the 3' adjacent monomer via a linkage group.

The person having ordinary skill in the art would understand that, in the context of the present invention, the 5' monomer at the end of an oligomer does not comprise a 5' linkage group, although it may or may not comprise a 5' terminal group.

The terms "linkage group" or "internucleotide linkage" are intended to mean a group capable of covalently coupling together two nucleotides. Specific and preferred examples include phosphate groups and phosphorothioate groups.

The nucleotides of the oligomer of the invention or contiguous nucleotides sequence thereof are coupled together via linkage groups. Suitably each nucleotide is linked to the 3' adjacent nucleotide via a linkage group.

Suitable internucleotide linkages include those listed within WO2007/031091, for example the internucleotide linkages listed on the first paragraph of page 34 of WO2007/031091 (hereby incorporated by reference).

It is, in some embodiments, other than the phosphodiester linkage(s) or region B, the preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

Suitable sulphur (S) containing internucleotide linkages as provided herein may be preferred, such as phosphorothioate or phosphodithioate. Phosphorothioate internucleotide linkages are also preferred, particularly for the first region, such as in gapmers, mixmers, antimirs splice switching oligomers, and totalmers.

For gapmers, the internucleotide linkages in the oligomer may, for example be phosphorothioate or boranophosphate so as to allow RNase H cleavage of targeted RNA. Phosphorothioate is preferred, for improved nuclease resistance and other reasons, such as ease of manufacture.

In one aspect, with the exception of the phosphodiester linkage between the first and second region, and optionally within region B, the remaining internucleoside linkages of the oligomer of the invention, the nucleotides and/or nucleotide analogues are linked to each other by means of phosphorothioate groups. In some embodiments, at least 50%, such as at least 70%, such as at least 80%, such as at least 90% such as all the internucleoside linkages between nucleosides in the first region are other than phosphodiester (phosphate), such as are selected from the group consisting of phosphorothioate phosphorodithioate, or boranophosphate. In some embodiments, at least 50%, such as at least 70%, such as at least 80%, such as at least 90% such as all the internucleoside linkages between nucleosides in the first region are phosphorothioate.

WO09124238 refers to oligomeric compounds having at least one bicyclic nucleoside attached to the 3' or 5' termini by a neutral internucleoside linkage. The oligomers of the invention may therefore have at least one bicyclic nucleoside attached to the 3' or 5' termini by a neutral internucleoside linkage, such as one or more phosphotriester, methylphosphonate, MMI, amide-3, formacetal or thioformacetal. The remaining linkages may be phosphorothioate.

### Conjugates, targeting moieties and blocking groups

The term "conjugate" is intended to indicate a heterogenous molecule formed by the covalent attachment ("conjugation") of the oligomer as described herein has one or more non-nucleotide, or non-polynucleotide moieties conjugated thereto.

### Carbohydrate conjugates

In the invention, the conjugate moiety comprises a carbohydrate which is an asialoglycoprotein receptor targeting moiety.

In some embodiments, the carbohydrate is selected from the group consisting of galactose, lactose, n-acetylgalactosamine, mannose, and mannose-6-phosphate. In some embodiments, the conjugate group is or may comprise mannose or mannose-6-phosphate. Carbohydrate conjugates may be used to enhance delivery or activity in a range of tissues, such as liver and/or muscle. See, for example, EP1495769, WO99/65925, Yang et al., Bioconjug Chem (2009) 20(2): 213-21. Zatsepin & Oretskaya Chem Biodivers. (2004) 1(10): 1401-17.

In addition, the oligomer may further comprise one or more additional conjugate moieties, of which lipophilic or hydrophobic moieties are particularly interesting. These may for example, act as pharmacokinetic modulators, and may be covalently linked to either the carbohydrate conjugate, a linker linking the carbohydrate conjugate to the oligomer or a linker linking multiple carbohydrate conjugates (multi-valent) conjugates, or to the oligomer, optionally via a linker, such as a bio cleavable linker.

### Pharmacokinetic Modulators

The compunds decribed herein may further comprise one or more additional conjugate moieties, of which lipophilic or hydrophobic moieties are particularly interesting, such as when the conjugate group is a carbohydrate moiety. Such lipophilic or hydrophobic moieties may act as pharmacokinetic modulators, and may be covalently linked to either the carbohydrate conjugate, a linker linking the carbohydrate conjugate to the oligomer or a linker linking multiple carbohydrate conjugates (multi-valent) conjugates, or to the oligomer, optionally via a linker, such as a bio cleavable linker.

The oligomer conjugate moiety of the invention comprises a pharmacokinetic modulator selected from the group concsisting of C8 - C36 saturated or un-saturated fatty acid, sterol, cholesterol, palmitoyl, hexadec-8-enoyl, oleuyl, (9E, 12E)-octadeca-9,12-dienoyl, dioctanoyl, and C16-C20 acyl. Such moieties are disclosed within the context of siRNA conjugates in WO2012/082046. The hydrophobic moiety may comprise a C8 - C36 fatty acid, which may be saturated or un-saturated. In some embodiments, C10, C12, C14, C16, C18, C20, C22, C24, C26, C28, C30, C32 and C34 fatty acids may be used. The hydrophobic group may have 16 or more carbon atoms. Exemplary suitable hydrophobic groups may be selected from the group comprising: sterol, cholesterol, palmitoyl, hexadec-8-enoyl, oleyl, (9E, 12E)-octadeca-9,12-dienoyl, dioctanoyl, and C16-C20 acyl. According to WO'346, hydrophobic groups having fewer than 16 carbon atoms are less effective in enhancing polynucleotide targeting, but they may be used in multiple copies (e.g. 2x, such as 2x C8 or C10, C12 or C14) to enhance efficacy. Pharmacokinetic modulators useful as polynucleotide targeting moieties may be selected from the group consisting of: cholesterol, alkyl group, alkenyl group, alkynyl group, aryl group, aralkyl group, aralkenyl group, and aralkynyl group, each of which may be linear, branched, or cyclic. Pharmacokinetic modulators are preferably hydrocarbons, containing only carbon and hydrogen atoms. However, substitutions or heteroatoms which maintain hydrophobicity, for example fluorine, may be permitted.

Surprisingly, the present inventors have found that GalNac conjugates for use with LNA oligomers do not require a pharmacokinetic modulator, and as such, the disclosure encompasses oligomer conjugates wherein the GalNac conjugate is not covalently linked to a lipophilic or hydrophobic moiety, such as those described here in, e.g. do not comprise a C8 - C36 fatty acid or a sterol. The disclosure therefore also provides for LNA oligomer GalNac conjugates which do not comprise a lipophilic or hydrophobic pharmacokinetic modulator or conjugate moiety/group.

### GaINAc Conjugates

The application describes LNA antisense oligonucleotides which are conjugated to an asialoglycoprotein receptor targeting moiety. In some embodiments, the conjugate moiety (such as the third region or region C) comprises an asialoglycoprotein receptor targeting moiety, such as galactose, galactosamine, N-formyl-galactosamine, Nacetylgalactosamine, N-propionyl-galactosamine, N-n-butanoyl-galactosamine, and N-isobutanoylgalactos-amine. In some embodiments the conjugate comprises a galactose cluster, such as N-acetylgalactosamine trimer. In some embodiments, the conjugate moiety comprises an GalNAc (N-acetylgalactosamine), such as a mono-valent, di-valent , tri-valent of tetra-valent GaINAc. Trivalent GalNAc conjugates may be used to target the compound to the liver. GalNAc conjugates have been used with methylphosphonate and PNA antisense oligonucleotides (e.g. US 5,994517 and Hangeland et al., Bioconjug Chem. 1995 Nov-Dec;6(6):695-701) and siRNAs (e.g. WO2009/126933, WO2012/089352 & WO2012/083046). The GalNAc references and the specific conjugates used therein are hereby incorporated by reference. Other GalNAc conjugate moieties can include, for example, oligosaccharides and carbohydrate clusters such as Tyr-Glu-Glu-(aminohexyl GaINAc)3 (YEE(ahGaINAc)3; a glycotripeptide that binds to Gal/GaINAc receptors on hepatocytes, see, e.g., Duff, et al., Methods Enzymol, 2000, 313, 297); lysine-based galactose clusters (e.g., L3G4; Biessen, et al., Cardovasc. Med., 1999, 214); and cholane-based galactose clusters (e.g., carbohydrate recognition motif for asialoglycoprotein receptor). Further suitable conjugates can include oligosaccharides that can bind to carbohydrate recognition domains (CRD) found on the asiologlycoprotein-receptor (ASGPR). Example conjugate moieties containing oligosaccharides and/or carbohydrate complexes are provided in U.S. Pat. No. 6,525,031.

WO2012/083046 discloses siRNAs withGaINAc conjugate moieties which comprise cleavable pharmacokinetic modulators, which are suitable for use in the present invention, the preferred pharmacokinetic modulators are C16 hydrophobic groups such as palmitoyl, hexadec-8-enoyl, oleyl, (9E, 12E)-octadeca-9,12-dienoyl, dioctanoyl, and C16-C20 acyl. The '046 cleavable pharmacokinetic modulators may also be cholesterol.

The targeting moieties (conjugate moieties) may be selected from the group consisting of: galactose, galactosamine, N-formyl-galactosamine, N-acetylgalactosamine, Npropionyl-galactosamine, N-n-butanoyl-galactosamine, N-iso-butanoylgalactos-amine, galactose cluster, and N-acetylgalactosamine trimer and may have a pharmacokinetic modulator selected from the group consisting of: hydrophobic group having 16 or more carbon atoms, hydrophobic group having 16-20 carbon atoms, palmitoyl, hexadec-8-enoyl, oleyl, (9E,12E)-octadeca-9,12dienoyl, dioctanoyl, and C16-C20 acyl, and cholesterol. Certain GalNac clusters disclosed in '046 include: (E)-hexadec-8-enoyl (C16), oleyl (C18), (9,E,12E)-octadeca-9,12-dienoyl (C18), octanoyl (C8), dodececanoyl (C12), C-20 acyl, C24 acyl, dioctanoyl (2xC8). The targeting moiety-pharmacokinetic modulator targeting moiety may be linked to the polynucleotide via a physiologically labile bond or, e.g. a disulfide bond, or a PEG linker. The invention also relates to the use of phospodiester linkers, such as DNA phosphodiester linkers, between the oligomer and the conjugate group (these are referred to as region B herein, and suitably are positioned between the LNA oligomer and the carbohydrate conjugate group).

For targeting hepatocytes in liver, a preferred targeting ligand is a galactose cluster. A galactose cluster comprises a molecule having e.g. comprising two to four terminal galactose derivatives. As used herein, the term galactose derivative includes both galactose and derivatives of galactose having affinity for the asialoglycoprotein receptor equal to or greater than that of galactose. A terminal galactose derivative is attached to a molecule through its C-I carbon. The asialoglycoprotein receptor (ASGPr) is unique to hepatocytes and binds branched galactose-terminal glycoproteins. A preferred galactose cluster has three terminal galactosamines or galactosamine derivatives each having affinity for the asialoglycoprotein receptor. A more preferred galactose cluster has three terminal N-acetyl-galactosamines. Other terms common in the art include tri-antennary galactose, tri-valent galactose and galactose trimer. It is known that tri-antennary galactose derivative clusters are bound to the ASGPr with greater affinity than bi-antennary or mono-antennary galactose derivative structures (Baenziger and Fiete, 1980, Cell, 22, 611-620; Connolly et al., 1982,1. Biol. Chern., 257,939-945). Multivalency is required to achieve nM affinity. According to WO 2012/083046 the attachment of a single galactose derivative having affinity for the asialoglycoprotein receptor does not enable functional delivery of the RNAi polynucleotide to hepatocytes in vivo when co-administered with the delivery polymer.

A galactose cluster may comprise two or preferably three galactose derivatives each linked to a central branch point. The galactose derivatives are attached to the central branch point through the C-I carbons of the saccharides. The galactose derivative is preferably linked to the branch point via linkers or spacers. A preferred spacer is a flexible hydrophilic spacer (U.S. Patent 5885968; Biessen et al. J. Med. Chern. 1995 Vol. 39 p. 1538-1546). A preferred flexible hydrophilic spacer is a PEG spacer. A preferred PEG spacer is a PEG3 spacer. The branch point can be any small molecule which permits attachment of the three galactose derivatives and further permits attachment of the branch point to the oligomer. An exemplary branch point group is a di-lysine. A di-lysine molecule contains three amine groups through which three galactose derivatives may be attached and a carboxyl reactive group through which the di-lysine may be attached to the oligomer. Attachment of the branch point to oligomer may occur through a linker or spacer. A preferred spacer is a flexible hydrophilic spacer. A preferred flexible hydrophilic spacer is a PEG spacer. A preferred PEG spacer is a PEG3 spacer (three ethylene units). The galactose cluster may be attached to the 3' or 5' end of the oligomer using methods known in the art.

A preferred galactose derivative is an N-acetyl-galactosamine (GalNAc). Other saccharides having affinity for the asialoglycoprotein receptor may be selected from the list comprising: galactosamine, N-n-butanoylgalactosamine, and N-iso-butanoylgalactosamine. The affinities of numerous galactose derivatives for the asialoglycoprotein receptor have been studied (see for example: Jobst, S.T. and Drickamer, K. JB.C. 1996,271,6686) or are readily determined using methods typical in the art.

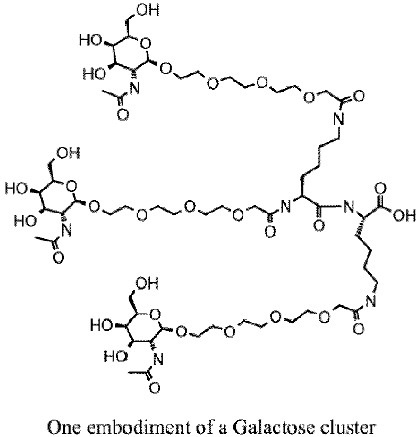

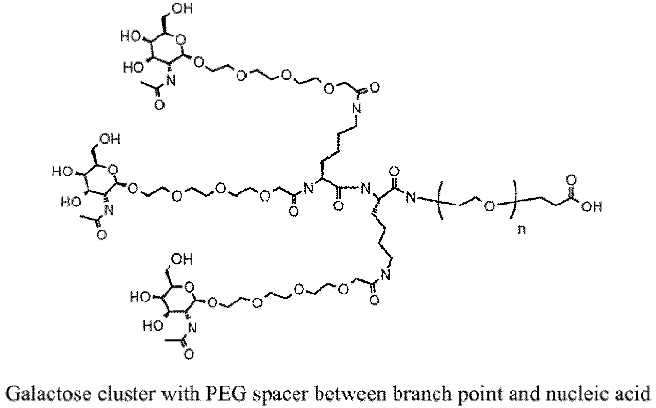

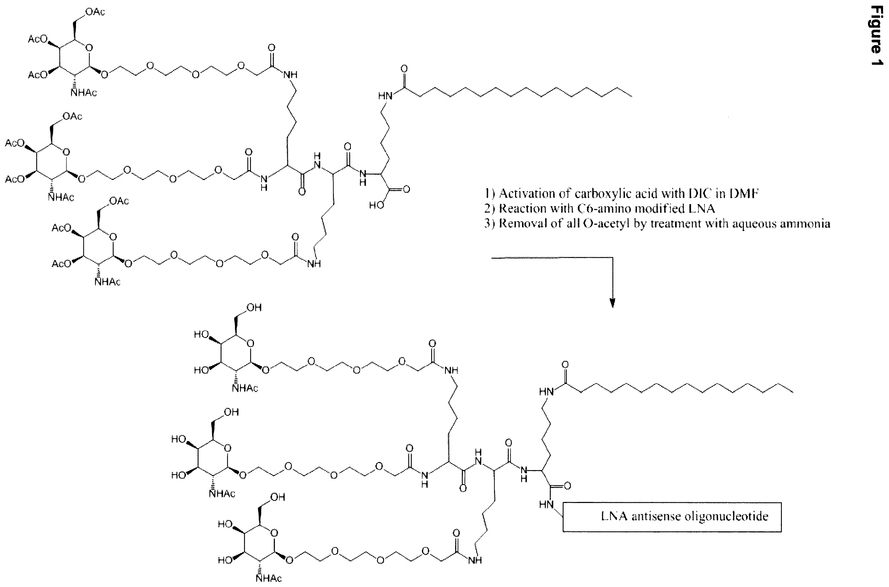

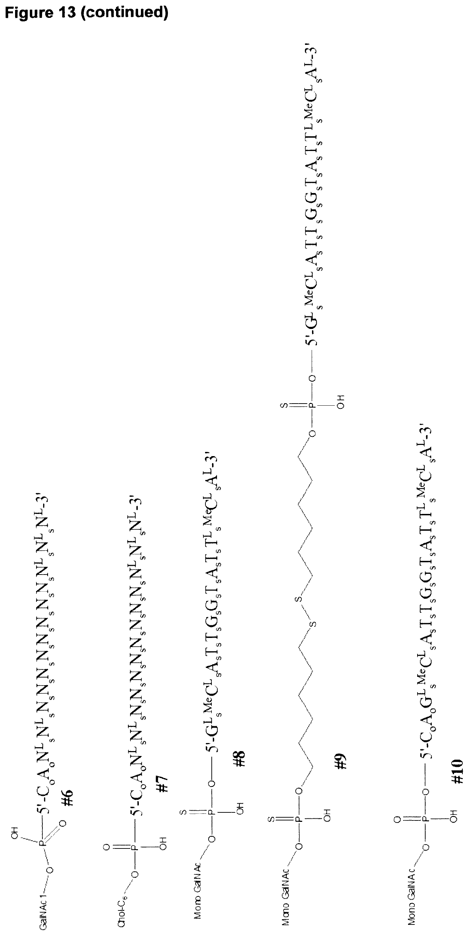

A GalNac conjugate is illustrated in figure 1. Further examples of the conjugate are illustrated below:

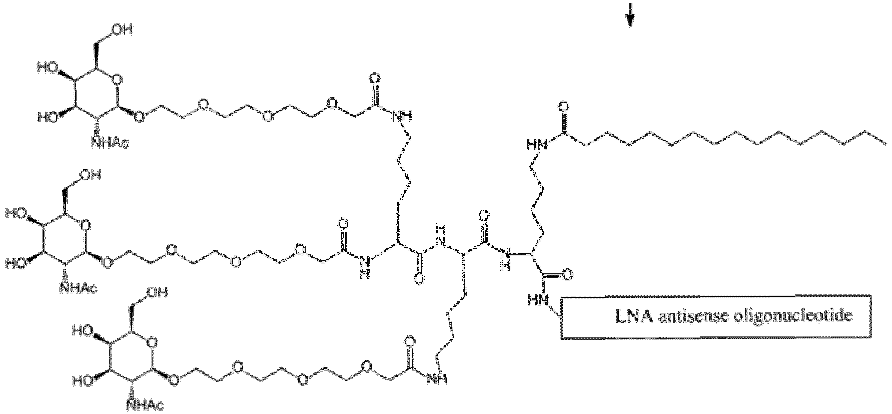

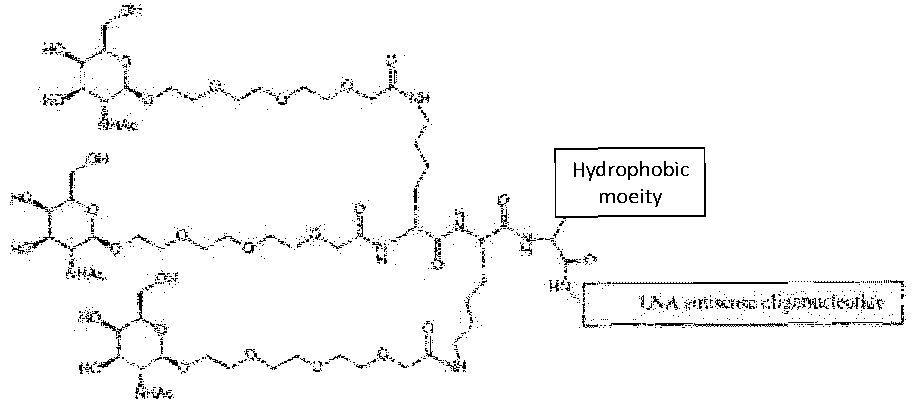

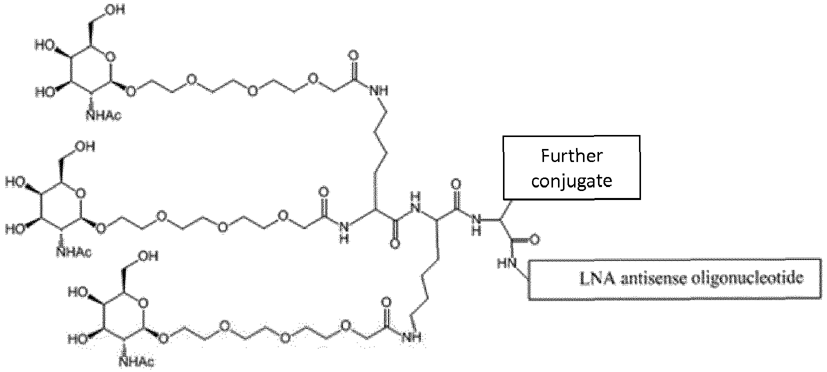

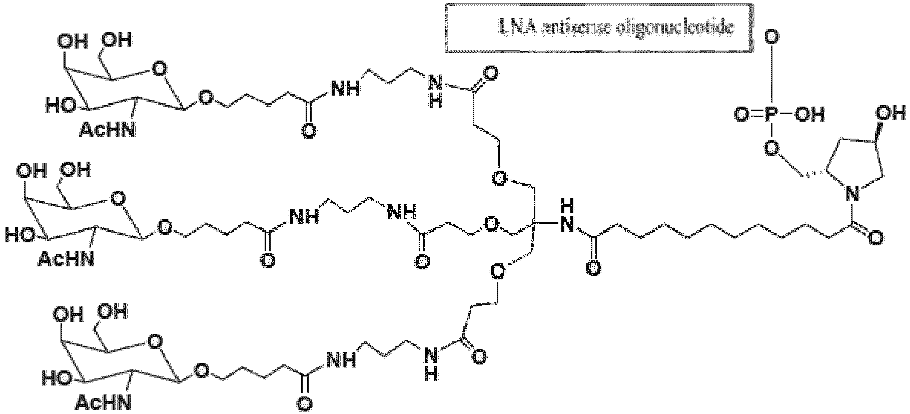

The carbohydrate conjugate (e.g. GalNAc) may therefore be linked to the oligomer via a linker, such as (poly)ethylene glycol linker (PEG), such as a di, tri, tetra, penta, hexa-ethylene glycol linker.

As described herein, a carbohydrate conjugate (e.g. GalNAc) may therefore be linked to the oligomer via a biocleavable linker, such as region B as defined herein, and optionally region Y, which is illustrated as a di-lysine in the above diagrams.

Where at the hydrophobic or lipophilic (or further conjugate) moiety (i.e. pharmacokinetic modulator) in the above GalNac cluster conjugates is, when using BNA or LNA oligomers, such as LNA antisense oligonucleotides, optional.

See the figures for specific GalNAc clusters used in the present study, Conj 1, 2, 3, 4 and Conj1a, 2a, 3a and 4a (which are shown with an optional C6 linker which joins the GalNac cluster to the oligomer).

Each carbohydrate moiety of a GalNAc cluster (e.g. GalNAc) may therefore be joined to the oligomer via a spacer, such as (poly)ethylene glycol linker (PEG), such as a di, tri, tetra, penta, hexa-ethylene glycol linker. As is shown above the PEG moiety forms a spacer between the galactose sugar moiety and a peptide (trilysine is shown) linker.

The carbohydrate conjugate(e.g. GalNAc), or carbohydrate-linker moiety (e.g. carbohydrate-PEG moiety) may be covalently joined (linked) to the oligomer via a branch point group such as, an amino acid, or peptide, which suitably comprises two or more amino groups (such as 3, 4, or5), such as lysine, di-lysine or tri-lysine or tetra-lysine. A tri-lysine molecule contains four amine groups through which three carbohydrate conjugate groups, such as galactose & derivatives (e.g.GalNAc) and a further conjugate such as a hydrophobic or lipophilic moiety/group may be attached and a carboxyl reactive group through which the tri-lysine may be attached to the oligomer. The further conjugate, such as lipophilic/hyrodphobic moiety may be attached to the lysine residue that is attached to the oligomer.

In some instances, the GalNac cluster comprises a peptide linker, e.g. a Tyr-Asp(Asp) tripeptide or Asp(Asp) dipeptide, which is attached to the oligomer (or to region Y or region B) via a biradical linker, for example the GalNac cluster may comprise the following biradical linkers:

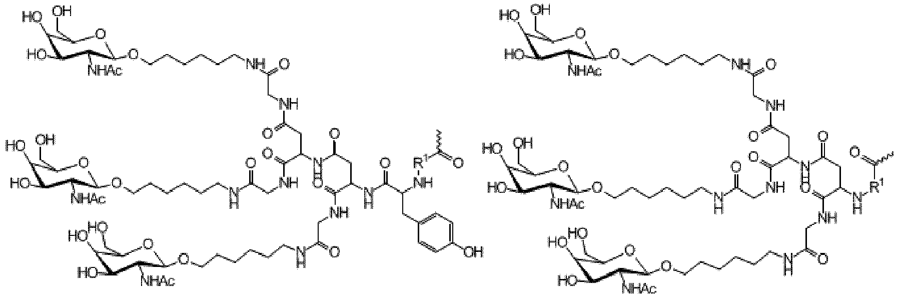

R¹ is a biradical preferably selected from -C₂H₄-, -C₃H₆-, -C₄H₈-, -C₅H₁₀-, -C₆H₁₂-, 1,4-cyclohexyl (-C6H10-), 1,4-phenyl (-C₆H₄-), -C₂H₄OC₂H₄-, -C₂H₄(OC₂H₄)₂- or -C₂H₄(OC₂H₄)₃-, C(O)CH₂-, -C(O)C₂H₄-, - C(O)C₃H₆-, - C(O)C₄H₈-, - C(O)C₅H₁₀-, - C(O)C₆H₁₂-, 1,4-cyclohexyl (-C(O)C6H10-), 1,4-phenyl (-C(O)C₆H₄-), - C(O)C₂H₄OC₂H₄-, - C(O)C₂H₄(OC₂H₄)₂- or-C(O)C₂H₄(OC₂H₄)₃- . In some emboidments, R¹ is a biradical preferably selected from -C₂H₄-, -C₃H₆-, -C₄H₈-, -C₅H₁₀-, -C₆H₁₂-, 1,4-cyclohexyl (-C6H10-), 1,4-phenyl (-C₆H₄-),-C₂H₄OC₂H₄-, -C₂H₄(OC₂H₄)₂- or -C₂H₄(OC₂H₄)₃-.

### Amino alkyl Intermediates

The application further describes the LNA oligomer intermediates which comprise an antisense LNA oligomer which comprises an (e.g. terminal, 5' or 3') amino alkyl, such as a C2 - C36 amino alkyl group, including, for example C6 and C12 amino alkyl groups. The amino alkyl group may be added to the LNA oligomer as part of standard oligonucleotide synthesis, for example using a (e.g. protected) amino alkyl phosphoramidite. The linkage group between the amino alkyl and the LNA oligomer may for example be a phosphorothioate or a phosphodiester, or one of the other nucleoside linkage groups referred to herein, for example. The amino alkyl group may be covalently linked to, for example, the 5' or 3' of the LNA oligomer, such as by the nucleoside linkage group, such as phosphorothioate or phosphodiester linkage.

The application also describes a method of synthesis of the LNA oligomer comprising the sequential synthesis of the LNA oligomer, such as solid phase oligonucleotide synthesis, comprising the step of adding an amino alkyl group to the oligomer, such as e.g. during the first or last round of oligonucleotide synthesis. The method of synthesis my further comprise the step of reacting the carbohydrate conjugate to the amino alkyl -LNA oligomer (the conjugation step). The carbohydrate conjugate may comprise suitable linkers and/or branch point groups, and optionally further conjugate groups, such as hydrophobic or lipophilic groups, as described herein. The conjugation step may be performed whilst the oligomer is bound to the solid support (e.g. after oligonucleotide synthesis, but prior to elution of the oligomer from the solid support), or subsequently (i.e. after elution). The disclosure provides for the use of an amino alkyl linker in the synthesis of the oligomer of the invention.

The application describes a method of synthesizing (or manufacture) of an oligomeric compound, such as the oligomeric compound of the invention, said method comprising a step of [sequential] oligonucleotide synthesis of a first region (A) and (optionally, when present), a second region (B), wherein the synthesis step is followed by a step of adding a third region [phosphoramidite comprising] comprising a carbohydrate conjugate group as described herein (X or with a linker X-Y), such as for example a GalNAc conjugate, such as a trivalent GalNAc (e.g. a conjugate selected from the group consisting of Conj1, Conj2, Conj3, Conj 4, Conj1a, Conj2a, Conj3a, and Conj 4a, or other trivalent GalNAc conjugate moieties, such as those disclosed herein. followed by the cleavage of the oligomeric compound from the [solid phase] support.

It is however recognized that the conjuigate region (e.gX or X-Y) may be added after the cleavage from the solid support. Alternatively, the method of synthesis may comprise the steps of synthesizing a first (A), and optionally second region (B), followed by the cleavage of the oligomer from the support, with a subsequent step of adding the third region, such as X or X-Y group to the oligomer. The addition of the third region may be achieved, by example, by adding an amino phosphoramidite unit in the final step of oligomer synthesis (on the support), which can, after cleavage from the support, be used to join to the X or X-Y group, optionally via an activation group on the X or Y (when present) group. In the embodiments where the cleavable linker is not a nucleotide region, region B may be a non-nucleotide cleavable linker for example a peptide linker, which may form part of region X (also referred to as region C) or be region Y (or part thereof).

Region X (such as C) or (X-Y), such as the conjugate (e.g. a GalNAc conjugate) may comprise an activation group, (an activated functional group) and in the method of synthesis the activated conjugate (or region x, or X-Y) is added to the first and second regions, such as an amino linked oligomer. The amino group may be added to the oligomer by standard phosphoramidite chemistry, for example as the final step of oligomer synthesis (which typically will result in amino group at the 5' end of the oligomer). For example during the last step of the oligonucleotide synthesis a protected amino-alkyl phosphoramidite is used, for example a TFA-aminoC6 phosphoramidite (6-(Trifluoroacetylamino)-hexyl-(2-cyanoethyl)-(N,N-diisopropyl)-phosphoramidite). The amino group may be added to the oligomer, for example as the first step of oligomer synthesis (which typically will result in amino group at the 3' end of the oligomer). For example by using a solid support for example with N-(6-(O-(4,4'-Dimethoxytrityl))-hexyl)-(2-carboxamide)-phthalimidyl.

Region X (or region C as referred to herein), such as the conjugate (e.g. a GalNac conjugate) may be activated via NHS ester method and then the aminolinked oligomer is added. For example a N-hydroxysuccinimide (NHS) may be used as activating group for region X (or region C, such as a conjugate, such as a GalNac conjugate moiety.

### Lipophilic conjugates

The oligomer may further comprise another conjugate such as a lipophilic conjugate (for example as a pharmacokinetic modulator). Representative conjugate moieties can include lipophilic molecules (aromatic and non-aromatic) including steroid molecules; proteins (e.g., antibodies, enzymes, serum proteins); peptides; vitamins (water-soluble or lipid-soluble); polymers (water-soluble or lipid-soluble); small molecules including drugs, toxins, reporter molecules, and receptor ligands; carbohydrate complexes; nucleic acid cleaving complexes; metal chelators (e.g., porphyrins, texaphyrins, crown ethers, etc.); intercalators including hybrid photonuclease/intercalators; crosslinking agents (e.g., photoactive, redox active), and combinations and derivatives thereof. Numerous suitable conjugate moieties, their preparation and linkage to oligomeric compounds are provided, for example, in WO 93/07883 and U.S. Pat. No. 6,395,492. Oligonucleotide conjugates and their syntheses are also reported in comprehensive reviews by Manoharan in Antisense Drug Technology, Principles, Strategies, and Applications, S.T. Crooke, ed., Ch. 16, Marcel Dekker, Inc., 2001 and Manoharan, Antisense and Nucleic Acid Drug Development, 2002, 12, 103. Lipophilic conjugate moieties can be used, for example, to counter the hydrophilic nature of an oligomeric compound and enhance cellular penetration. Lipophilic moieties include, for example, steroids and related compounds such as cholesterol (U.S. Pat. No. 4,958,013 and Letsinger et al., Proc. Natl. Acad. Sci. USA, 1989, 86, 6553), thiocholesterol (Oberhauser et al, Nucl Acids Res., 1992, 20, 533), lanosterol, coprostanol, stigmasterol, ergosterol, calciferol, cholic acid, deoxycholic acid, estrone, estradiol, estratriol, progesterone, stilbestrol, testosterone, androsterone, deoxycorticosterone, cortisone, 17-hydroxycorticosterone, their derivatives, and the like.

Other lipophilic conjugate moieties include aliphatic groups, such as, for example, straight chain, branched, and cyclic alkyls, alkenyls, and alkynyls. The aliphatic groups can have, for example, 5 to about 50, 6 to about 50, 8 to about 50, or 10 to about 50 carbon atoms. Example aliphatic groups include undecyl, dodecyl, hexadecyl, heptadecyl, octadecyl, nonadecyl, terpenes, bornyl, adamantyl, derivatives thereof and the like. In some embodiments, one or more carbon atoms in the aliphatic group can be replaced by a heteroatom such as O, S, or N (e.g., geranyloxyhexyl). Further suitable lipophilic conjugate moieties include aliphatic derivatives of glycerols such as alkylglycerols, bis(alkyl)glycerols, tris(alkyl)glycerols, monoglycerides, diglycerides, and triglycerides. In some embodiments, the lipophilic conjugate is di-hexyldecyl-rac-glycerol or 1,2-di-O- hexyldecyl-rac-glycerol (Manoharan et al., Tetrahedron Lett., 1995, 36, 3651; Shea, et al., Nuc. Acids Res., 1990, 18, 3777) or phosphonates thereof. Saturated and unsaturated fatty functionalities, such as, for example, fatty acids, fatty alcohols, fatty esters, and fatty amines, can also serve as lipophilic conjugate moieties. In some embodiments, the fatty functionalities can contain from about 6 carbons to about 30 or about 8 to about 22 carbons. Example fatty acids include, capric, caprylic, lauric, palmitic, myristic, stearic, oleic, linoleic, linolenic, arachidonic, eicosenoic acids and the like.

Lipophilic conjugate groups can be polycyclic aromatic groups having from 6 to about 50, 10 to about 50, or 14 to about 40 carbon atoms. Example polycyclic aromatic groups include pyrenes, purines, acridines, xanthenes, fluorenes, phenanthrenes, anthracenes, quinolines, isoquinolines, naphthalenes, derivatives thereof and the like. Other suitable lipophilic conjugate moieties include menthols, trityls (e.g., dimethoxytrityl (DMT)), phenoxazines, lipoic acid, phospholipids, ethers, thioethers (e.g., hexyl-S-tritylthiol), derivatives thereof and the like. Preparation of lipophilic conjugates of oligomeric compounds are well-described in the art, such as in, for example, Saison-Behmoaras et al, EMBO J., 1991, 10, 1111; Kabanov et al., FEBSLett., 1990, 259, 327; Svinarchuk et al, Biochimie, 1993, 75, 49; (Mishra et al., Biochim. Biophys. Acta, 1995, 1264, 229, and Manoharan et al., Tetrahedron Lett., 1995, 36, 3651.

Oligomeric compounds containing conjugate moieties with affinity for low density lipoprotein (LDL) can help provide an effective targeted delivery system. High expression levels of receptors for LDL on tumor cells makes LDL an attractive carrier for selective delivery of drugs to these cells (Rump, et al., Bioconjugate Chem., 1998, 9, 341; Firestone, Bioconjugate Chem., 1994, 5, 105; Mishra, et al., Biochim. Biophys. Acta, 1995, 1264, 229). Moieties having affinity for LDL include many lipophilic groups such as steroids (e.g., cholesterol), fatty acids, derivatives thereof and combinations thereof. In some embodiments, conjugate moieties having LDL affinity can be dioleyl esters of cholic acids such as chenodeoxycholic acid and lithocholic acid.

The conjugate group is or may comprise a lipophilic moiety, such as a sterol (for example, cholesterol, cholesteryl, cholestanol, stigmasterol, cholanic acid and ergosterol). In some embodiments, the conjugate is or may comprise cholesterol. See for example, Soutschek et al., Nature (2004) 432, 173; Krützfeldt Nature 2005, NAR 2007.

The conjugate is, or may comprise a lipid, a phospholipid or a lipophilic alcohol, such as a cationic lipids, a neutral lipids, sphingolipids, and fatty acids such as stearic, oleic, elaidic, linoleic, linoleaidic, linolenic, and myristic acids. In some embodiments the fatty acid comprises a C4 - C30 saturated or unsaturated alkyl chain. The alkyl chain may be linear or branched.

The lipophillic conjugates may be or may comprise biotin. In some embodiments, the lipophilic conjugate may be or may comprise a glyceride or glyceride ester.

Lipophillic conjugates, such as cholesterol or as disclosed herein, may be used to enhance delivery of the oligonucleotide to, for example, the liver (typically hepatocytes).

The following references refer to the use of lipophilic conjugates: Kobylanska et al., Acta Biochim Pol. (1999); 46(3): 679 - 91. Felber et al,. Biomaterials (2012) 33(25): 599-65); Grijalvo et al., J Org Chem (2010) 75(20): 6806 - 13. Koufaki et al., Curr Med Chem (2009) 16(35): 4728-42. Godeau et al J. Med. Chem. (2008) 51(15): 4374-6.

The conjugate moiety is hydrophilic. The application also describes conjugate groups that do not comprise a lipophilic substituent group, such as a fatty acid substituent group, such as a C8 - C26, such as a palmotyl substituent group, or does not comprise a sterol, e.g. a cholesterol subtituent group. In this regards, part of the invention is based on the suprising discovery that LNA oligomers GalNAC conjugates have remarkable pharmacokinetic properties even without the use of pharmacokinetic modulators, such as fatty acid substituent groups (e.g. >C8 or >C16 fatty acid groups).

### Linkers

A linkage or linker is a connection between two atoms that links one chemical group or segment of interest to another chemical group or segment of interest via one or more covalent bonds. Conjugate moieties (or targeting or blocking moieties) can be attached to the oligomeric compound directly or through a linking moiety (linker or tether) - a linker. Linkers are bifunctional moieties that serve to covalently connect a third region, e.g. a conjugate moiety, to an oligomeric compound (such as to region B). In some embodiments, the linker comprises a chain structure or an oligomer of repeating units such as ethylene glyol or amino acid units. The linker can have at least two functionalities, one for attaching to the oligomeric compound and the other for attaching to the conjugate moiety. Example linker functionalities can be electrophilic for reacting with nucleophilic groups on the oligomer or conjugate moiety, or nucleophilic for reacting with electrophilic groups. In some embodiments, linker functionalities include amino, hydroxyl, carboxylic acid, thiol, phosphoramidate, phophate, phosphite, unsaturations (e.g., double or triple bonds), and the like. Some example linkers include 8-amino-3,6-dioxaoctanoic acid (ADO), succinimidyl 4-(N-maleimidomethyl)cyclohexane-I-carboxylate (SMCC), 6- aminohexanoic acid (AHEX or AHA), 6-aminohexyloxy, 4-aminobutyric acid, 4- aminocyclohexylcarboxylic acid, succinimidyl 4-(N-maleimidomethyl)cyclohexane-I-carboxy-(6-amido-caproate) (LCSMCC), succinimidyl m-maleimido-benzoylate (MBS), succinimidyl N-e-maleimido-caproylate (EMCS), succinimidyl 6-(beta - maleimido-propionamido) hexanoate (SMPH), succinimidyl N-(a-maleimido acetate) (AMAS), succinimidyl 4-(p-maleimidophenyl)butyrate (SMPB), beta -alanine (beta -ALA), phenylglycine (PHG), 4-aminocyclohexanoic acid (ACHC), beta - (cyclopropyl) alanine (beta -CYPR), amino dodecanoic acid (ADC), alylene diols, polyethylene glycols, amino acids, and the like.

A wide variety of further linker groups are known in the art that can be useful in the attachment of conjugate moieties to oligomeric compounds. A review of many of the useful linker groups can be found in, for example, Antisense Research and Applications, S. T. Crooke and B. Lebleu, Eds., CRC Press, Boca Raton, Fla., 1993, p. 303-350. A disulfide linkage has been used to link the 3' terminus of an oligonucleotide to a peptide (Corey, et al., Science 1987, 238, 1401; Zuckermann, et al, J Am. Chem. Soc. 1988, 110, 1614; and Corey, et al., J Am. Chem. Soc. 1989, 111, 8524). Nelson, et al., Nuc. Acids Res. 1989, 17, 7187 describe a linking reagent for attaching biotin to the 3'-terminus of an oligonucleotide. This reagent, N-Fmoc-O- DMT-3 -amino- 1,2-propanediol is commercially available from Clontech Laboratories (Palo Alto, Calif.) under the name 3'-Amine. It is also commercially available under the name 3'-Amino-Modifier reagent from Glen Research Corporation (Sterling, Va.). This reagent was also utilized to link a peptide to an oligonucleotide as reported by Judy, et al., Tetrahedron Letters 1991, 32, 879. A similar commercial reagent for linking to the 5 '-terminus of an oligonucleotide is 5'- Amino-Modifier C6. These reagents are available from Glen Research Corporation (Sterling, Va.). These compounds or similar ones were utilized by Krieg, et al, Antisense Research and Development 1991, 1, 161 to link fluorescein to the 5'- terminus of an oligonucleotide. Other compounds such as acridine have been attached to the 3 '-terminal phosphate group of an oligonucleotide via a polymethylene linkage (Asseline, et al., Proc. Natl. Acad. Sci. USA 1984, 81, 3297). [0074] Any of the above groups can be used as a single linker or in combination with one or more further linkers.

Linkers and their use in preparation of conjugates of oligomeric compounds are provided throughout the art such as in WO 96/11205 and WO 98/52614 and U.S. Pat. Nos. 4,948,882; 5,525,465; 5,541,313; 5,545,730; 5,552,538; 5,580,731; 5,486,603; 5,608,046; 4,587,044; 4,667,025; 5,254,469; 5,245,022; 5,112,963; 5,391,723; 5,510475; 5,512,667; 5,574,142; 5,684,142; 5,770,716; 6,096,875; 6,335,432; and 6,335,437,Wo2012/083046.

As used herein, a physiologically labile bond is a labile bond that is cleavable under conditions normally encountered or analogous to those encountered within a mammalian body (also referred to as a cleavable linker). Physiologically labile linkage groups are selected such that they undergo a chemical transformation (e.g., cleavage) when present in certain physiological conditions. Mammalian intracellular conditions include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic or hydrolytic enzymes. In some embodiments, the cleavable linker is susceptible to nuclease(s) which may for example, be expressed in the target cell - and as such, as detailed herein, the linker may be a short region (e.g. 1 - 10) phosphodiester linked nucleosides, such as DNA nucleosides,

Chemical transformation (cleavage of the labile bond) may be initiated by the addition of a pharmaceutically acceptable agent to the cell or may occur spontaneously when a molecule containing the labile bond reaches an appropriate intra-and/or extra-cellular environment. For example, a pH labile bond may be cleaved when the molecule enters an acidified endosome. Thus, a pH labile bond may be considered to be an endosomal cleavable bond. Enzyme cleavable bonds may be cleaved when exposed to enzymes such as those present in an endosome or lysosome or in the cytoplasm. A disulfide bond may be cleaved when the molecule enters the more reducing environment of the cell cytoplasm. Thus, a disulfide may be considered to be a cytoplasmic cleavable bond. As used herein, a pH-labile bond is a labile bond that is selectively broken under acidic conditions (pH<7). Such bonds may also be termed endosomally labile bonds, since cell endosomes and lysosomes have a pH less than 7.

### Linkers (e.g. Region Y)

A linkage or linker is a connection between two atoms that links one chemical group or segment of interest to another chemical group or segment of interest via one or more covalent bonds. Conjugate moieties (or targeting or blocking moieties) can be attached to the oligomeric compound directly or through a linking moiety (linker or tether) - a linker. Linkers are bifunctional moieties that serve to covalently connect a third region, e.g. a conjugate moiety, to an oligomeric compound (such as to region B). In some embodiments, the linker comprises a chain structure or an oligomer of repeating units such as ethylene glyol or amino acid units. The linker can have at least two functionalities, one for attaching to the oligomeric compound and the other for attaching to the conjugate moiety. Example linker functionalities can be electrophilic for reacting with nucleophilic groups on the oligomer or conjugate moiety, or nucleophilic for reacting with electrophilic groups. In some embodiments, linker functionalities include amino, hydroxyl, carboxylic acid, thiol, phosphoramidate, phosphorothioate, phosphate, phosphite, unsaturations (e.g., double or triple bonds), and the like. Some example linkers include 8-amino-3,6-dioxaoctanoic acid (ADO), succinimidyl 4-(N-maleimidomethyl)cyclohexane-1-carboxylate (SMCC), 6-aminohexanoic acid (AHEX or AHA), 6-aminohexyloxy, 4-aminobutyric acid, 4-aminocyclohexylcarboxylic acid, succinimidyl 4-(N-maleimidomethyl)cyclohexane-I-carboxy-(6-amido-caproate) (LCSMCC), succinimidyl m-maleimido-benzoylate (MBS), succinimidyl N-e-maleimido-caproylate (EMCS), succinimidyl 6-(beta - maleimido-propionamido) hexanoate (SMPH), succinimidyl N-(a-maleimido acetate) (AMAS), succinimidyl 4-(p-maleimidophenyl)butyrate (SMPB), beta -alanine (beta -ALA), phenylglycine (PHG), 4-aminocyclohexanoic acid (ACHC), beta -(cyclopropyl) alanine (beta -CYPR), amino dodecanoic acid (ADC), alylene diols, polyethylene glycols, amino acids, and the like.

A wide variety of further linker groups are known in the art that can be useful in the attachment of conjugate moieties to oligomeric compounds. A review of many of the useful linker groups can be found in, for example, Antisense Research and Applications, S. T. Crooke and B. Lebleu, Eds., CRC Press, Boca Raton, Fla., 1993, p. 303-350. A disulfide linkage has been used to link the 3' terminus of an oligonucleotide to a peptide (Corey, et al., Science 1987, 238, 1401; Zuckermann, et al, J Am. Chem. Soc. 1988, 110, 1614; and Corey, et al., J Am. Chem. Soc. 1989, 111, 8524). Nelson, et al., Nuc. Acids Res. 1989, 17, 7187 describe a linking reagent for attaching biotin to the 3'-terminus of an oligonucleotide. This reagent, N-Fmoc-O- DMT-3 -amino- 1,2-propanediol is commercially available from Clontech Laboratories (Palo Alto, Calif.) under the name 3'-Amine. It is also commercially available under the name 3'-Amino-Modifier reagent from Glen Research Corporation (Sterling, Va.). This reagent was also utilized to link a peptide to an oligonucleotide as reported by Judy, et al., Tetrahedron Letters 1991, 32, 879. A similar commercial reagent for linking to the 5 '-terminus of an oligonucleotide is 5'- Amino-Modifier C6. These reagents are available from Glen Research Corporation (Sterling, Va.). These compounds or similar ones were utilized by Krieg, et al, Antisense Research and Development 1991, 1, 161 to link fluorescein to the 5'- terminus of an oligonucleotide. Other compounds such as acridine have been attached to the 3 '-terminal phosphate group of an oligonucleotide via a polymethylene linkage (Asseline, et al., Proc. Natl. Acad. Sci. USA 1984, 81, 3297). [0074] Any of the above groups can be used as a single linker or in combination with one or more further linkers.

Linkers and their use in preparation of conjugates of oligomeric compounds are provided throughout the art such as in WO 96/11205 and WO 98/52614 and U.S. Pat. Nos. 4,948,882; 5,525,465; 5,541,313; 5,545,730; 5,552,538; 5,580,731; 5,486,603; 5,608,046; 4,587,044; 4,667,025; 5,254,469; 5,245,022; 5,112,963; 5,391,723; 5,510475; 5,512,667; 5,574,142; 5,684,142; 5,770,716; 6,096,875; 6,335,432; and 6,335,437,Wo2012/083046.

As used herein, a physiologically labile bond is a labile bond that is cleavable under conditions normally encountered or analogous to those encountered within a mammalian body (also referred to as a cleavable linker). Physiologically labile linkage groups are selected such that they undergo a chemical transformation (e.g., cleavage) when present in certain physiological conditions. Mammalian intracellular conditions include chemical conditions such as pH, temperature, oxidative or reductive conditions or agents, and salt concentration found in or analogous to those encountered in mammalian cells. Mammalian intracellular conditions also include the presence of enzymatic activity normally present in a mammalian cell such as from proteolytic or hydrolytic enzymes. In some embodiments, the cleavable linker is susceptible to nuclease(s) which may for example, be expressed in the target cell - and as such, as detailed herein, the linker may be a short region (e.g. 1 - 10) phosphodiester linked nucleosides, such as DNA nucleosides,

Chemical transformation (cleavage of the labile bond) may be initiated by the addition of a pharmaceutically acceptable agent to the cell or may occur spontaneously when a molecule containing the labile bond reaches an appropriate intra-and/or extra-cellular environment. For example, a pH labile bond may be cleaved when the molecule enters an acidified endosome. Thus, a pH labile bond may be considered to be an endosomal cleavable bond. Enzyme cleavable bonds may be cleaved when exposed to enzymes such as those present in an endosome or lysosome or in the cytoplasm. A disulfide bond may be cleaved when the molecule enters the more reducing environment of the cell cytoplasm. Thus, a disulfide may be considered to be a cytoplasmic cleavable bond. As used herein, a pH-labile bond is a labile bond that is selectively broken under acidic conditions (pH<7). Such bonds may also be termed endosomally labile bonds, since cell endosomes and lysosomes have a pH less than 7.

### Oligomer linked biocleavable conjugates

The oligomeric compound may optionally, comprise a second region (region B) which is positioned between the oligomer (referred to as region A) and the conjugate (referred to as region C). Region B may be a linker such as a cleavable linker (also referred to as a physiologically labile linkage).

The susceptibility to cleavage in the assays shown in Example 9 may be used to determine whether a linker is biocleavable or physiologically labile.

Biocleavable linkers according to the present invention refers to linkers which are susceptible to cleavage in a target tissue (i.e. physiologically labile), for example liver and/or kidney. It is preferred that the cleavage rate seen in the target tissue is greater than that found in blood serum. Suitable methods for determining the level (%) of cleavage in tissue (e.g. liver or kidney) and in serum are found in example 9. In some embodiments, the biocleavable linker (also referred to as the physiologically labile linker, or nuclease susceptible linker), such as region B, in a compound of the invention, are at least about 20% cleaved, such as at least about 30% cleaved, such as at least about 40% cleaved, such as at least about 50% cleaved, such as at least about 60% cleaved, such as at least about 70% cleaved, such as at least about 75% cleaved, in the liver or kidney homogenate assay of Example 9. In some embodiments, the cleavage (%) in serum, as used in the assay in Example 9, is less than about 30%, is less than about 20%, such as less than about 10%, such as less than 5%, such as less than about 1%.

In some embodiments, which may be the same of different, the biocleavable linker (also referred to as the physiologically labile linker, or nuclease susceptible linker), such as region B, in a compound of the invention, are susceptible to S1 nuclease cleavage. Susceptibility to S1 cleavage may be evaluated using the S1 nuclease assay shown in Example 9. In some embodiments, the biocleavable linker (also referred to as the physiologically labile linker, or nuclease susceptible linker), such as region B, in a compound of the invention, are at least about 30% cleaved, such as at least about 40% cleaved, such as at least about 50% cleaved, such as at least about 60% cleaved, such as at least about 70% cleaved, such as at least about 80% cleaved, such as at least about 90% cleaved, such as at least 95% cleaved after 120min incubation with S1 nuclease according to the assay used in Example 9.

### Nuclease Susceptible Physiological Labile Linkages

The oligomeric compound may optionally, comprise a second region (region B) which is positioned between the LNA oligomer (referred to as region A) and the carbohydrate conjugate (referred to as region C). Region B may be a linker such as a cleavable linker (also referred to as a physiologically labile linkage).

In some embodiments, region B comprises between 1 - 10 nucleotides, which is covalently linked to the 5' or 3' nucleotide of the first region, such as via a internucleoside linkage group such as a phosphodiester linkage, wherein either
a. the internucleoside linkage between the first and second region is a phosphodiester linkage and the nucleoside of the second region [such as immediately] adjacent to the first region is either DNA or RNA; and/or
b. at least 1 nucleoside of the second region is a phosphodiester linked DNA or RNA nucleoside;

In some embodiments, region A and region B form a single contiguous nucleotide sequence of 8 - 35 nucleotides in length. In some aspects the internucleoside linkage between the first and second regions may be considered part of the second region.

In some embodiments, there is a phosphorus containing linkage group between the second and third region. The phosphorus linkage group, may, for example, be a phosphate (phosphodiester), a phosphorothioate, a phosphorodithioate or a boranophosphate group. In some embodiments, this phosphorus containing linkage group is positioned between the second region and a linker region which is attached to the third region. In some embodiments, the phosphate group is a phosphodiester.

Therefore, in some aspects the oligomeric compound comprises at least two phosphodiester groups, wherein at least one is as according to the above statement of invention, and the other is positioned between the second and third regions, optionally between a linker group and the second region.

The application also describe a third region which is an activation group, such as an activation group for use in conjugation. In this respect, the invention also provides activated oligomers comprising region A and B and a activation group, e.g an intermediate which is suitable for subsequent linking to the third region, such as suitable for conjugation.

The application also describea third region which is a reactive group, such as a reactive group for use in conjugation. In this respect, the invention also provides oligomers comprising region A and B and a reactive group, e.g an intermediate which is suitable for subsequent linking to the third region, such as suitable for conjugation. The reactive group may, in some embodiments comprise an amine of alcohol group, such as an amine group.

In some embodiments region A comprises at least one, such as 2, 3, 4, 5, 6, 7, 8, 9, 10, 11, 12, 13, 14, 15, 16, 17, 18, 19, 20, 21, 22, 23, or 24 internucleoside linkages other than phosphodiester, such as internucleoside linkages which are (optionally independently] selected from the group consisting of phosphorothioate, phosphorodithioate,and boranophosphate, and methylphosphonate, such as phosphorothioate. In some embodiments region A comprises at least one phosphorothioate linkage. In some embodiments at least 50%, such as at least 75%, such as at least 90% of the internucleoside linkages, such as all the internucleoside linkages within region A are other than phosphodiester, for example are phosphorothioate linkages. In some embodiments, all the internucleoside linkages in region A are other than phosphodiester.

In some embodiments, the oligomer part of the conjugate may be or may comprise the first region, and optionally the second region. In this respect, in some embodiments, region B may form part of a contiguous nucleobase sequence which is complementary to the (nucleic acid) target. In other embodiments, region B may lack complementarity to the target.

Alternatively stated, in some embodiments, the invention provides a non-phosphodieser linked, such as a phosphorothioate linked, oligonucleotide (e.g. an antisense oligonucleotide) which has at least one terminal (5' and/or 3') DNA or RNA nucleoside linked to the adjacent nucleoside of the oligonucleotide via a phosphodiester linkage, wherein the terminal DNA or RNA nucleoside is further covalently linked to a conjugate moiety, a targeting moiety or a blocking moiety, optionally via a linker moiety.

The first region is covalently linked to the second region, such as via a 5' terminal or 3' terminal internucleoside linkage, such as a phosphodiester linkage. The phosphodiester linkage may therefore be positioned between the 5' most nucleoside of region A and the 3' most nucleoside of region B, and/or between the 3' most nucleoside of region A and the 5' most nucleoside of region B. In this respect, in some embodiments, there may be two region B covalently joined to region A, one at the 5' terminus of region A and one at the 3' terminus of region A. The two region Bs may be the same or different, and they may be covalently linked to the same or different third regions, optionally and independently via a linker (Y).

The oligomers may have a length of 8 - 35 contiguous nucleotides and comprise a first region of e.g. 7 - 25 contiguous nucleotides, and a second region of 1 - 10 contiguous nucleotides, wherein, for example, either the internucleoside linkage between the first and second region is a phosphodiester linked to the first (or only) DNA or RNA nucleoside of the second region, or region B comprises at least one phosphodiester linked DNA or RNA nucleoside.

The second region may, in some embodiments, comprise further DNA or RNA nucleosides which may be phosphodester linked. The second region is further covalently linked to a third region which may, for example, be or comprise the conjugate moiety.

The second region may comprise or consists of at least one DNA or RNA nucleosides linked to the first region via a phosphodiester linkage. In some aspects, the internucleoside linkage between the first and second region is considered as part of region B.

In some embodiments, the second region comprises or consists of at least between 1 and 10 linked nucleosides, such as 1, 2, 3, 4, 5, 6, 7, 8, 9 or 10 linked DNA or RNA nucleotides. Whilst a region of DNA/RNA phosphodiester is considered important in the provision of a cleavable linker, it is possible that region B also comprises sugar-modified nucleoside analogues, such as those referred to under the first region above. However in some embodiments, the nucleosides of region B are (optionally independently) selected from the group consisting of DNA and RNA. It will be recognised that the nucleosides of region B may comprise naturally occurring or non-naturally occurring nucleobases. Region B comprises at least one phosphodiester linked DNA or RNA nucleoside (which may, in some embodiments. be the first nucleoside adjacent to region A). If region B comprises other nucleosides, region B may also comprise of other nucleoside linkages other than phosphodiester, such as (optionally independently) phosphorothioate, phosphodithioate, boranophosphate or methyl phosphonate. However, in other embodiments, all the internucleoside linkages in region B are phosphorothioate. In some embodiments, all the nucleosides of region B comprise (optionally independently) either a 2'-OH ribose sugar (RNA) or a 2'-H sugar - i.e. RNA or DNA.

In some embodiments, the second region comprises or consists of at least between 1 and 10 (e.g. phosphodiester) linked DNA or RNA nucleosides, such as 1, 2, 3, 4, 5, 6, 7, 8, 9 or 10 (e.g. phosphodiester) linked DNA or RNA nucleotides.

In some embodiments, region B comprises no more than 3 or no more than 4 consecutive DNA or RNA nucleosides (such as DNA nucleosides. As such region B may be so short as it does not recruit RNAseH, an aspect which may be important when region B does not form a part of a single contiguous nucleobase sequence which is complementary to the target. Shorter region Bs, e.g. of 1 - 4nts in length may also be preferable in some embodiments, as they are unlikely to be the target of sequence specific restriction enzymes. As such it is possible to vary the susceptibility of the region B to endonuclease cleavage, and thereby fine-tune the rate of activation of the active oligomer in vivo, or even intra-cellular. Suitably, if very rapid activation is required, longer region Bs may be employed and/or region Bs which comprise the recognition sites of (e.g. cell or tissue specific or differentially expressed) restriction enzymes.

As illustrated in the examples, region B may be conjugated to a functional group (X), such as the conjugate, targeting reactive group, an activation group, or blocking group via a linker group which may, for example, comprise a phosphodiester linkage, and/or optionally a suitable linker group, such as those provided herein. For example a phosphate nucleoside linkage (e.g. phosphodiester, phosphorothioate, phosphodithioate, boranophosphate or methylphosphonate) or a triazol group. In some aspects, the linkage group is the same as the linkage group between regions A and B, and as such may be a phosphodiester linkage. In some aspects, the linkage group is a phiosphorothioate linkage.

In some embodiments the DNA or RNA nucleotides of the second region are independently selected from DNA and RNA nucleotides. In some embodiments the DNA or RNA nucleotides of the second region are DNA nucleotides. In some embodiments the DNA or RNA nucleotides of the second region are RNA nucleotides.

In the context of the second region, the term DNA and RNA nucleoside may comprise a naturally occurring or non-naturally occurring base (also referred to as a base analogue or modified base).

It will be recognized that, in some embodiments, the second region may further comprise other nucleotides or nucleotide analogues. In some embodiments, the second region comprises only DNA or RNA nucleosides. In some embodiments, when the second region comprises more than one nucleoside, the internucleoside linkages in the second region comprise phosphodiester linkages. In some embodiments, when the second region comprises more than one nucleoside, all the internucleoside linkages in the second region comprise phosphodiester linkages.

In some embodiments, at least two consecutive nucleosides of the second region are DNA nucleosides (such as at least 3 or 4 or 5 consecutive DNA nucleotides). In some embodiments the at least two consecutive nucleosides of the second region are RNA nucleosides (such as at least 3 or 4 or 5 consecutive RNA nucleotides). In some embodiments the at least two consecutive nucleosides of the second region are at least one DNA and at least one RNA nucleoside. The internucleoside linkage between region A and region B is a phosphodiester linkage. In some embodiments, when region B comprises more than one nucleoside, at least one further internucleoside linkage is phosphodiester - such as the linkage group(s) between the 2 (or 3 or 4 or 5) nucleosides adjacent to region A.

The second region is flanked on one side (either 5' or 3') by the first region, e.g. an antisense oligonucleotide, and on the other side (either 3' or 5' respectfully, via a conjugate moiety or similar group (e.g. a blocking moiety/group, a targeting moiety/group or therapeutic small molecule moiety), optionally via a linker group (i.e. between the second region and the conjugate/blocking group etc. moiety).

In such an embodiment, the oligonucleotide of the invention may be described according to the following formula:

5'-A-PO-B [Y)X- 3'

or

3'-A-PO-B [Y)X- 5'

wherein A is region A, PO is a phosphodiester linkage, B is region B, Y is an optional linkage group, and X is the conjugate group.

In some embodiments, region B comprises 3' - 5' or 5'-3': i) a phosphodiester linkage to the 5' or 3' nucleoside of region A, ii) a DNA or RNA nucleoside, such as a DNA nucleoside, and iii) a further phosphodiester linkage

5'-A-PO-B - PO- 3'

or

3'-A-PO-B - PO- 5'

The further phosphodiester linkage link the region B nucleoside with one or more further nucleoside, such as one or more DNA or RNA nucleosides, or may link to X (is the conjugate group) optionally via a linkage group (Y).

In some embodiments, region B comprises 3' - 5' or 5'-3': i) a phosphodiester linkage to the 5' or 3' nucleoside of region A, ii) between 2 - 10 DNA or RNA phosphodiester linked nucleosides, such as a DNA nucleoside, and optionally iii) a further phosphodiester linkage:

5'-A-[PO-B]n-[Y]-X3'

or

3'-A-[PO-B]n-[Y]-X5'

5'-A-[PO-B]n - PO-[Y]-X 3'

or

3'-A-[PO-B]n - PO-[Y]-X 5'

Wherein A represent region A, [PO-B]n represents region B, wherein n is 1 - 10, such as 1, 2, 3,4, 5, 6, 7, 8, 9 or 10, PO is an optional phosphodiester linkage group between region B and X (or Y if present).

In some embodiments the invention provides compounds according to (or comprising) one of the following formula:

5'[RegionA]-PO-[regionB]3'-Y-X

5'[RegionA]-PO-[regionB]-PO3'-Y-X

5'[RegionA]-PO-[regionB]3'-X

5'[RegionA]-PO-[regionB]-PO3'-X

3'[RegionA]-PO-[regionB]5'-Y-X

3'[RegionA]-PO-[regionB]-PO5'-Y-X

3'[RegionA]-PO-[regionB]5'-X

3'[RegionA]-PO-[regionB]-PO5'-X

Region B, may for example comprise or consist of:
5' DNA3'
3' DNA 5'
5' DNA-PO-DNA-3'
3' DNA-PO-DNA-5'
5' DNA-PO-DNA-PO-DNA 3'
3' DNA-PO-DNA-PO-DNA 5'
5' DNA-PO-DNA-PO-DNA-PO-DNA 3'
3' DNA-PO-DNA-PO-DNA-PO-DNA 5'
5' DNA-PO-DNA-PO-DNA-PO-DNA-PO-DNA 3'
3' DNA-PO-DNA-PO-DNA-PO-DNA-PO-DNA 5'

### Sequence selection in the second region:

In some embodiments, region B does not form a complementary sequence when the oligonucleotide region A and B is aligned to the complementary target sequence.

In some embodiments, region B does form a complementary sequence when the oligonucleotide region A and B is aligned to the complementary target sequence. In this respect region A and B together may form a single contiguous sequence which is complementary to the target sequence.

In some embodiments, the sequence of bases in region B is selected to provide an optimal endonuclease cleavage site, based upon the predominant endonuclease cleavage enzymes present in the target tissue or cell or sub-cellular compartment. In this respect, by isolating cell extracts from target tissues and non-target tissues, endonuclease cleavage sequences for use in region B may be selected based upon a preferential cleavage activity in the desired target cell (e.g. liver/hepatocytes) as compared to a non-target cell (e.g. kidney). In this respect, the potency of the compound for target down-regulation may be optimized for the desired tissue/cell.

In some embodiments region B comprises a dinucleotide of sequence AA, AT, AC, AG, TA, TT, TC, TG, CA, CT, CC, CG, GA, GT, GC, or GG, wherein C may be 5-mthylcytosine, and/or T may be replaced with U. In some embodiments region B comprises a trinucleotide of sequence AAA, AAT, AAC, AAG, ATA, ATT, ATC, ATG, ACA, ACT, ACC, ACG, AGA, AGT, AGC, AGG, TAA, TAT, TAC, TAG, TTA, TTT, TTC, TAG, TCA, TCT, TCC, TCG, TGA, TGT, TGC, TGG, CAA, CAT, CAC, CAG, CTA, CTG, CTC, CTT, CCA, CCT, CCC, CCG, CGA, CGT, CGC, CGG, GAA, GAT, GAC, CAG, GTA, GTT, GTC, GTG, GCA, GCT, GCC, GCG, GGA, GGT, GGC, and GGG wherein C may be 5-mthylcytosine and/or T may be replaced with U. In some embodiments region B comprises a trinucleotide of sequence AAAX, AATX, AACX, AAGX, ATAX, ATTX, ATCX, ATGX, ACAX, ACTX, ACCX, ACGX, AGAX, AGTX, AGCX, AGGX, TAAX, TATX, TACX, TAGX, TTAX, TTTX, TTCX, TAGX, TCAX, TCTX, TCCX, TCGX, TGAX, TGTX, TGCX, TGGX, CAAX, CATX, CACX, CAGX, CTAX, CTGX, CTCX, CTTX, CCAX, CCTX, CCCX, CCGX, CGAX, CGTX, CGCX, CGGX, GAAX, GATX, GACX, CAGX, GTAX, GTTX, GTCX, GTGX, GCAX, GCTX, GCCX, GCGX, GGAX, GGTX, GGCX, and GGGX, wherein X may be selected from the group consisting of A, T, U, G, C and analogues thereof, wherein C may be 5-mthylcytosine and/or T may be replaced with U. It will be recognised that when referring to (naturally occurring) nucleobases A, T, U, G, C, these may be substituted with nucleobase analogues which function as the equivalent natural nucleobase (e.g. base pair with the complementary nucleoside).

The application describes compounds that may comprise more than one conjugate group (or more than one functional group X - such as a conjugate, targeting, blocking or activated group or a reactive or activation group), such as 2 or 3 such groups. In some embodiments, region B is covalently linked, optionally via a [e.g. non-nucleotide] linker group), to at least one functional group, such as two or three functional groups. The first region may be covalently linked (e.g. via internucleoside linkages, such as phosphodiester linkages), to two region Bs, for example, one 5' and one 3' to the first region, wherein each region B may be (optionally independently) selected from the region B described herein. In this respect one region B may have one or more functional groups, and the second region B may have one or more function groups, wherein the functional groups of each region B may be independently selected from a conjugate, a targeting group, a blocking group or a reactive/activation group.

The use of a region B, such as a "PO DNA linker", as decribed above, between the oligomer and the conjugate moiety (X, or X-Y), is particularly advantageous as it ensures a uniform cleavage of the conjugate moiety from the oligomer sequence, once the oligomer has been delivered to the target cell (e.g. a hepatocyte). Uniform cleavage may be useful in retaining maximal intra-cellular potency of the parent compound, as well as enhancing the safety profile of the oligomer conjugate.

### Poly oligomeric compounds

The application describes a poly oligomeric compound which may comprise the first region (region A), (optionally the second region (region B)) and the third region (region C), wherein the first region is covalently linked to at least one further oligomeric compound (region A'), wherein the first region (region A) and region A' are covalently linked via a biocleavable linker (region B'), which may be, by way of example, as according to the second region (region B) as disclosed here, for example a region of at least one phosphodiester linked DNA or RNA (such as DNA), such as two, three, four or five phosphodiester linked DNA or RNA nucleosides (such as DNA nucleosides). In embodiments comprising a region B, regions B and B' may, in some embodiments have the same structure, e.g. the same number of DNA/RNA nucleosides and phosphodiester linkages and/or the same nucleobase sequence. In other embodiments Regions B and B' may be different. By way of example such poly oligomeric compounds may have a structure such as: (5' - 3' or 3' - 5') Conjugate-PO-ON-PO'-ON', wherein conjugate is region C, PO is region B, PO' is region B', and ON 1 is region A, and ON' is region A'

It should be understood that oligomer region A' may, in some embodiments, comprise multiple further oligomeric compounds (such as a further 2 or 3 oligomeric compounds) linked in series (or in parallel) via biocleavable linkers, for example: Conjugate-PO-ON-PO-ON'-PO"-ON", or Conjugate-PO-ON-[PO-ON']n, wherein n may, for example be 1, 2 or 3, and each ON' may be the same or different, and if different may have the same or different targets.

The application describes for an oligomeric compound comprising a contiguous nucleotide sequence of formula [LNAₛ]₇₋₁₈-[DNA]₁₋₅-[LNAₛ]₇₋₁₈, and a non-nucleobase conjugate, such as a GalNAc conjugate moeity, for example a trivalent GalNAc conjugate conjugate, such as a conjugate moeity selected from the group consisting of any one of Conj1, 2, 3, 4, 1a, 2a, 3a, 4a, or other trivalent GalNAc conjugates, such as those disclosed herein. Subscript s refers to a phosphorothioate linkage. At least one internucleoside linkage within or adjacent to the - [DNA]₁₋₅₋ region are phosphodiester linkages. In some embodiments, all internucleoside linkages within or adjacent to the -[DNA]₁₋₅₋ region are phosphodiester linkages. In some embodiments, the -[DNA]₁₋₅₋ region has 2, 3, 4 or 5 contiguous DNA nucleoside which are joined by phosphodiester linkages. In such an embodiment, the internucleoside linkages between the -[DNA]₂₋₅₋ are phosphodiester linkages, and optionally the internucleoside linkages between region -[DNA]₁₋₅ and the LNA regions [LNAₛ]₇₋₁₈ are independently phosphorothioate or phosphodiester linkages, such as both phosphodiester or both phosphorothioate, or one phosphodiester and one phosphorothioate. In the embodiment when the DNA region is a single DNA nucleoside, at least one or both the the internucleoside linkages adjacent to the DNA region is a phosphodiester, and if only a single phosphodiester, the other may be a phosphorothioate. The region -[DNA]₁₋₅ may be as defined as described by region B herein - *i.e.* may be a physiologically cleavable nucleoside linker region. Each [LNAₛ]₇₋₁₈ is a LNA phosphorothioate oligomer, and may for example be independently selected from the group consisting of an LNA gapmer, an LNA mixmer or an LNA totalmer. The GalNAc conjugate may for example be located 5' or 3' to the contiguous nucleotide sequence. In a preferred embodiment, at least one of the LNA oligomers, or both the poly oligomer conjugate is a LNA totalmer of 7 - 12, such as 8, 9 or 10 nucleotides in length. In some embodiments, the LNA totalmer may comprise only LNA nucleotides, such as beta-D-oxy LNA nucleoside, which are linked by phosphorothioate linkages. For example the poly oligomer conjugate may comprise a contiguous nucleositide sequence [LNAₛ]₇₋₁₀-[DNA]₁₋₅-[LNAₛ]₇₋₁₀, such as [LNAₛ]₇₋₁₀-[DNA]₂-[LNAₛ]₇₋₁₀ or [LNAₛ]₇₋₁₀-[DNA]₃-[LNAₛ]₇₋₁₀ or [LNAₛ]₇₋₁₀-[DNA]₄-[LNAₛ]₇₋₁₀. In one embodiment the contiguous nucleositide sequence comprises [LNAₛ]₈-[DNA]₁₋₅-[LNAₛ]₈, such as [LNAₛ]₈-[DNA]₂-[LNAₛ]₈, [LNAₛ]₈-[DNA]₃-[LNAₛ]₈, or [LNAₛ]₈-[DNA]₄-[LNAₛ]₈. Such poly oligomeric complexes are particularly useful to target microRNAs, such as mature microRNAs. By utilising a first LNA oligomer region which targets a first target (e.g. a mRNA, a microRNA, or a viral sequence), and a second LNA oligomer region which targets a second target (e.g. a mRNA, a microRNA, or a viral sequence), single compounds can be made which target two distinct targets, for example, the first oligomer region may target ApoB, and the second oligomer region may target another mRNA, such as mtGPAT mRNA, for example:
Region C-5' [SEQ ID No 50]-[region B]-[SEQ ID No 59] 3'. (e.g. SEQ ID NO 61: (Trivalent GalNAc)- GₛTₛtₛgₛaₛcₛaₛcₛtₛgₛTₛCcaAₛTₛTₛcₛcₛcₛtₛgₛcₛcₛtₛGₛTₛG-3')

Region C may be a trivalent GalNAc conjugates, such as Conj2a, or other GalNAc conjugates disclosed herein. Region B may be a DNA dinucleotide "ca", where the internucleotise linkages between the DNA dinucleotide and adjacent to the dinucleotide are phosphodiester.

By utilising a first LNA oligomer regions (e.g. [LNAₛ]₇₋₁₀) which targets one microRNA, and a second LNA oligomer region which targets a second microRNA, single compounds can be made which target two different microRNA targets, for example miR-21 and miR-221, both of which are indicated in hepatocellular carcinoma. Alternatively the first and the second may target the same microRNA, such as e.g. miR-122, miR-21, miR-155, miR-33, miR-221, which allows two oligomers to be delivered to the target cell for a single conjugate moiety.

This of particular importance for receptor mediate conjugate targeting, such as with asialoglycoprotein receptor conjugates, where the receptor mediated uptake of e.g. GalNAc conjugated oligomers is limited by the availability of free receptors on the surface of the target cell, the use of poly-oligomer conjugates allows for enhanced delivery to the target cell. It is also important to avoid complete saturation of cell -suface receptors which are performing an important biological function, the use of the poly-oligomer strategy therefore allows for effective delivery of sufficient compound to ensure relevant pharmacology, whilst reducing the risk of side effects due to receptor saturation/competition by the conjugate moiety. The use of the poly-oligomer conjugate therefore provides an effective solution for enhancing the therapeutic index - increased oligomer delivery and activity with a reduction of undesirable side-effects.

### Compositions

The LNA antisense oligomer conjugate of the invention may be used in pharmaceutical formulations and compositions. Suitably, such compositions comprise a pharmaceutically acceptable diluent, carrier, salt or adjuvant. WO2007/031091 provides suitable and preferred pharmaceutically acceptable diluent, carrier and adjuvants. Suitable dosages, formulations, administration routes, compositions, dosage forms, combinations with other therapeutic agents, pro-drug formulations are also provided in WO2007/031091.

Antisense compounds may be admixed with pharmaceutically acceptable active or inert substances for the preparation of pharmaceutical compositions or formulations. Compositions and methods for the formulation of pharmaceutical compositions are dependent upon a number of criteria, including, but not limited to, route of administration, extent of disease, or dose to be administered.

An antisense compound can be utilized in pharmaceutical compositions by combining the antisense compound with a suitable pharmaceutically acceptable diluent or carrier. A pharmaceutically acceptable diluent includes phosphate-buffered saline (PBS). PBS is a diluent suitable for use in compositions to be delivered parenterally.

Pharmaceutical compositions comprising antisense compounds encompass any pharmaceutically acceptable salts, esters, or salts of such esters, or any other oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts. In some embodiments, the oligomer of the invention is a pro-drug.

### Applications

The LNA antisense oligomer conjugate of the invention may be utilized as research reagents for, for example, diagnostics, therapeutics and prophylaxis.

In research, in some embodiments, such conjugates may be used to specifically inhibit the synthesis of protein (typically by degrading or inhibiting the mRNA and thereby prevent protein formation) in cells and experimental animals thereby facilitating functional analysis of the target or an appraisal of its usefulness as a target for therapeutic intervention.

For therapeutics, an animal or a human, suspected of having a disease or disorder, which can be treated by modulating the expression of the target is treated by administering conjugates in accordance with this invention. Further provided are methods of treating a mammal, such as treating a human, suspected of having or being prone to a disease or condition, associated with expression of the target by administering a therapeutically or prophylactically effective amount of one or more of the conjugates or compositions of the invention. The oligomer, a conjugate or a pharmaceutical composition according to the invention is typically administered in an effective amount.

The invention also provides for the use of the conjugate of the invention as described for the manufacture of a medicament for the treatment of a disorder as referred to herein, or for a method of the treatment of as a disorder as referred to herein. In some embodiments the disease or disorder is a liver related or liver associated disease or disorder.

The application describes a method for treating a disorder as referred to herein said method comprising administering a conjugate according to the invention, and/or a pharmaceutical composition according to the invention to a patient in need thereof.

### Medical Indications

The invention provides for the LNA antisense oligomer conjugate for use in medicine.

The invention provides for the LNA antisense oligomer conjugate, for use in down-regulating a liver-expressed RNA. The invention provides for the LNA antisense oligomer conjugate, for use in treatment of a metabolic disease or disorder, such as a hepatic disease or disorder. The invention provides for the LNA antisense oligomer conjugate, for use in treatment of hepatitis, such as hepatitis B or C.

In some embodiments, the disease is liver disease or disorder. In some embodiments, the disease or disorder is, or results in or is associated with liver-fibrosis. In some embodiments, the disease or disorder is blood clotting disorder, such as treating or preventing thromboembolic complications, such as thrombosis, embolism, and thromboembolism, deep vein thrombosis, pulmonary embolism, myocardial infarction, & stroke.. FactorVII targeting oligomers may be used for the treatment of blood clotting disorders or otherwise to control the blood clotting in a subject - see US2010/0298417 and WO2012/174154 for examples of Factor VII antisense oligonucleotides.

FactorVII targeting oligomers may also be used for the treatment of inflammatory disease/disorders, cancer, rheumatoid arthritis and fibrosis (see US2010/0298417 and WO2012/174154). In some embodiments, the above diseases and disorders may therefore also be treated by the compounds of the invention. In some embodiments, the invention therefore provides for the oligomers for use in the treatment of treatment of inflammatory disease/disorders, cancer, rheumatoid arthritis and fibrosis; or a disease or disorder selected from the group consisting of blood clotting disorder, such as treating or preventing thromboembolic complications, such as thrombosis, embolism, and thromboembolism, deep vein thrombosis, pulmonary embolism, myocardial infarction, & stroke.

In some embodiments, the disease or disorder is a liver disease or disorder.

In some embodiments the disease or disorder is a metabolic disorder, which may for example be a liver disease or disorder, and/or in some aspects a cardiovascular disease or disorder).

Cardiovascular/Metabolic diseases include, for examples, metabolic syndrome, obesity, hyperlipidaemia , atherosclerosis, HDL/LDL cholesterol imbalance, dyslipidemias, e.g., familial combined hyperlipidaemia (FCHL), acquired hyperlipidaemia, statin-resistant, hypercholesterolemia, coronary artery disease (CAD), and coronary heart disease (CHD)., atherosclerosis, heart disease, diabetes (I and/or II), NASH, acute coronary syndrome (ACS), NASH, chronic heart failure, cardiovascular disease, cardio metabolic disease, hyperlipidaemia and related disorders, metabolic syndrome, atherosclerosis, chronic heart failure, vascular disease, peripheral arterial disease, heart disease, ischemia, type 2 diabetes and/or type 1 diabetes,

In some embodiments, the disease or disorder is selected from the group consisting of metabolic syndrome, obesity, hyperlipidaemia, atherosclerosis, HDL/LDL cholesterol imbalance, dyslipidemias, e.g., familial combined hyperlipidaemia (FCHL), acquired hyperlipidaemia, statin-resistant, hypercholesterolemia, coronary artery disease (CAD), and coronary heart disease (CHD).

In some embodiments, the disease or disorder is selected from the group consisting of chronic heart failure, cardiovascular disease, cardio metabolic disease, chronic heart failure, vascular disease, peripheral arterial disease, heart disease, ischemia, acute coronary syndrome (ACS).

In some embodiments, the disease or disorder is type 2 diabetes and/or type 1 diabetes.

In some embodiments, the disease or disorder is a viral disease, such viral hepatitis, such as hepatitis C, hepatitis B. In some embodiments, the liver disease may be a disease or disorder selected from the group consisting of non-alcoholic fatty liver disease and non-alcoholic steatohepatitis.

The invention further provides use of a cpnjugate of the invention in the manufacture of a medicament for the treatment of a disease, disorder or condition, such as those as referred to herein.

Generally stated, to the application describes a method of treating a mammal suffering from or susceptible to conditions associated with abnormal levels of the target, comprising administering to the mammal and therapeutically effective amount of an oligomer targeted to the target that comprises one or more LNA units. The oligomer, a conjugate or a pharmaceutical composition is typically administered in an effective amount.

An interesting aspect of the invention is directed to the use of the conjugate as defined herein for the preparation of a medicament for the treatment of a disease, disorder or condition as referred to herein.

A patient who is in need of treatment is a patient suffering from or likely to suffer from the disease or disorder.

In some embodiments, the term 'treatment' as used herein refers to both treatment of an existing disease (*e.g.* a disease or disorder as herein referred to), or prevention of a disease, *i.e.* prophylaxis. It will therefore be recognized that treatment as referred to herein may, in some embodiments, be prophylactic.

### EXAMPLES

Mouse Experiments: Unless otherwise specified, the mouse experiments may be performed as follows:

### Dose administration and sampling:

7-10 week old C57BI6-N mice were used, animals were age and sex matched (females for study 1, 2 and 4, males in study 3). Compounds were injected i.v. into the tail vein. For intermediate serum sampling, 2-3 drops of blood were collected by puncture of the vena facialis, final bleeds were taken from the vena cava inferior. Serum was collected in gel-containing serum-separation tubes (Greiner) and kept frozen until analysis.

C57BL6 mice were dosed i.v. with a single dose of 1mg/kg ASO (or amount shown) formulated in saline or saline alone according to the information shown. Animals were sacrificed at e.g. day 4 or 7 (or time shown) after dosing and liver and kidney were sampled. RNA isolation and mRNA analysis: mRNA analysis from tissue was performed using the Qantigene mRNA quantification kit ("bDNA-assay", Panomics/Affimetrix), following the manufacturers protocol. For tissue lysates, 50-80 mg of tissue was lysed by sonication in 1 ml lysis-buffer containing Proteinase K. Lysates were used directly for bDNA-assay without RNA extraction. Probesets for the target and GAPDH were obtained custom designed from Panomics. For analysis, luminescence units obtained for target genes were normalized to the housekeeper GAPDH.

Serum analysis for ALT, AST and cholesterol was performed on the "Cobas INTEGRA 400 plus" clinical chemistry platform (Roche Diagnostics), using 10µl of serum.

For quantification of Factor VII serum levels, the BIOPHEN FVII enzyme activity kit (#221304, Hyphen BioMed) was used according to the manufacturer's protocol.

For oligonucleotide quantification, a fluorescently-labeled PNA probe is hybridized to the oligo of interest in the tissue lysate. The same lysates are used as for bDNA-assays, just with exactly weighted amounts of tissue. The heteroduplex is quantified using AEX-HPLC and fluorescent detection.

### Example 1: Oligonucleotide synthesis

The following LNA gapmer oligonucleotides were prepared based on the same 13mer mouse Factor VII sequence.

| | SEQ ID NO | Structure | |
|---|---|---|---|
| A | 1 | LsLsDsDsDsDsDsDsDsDsLsLsL | Unconjugated LNA |
| B | 2 | (NH2C6)LsLsDsDsDsDsDsDsDsDsLsLsL | Precursor for GalNac conjugate |
| C | 3 | (GalNac)(NHC6)LsLsDsDsDsDsDsDsDsDsLsLsL | GalNac conjugate |
| D | 4 | (Chol1)(C6SSC6)LsLsDsDsDsDsDsDsDsDsLsLsL | Cholesterol Conjugate, full PS |
| E | 5 | (Chol1)(C6SSC6)LpLpDpDpDpDpDpDpDpDsLsLsL | Cholesterol Conjugate, partial PS |

Key: Upper case L: beta-D-oxy LNA; s: phosphorothioate; upper case D: DNA; (NH2C6): Aminolinker; (Chol1): cholesterol; (C6SSC6): bio-cleavable disulfide linker.

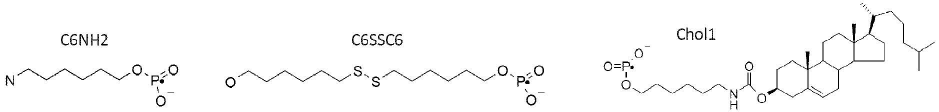

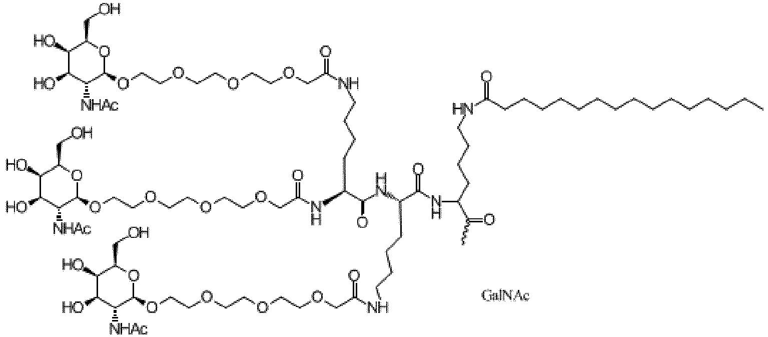

Other than the GalNac conjugate, compounds were synthesized via solid phase synthesis using commercially available phosphormidites, and purified via IEX HPLC. The trivalent GalNAc cluster was prepared according to US2012/0157509 (see Figure 1).

### Example 2: In vivo inhibition of FVII comparing GalNac and cholesterol conjugates

An *in vivo* mouse study was prepared testing GaINac and cholesterol conjugates side by side, using a total of 9 groups of mice (n03). Each mouse was administered a single *i.v.* dose of LNA compound, at either 1mg/kg or 4mg/kg. A saline control group was included. The mice were pre-bled 1 day before administration, and subsequent bleeds were taken at 6hours, 24hours, 48hours and after 3 days the mice were sacrificed and liver kidney and blood samples taken.

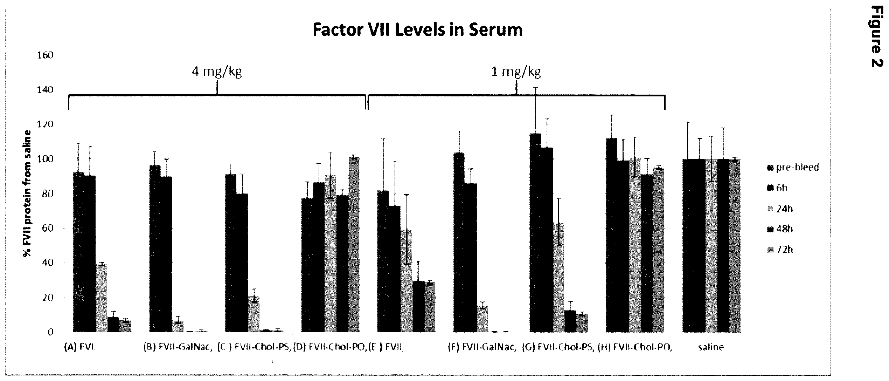

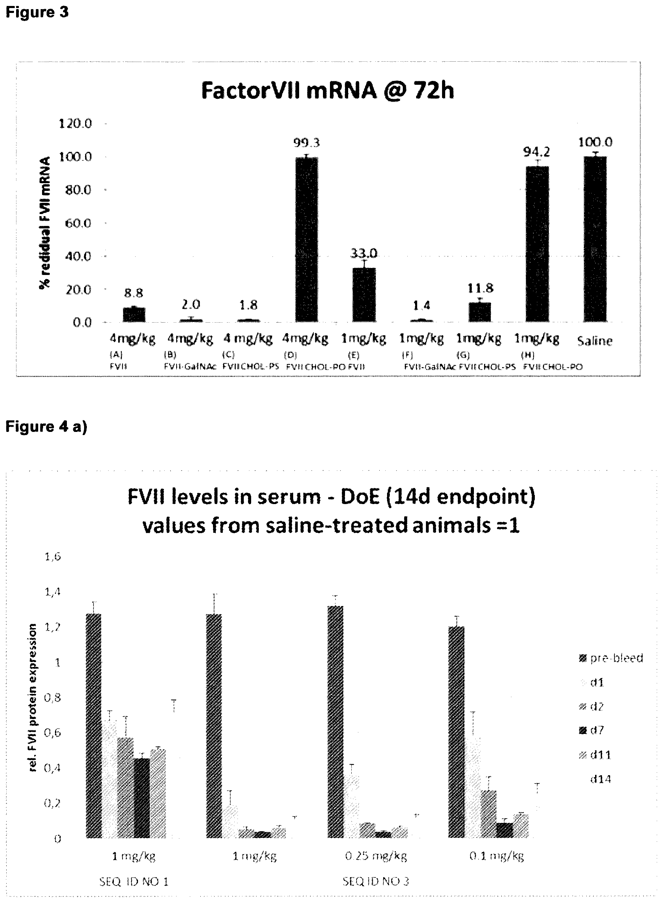

FactorVII serum levels and mRNA levels were measured using standard assay techniques (see Figure 2 and 3). Both FVII GaINac and cholesterol (phosphorothioate compound) improvied FVII knock-down in serum with the GalNac being more effective than cholesterol (see Fig 2 1mg/kg).

### Example 3: In vivo inhibition of FVII GaINac and cholesterol conjugates dose escalation study.

### Compounds

| SEQ ID NO | Seq (5'-3') (A) | Cleavable linker (B) | Conjugate (C) |
|---|---|---|---|
| 1 | LₛLₛdₛdₛdₛdₛdₛdₛdₛdₛLₛLₛL | no | no |
| 3 | LₛLₛdₛdₛdₛdₛdₛdₛdₛdₛLₛLₛL | | GalNAc cluster Conj1a |
| 6 | LₛLₛdₛdₛdₛdₛdₛdₛdₛdₛLₛLₛL | 2PO dd (5' ca 3') | GalNAc cluster Conj1a |
| 4 | LₛLₛdₛdₛdₛdₛdₛdₛdₛdₛLₛLₛL | SS | Cholesterol |
| 7 | LₛLₛdₛdₛdₛdₛdₛdₛdₛdₛLₛLₛL | 2PO dd (5' ca 3') | Cholesterol |

Capital L is a LNA nucleoside (such as beta-D-oxy LNA), lower case d is a DNA nucleoside. Subscript s represents a phosphorothioate internucleoside linkage (region A). The 2PO linker (region B) is 5' to the sequence region A, and comprises of two DNA nucleosides linked by phosphodiester linkage, with the internucleoside linkage between the 3' DNAnucleoside of region A and the 5' LNA nucleoside of region A also being phosphodiester. A linkage group (Y) is used to link the conjugate group, when present, to region B, or A (e.g. SEQ ID NO 3).

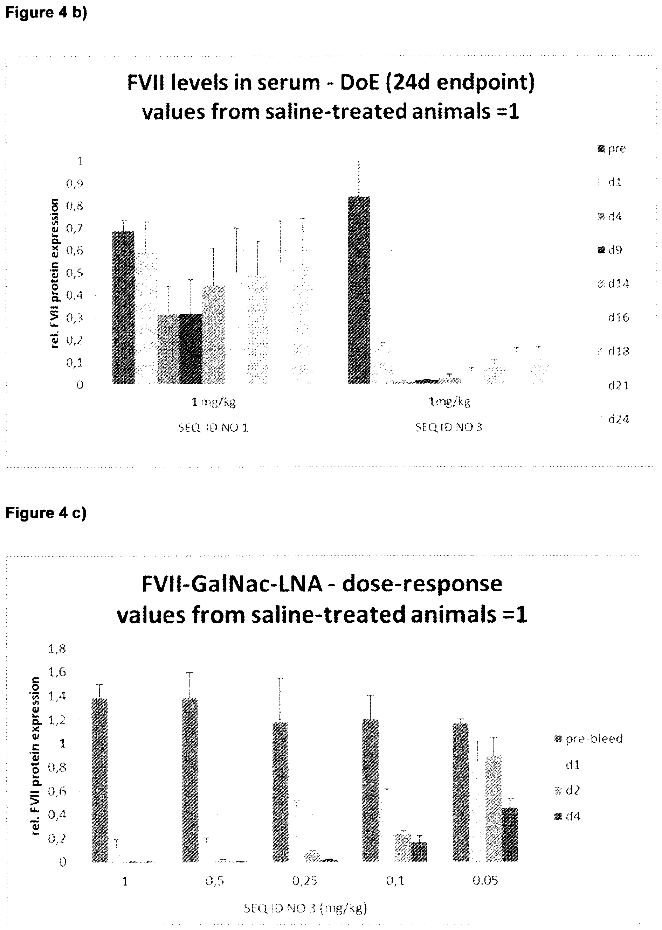

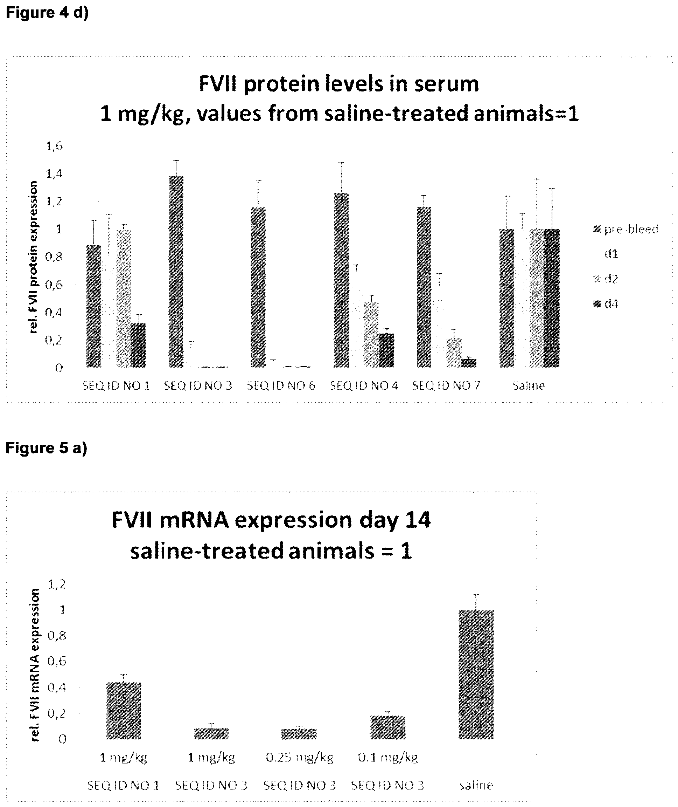

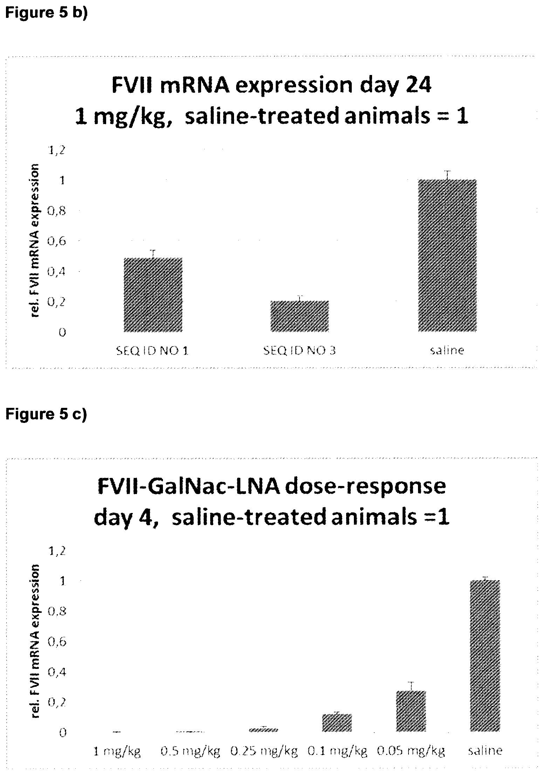

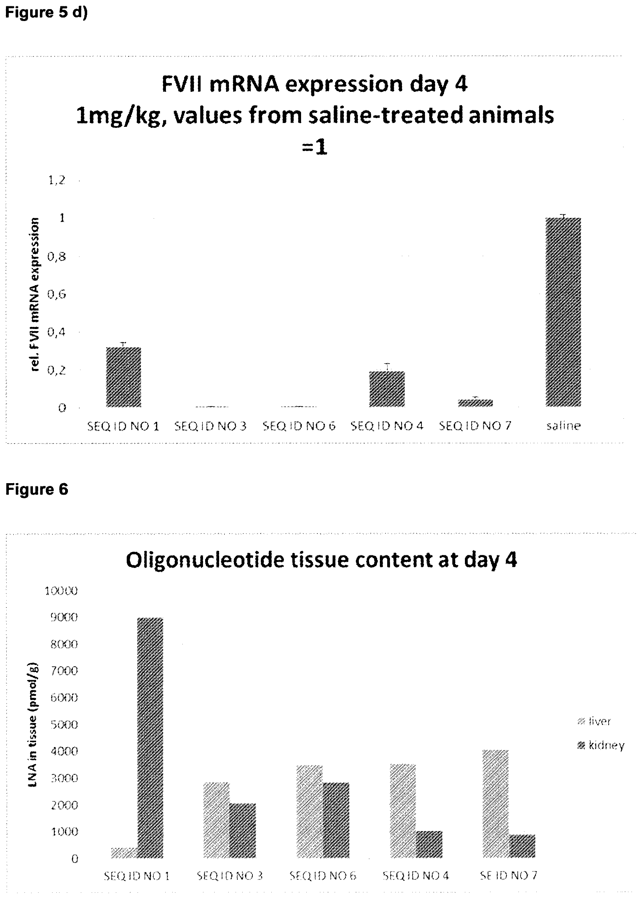

An *in vivo* mouse study was prepared using a total of 16 groups of mice (n=3). Each mouse was administered a single *i.v.* dose of LNA compound, at either 1, 0,5, 0,25, 0,1, or 0,05mg/kg. A saline control group was included. The mice were pre-bled 1 day before administration, and subsequent bleeds were taken at varying time points during the study. The mice were sacrificed at days 4, 14, or 24 and liver, kidney, and blood samples taken. See table below for study setup. FactorVII serum levels (figure 4), mRNA levels (figure 5), and oligonucleotide tissue content (Figure 6) were measured using standard assay techniques

Conclusions: GalNAc conjugated to a FVII targeting LNA oligonucleotide (SEQ ID NO 3 and 6) show very good activity on FVII protein in serum (Figure 4) and mRNA in liver (Figure 5) compared to the unconjugated LNA oligonucleotide (SEQ ID NO 1). Even at very low doses the activity is pronounced. Moreover, it is seen that the GalNAc cluster is more active for FVII protein and mRNA compared to the cholesterol conjugate (SEQ ID NO 4 and 7) (figure 4, d and Figure 5 d)). The tissue content in liver and kidney is shown in figure 6 where it is seen that the GalNAc cluster (SEQ ID NO 3 and 6) enhances the uptake in liver and gives lower uptake in kidney when compared to the unconjugated LNA FVII targeting oligonucleotide (SEQ ID NO 1). When comparing GalNAc conjugated LNA oligonucleotides (SEQ ID NO 3 and 6) to the cholesterol conjugated LNA oligonucleotides (SEQ ID NO 4 and 7) it is seen that the level reached in liver is similar but since the GalNAc conjugated LNA oligonucleotides had showed better activity they seem to give a better specific activity than the cholesterol conjugated LNA oligonucleotides. It appears that the PO linker may enhance tissue uptake (figure 6) and may also result in a higher potency (Figure 4).

### Materials and Methods:

### Experimental design:

| group | compound | termination time point post dose | group size | dose (d0) mg/kg |
|---|---|---|---|---|
| 1 | saline | d4 | 3 | none |
| 2 | | d14 | 3 | none |
| 3 | | d24 | 3 | none |
| 4 | SEQ ID NO1 | d4 | 3 | 1 |
| 5 | | d14 | 3 | 1 |
| 6 | | d24 | 3 | 1 |
| 7 | SEQ ID NO 3 | d4 | 3 | 1 |
| 8 | | d14 | 3 | 1 |
| 9 | | d24 | 3 | 1 |
| 10 | SEQ ID NO 3 | d4 | 3 | 0,5 |
| 11 | | d4 | 3 | 0,25 |
| | | d14 | 3 | 0,25 |
| 12 | | d4 | 3 | 0,1 |
| 13 | | d14 | 3 | 0,1 |
| | | d4 | 3 | 0,05 |
| 14 | SEQ ID NO 6 | d4 | 3 | 1 |
| 15 | SEQ ID NO 4 | d4 | 3 | 1 |
| 16 | SEQ ID NO 7 | d4 | 3 | 1 |

Female mice were administered iv and liver, kidney, and blood were sampled at sacrifice all according to the above scheme.

### Example 4. In vivo silencing of ApoB mRNA with different monoGalNAc conjugates.

### Compounds.

Oligonucleotides were synthesized on uridine universal supports using the phosphoramidite approach on an Expedite 8900/MOSS synthesizer (Multiple Oligonucleotide Synthesis System) at 4 µmol scale. At the end of the synthesis, the oligonucleotides were cleaved from the solid support using aqueous ammonia for 1-2 hours at room temperature, and further deprotected for 16 hours at 65°C. The oligonucleotides were purified by reverse phase HPLC (RP-HPLC) and characterized by UPLC, and the molecular mass was further confirmed by ESI-MS. See below for more details.

### Elongation of the oligonucleotide

The coupling of β-cyanoethyl- phosphoramidites (DNA-A(Bz), DNA- G(ibu), DNA- C(Bz), DNA-T, LNA-5-methyl-C(Bz), LNA-A(Bz), LNA- G(dmf), LNA-T or C6-S-S linker) is performed by using a solution of 0.1 M of the 5'-O-DMT-protected amidite in acetonitrile and DCI (4,5-dicyanoimidazole) in acetonitrile (0.25 M) as activator. For the final cycle a commercially available C6-linked cholesterol phosphoramidite was used at 0.1M in DCM. Thiolation for introduction of phosphorthioate linkages is carried out by using xanthane hydride (0.01 M in acetonitrile/pyridine 9:1). Phosphordiester linkages are introduced using 0.02 M iodine in THF/Pyridine/water 7:2:1. The rest of the reagents are the ones typically used for oligonucleotide synthesis. For post solid phase synthesis conjugation a commercially available C6 aminolinker phorphoramidite was used in the last cycle of the solid phase synthesis and after deprotection and cleavage from the solid support the aminolinked deprotected oligonucleotide was isolated. The conjugates were introduced via activation of the functional group using standard synthesis methods.

| SEQ ID NO | Seq (5'-3') (A) | Cleavable Linker (B) | Conjugate (C) |
|---|---|---|---|
| 8 | GₛCₛaₛtₛtₛgₛgₛtₛaₛtₛTₛCₛA | no | monoGalNAc |
| 10 | GₛCₛaₛtₛtₛgₛgₛtₛaₛtₛTₛCₛA | 2PO-DNA (5'ca3') | monoGalNAc |
| 9 | GₛCₛaₛtₛtₛgₛgₛtₛaₛtₛTₛCₛA | ss linker | monoGalNAc |
| 11 | GₛCₛaₛtₛtₛgₛgₛtₛaₛtₛTₛCₛA | | GalNAc cluster Conj2a |
| 12 | GₛCₛaₛtₛtₛgₛgₛtₛaₛtₛTₛCₛA | no | no |

Capital letters are LNA nucleosides (such as beta-D-oxy LNA), lower case letters are a DNA nucleoside. Subscript s represents a phosphorothioate internucleoside linkage (region A). LNA cytosines are optionally 5-methyl cytosine. The 2PO linker (region B) is 5' to the sequence region A, and comprises of two DNA nucleosides linked by phosphodiester linkage, with the internucleoside linkage between the 3' DNA nucleoside of region A and the 5' LNA nucleoside of region A also being phosphodiester. A linkage group (Y) may be used to link the conjugate group, when present, to region B, or A (SEQ ID NO 8 and 11).

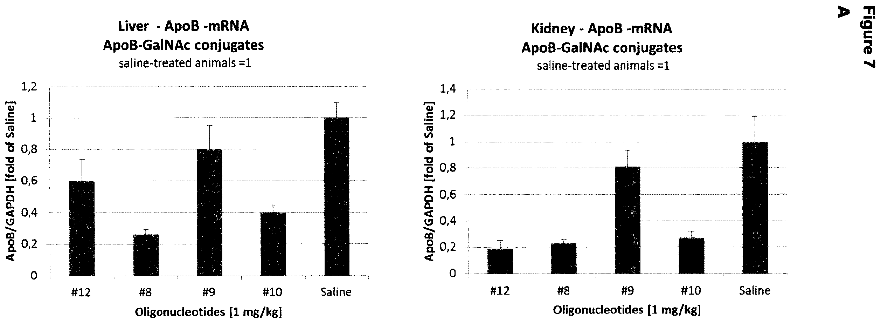

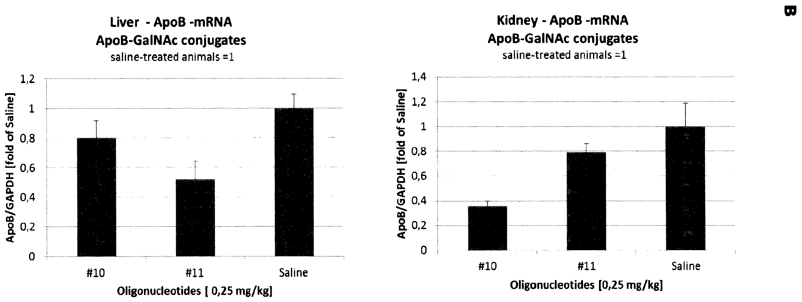

To compare the monoGalNAc conjugates with different linkers and the GalNAc cluster C57BL6 mice were treated i.v. with saline control or with a single dose of 1 mg/kg of ASO conjugated to Mono-GalNAc either without biocleavable linker, with Dithio-linker (SS) or with DNA/PO-linker (PO) (Fig.7a) or with 0,25mg/kg of ASO conjugated to Mono-GalNAc or GalNAc cluster (Fig. 7b). After 7 days the animals were sacrificed and RNA was isolated from liver and kidney samples and analysed for ApoB mRNA expression.

**Conclusions:** Compared to the unconjugated compound (#12) conjugation of mono-GalNAc without a biocleavable linker (#8) or with DNA/PO-linker (#10) shows clearly improved activity in the liver (Fig.7a). Conjugation of different GalNAc conjugates e.g. mono GalNAcPO (#10) and a GalNAc cluster (#11) also allows fine tuning of the compound acitivity with focus on either liver or kidney (Fig.7b).

### Materials and Methods:

### Experimental design:

| **Gr. no.** | **Animal ID no.** | **Animal strain/ gender/ feed** | **Compound Seq ID** | **Dose mg/kg** | **Adm. Route** | **Dosing Day** | **Sacrifice Day** |
|---|---|---|---|---|---|---|---|
| 1 | 1-5 | C57BL6 ♀- Chow | 12 | 1 | i.v. | 0 | 7 |
| 2 | 5-10 | C57BL6 ♀- Chow | 8 | 1 | i.v. | 0 | 7 |
| 3 | 11-15 | C57BL6 ♀- Chow | 9 | 1 | i.v. | 0 | 7 |
| 4 | 16-20 | C57BL6 ♀- Chow | 10 | 1 | i.v.. | 0 | 7 |
| 5 | 21-25 | C57BL6 ♀- Chow | 10 | 0,25 | i.v. | 0 | 7 |
| 6 | 26-30 | C57BL6 ♀- Chow | 11 | 0,25 | i.v. | 0 | 7 |

*Dose administration and sampling.* C57BL6 mice were dosed i.v. with a single dose of 1 mg/kg or 0,25 mg/kg ASO formulated in saline or saline alone according to the above table. Animals were sacrificed at day 7 after dosing and liver and kidney were sampled.

RNA isolation and mRNA analysis.Total RNA was extracted from liver and kidney samples and ApoB mRNA levels were analysed using a branched DNA assay.

### Example 5. Knock down of ApoB mRNA, tissue content, and total cholesterol with GalNAc-conjugates in vivo.

### Compounds

| SEQ ID NO | Seq (5'-3') (A) | Cleavable Linker (B) | Conjugate (C) |
|---|---|---|---|
| 12 | GₛCₛaₛtₛtₛgₛgₛtₛaₛtₛTₛCₛA | no | no |
| 13 | GₛCₛaₛtₛtₛgₛgₛtₛaₛtₛTₛCₛA | | GalNAc cluster Conj1a |
| 11 | GₛCₛaₛtₛtₛgₛgₛtₛaₛtₛTₛCₛA | | GalNAc cluster Conj2a |
| 14 | GₛCₛaₛtₛtₛgₛgₛtₛaₛtₛTₛCₛA | 2PO-DNA (5'ca3') | cholesterol |

Capital letters are LNA nucleosides (such as beta-D-oxy LNA), lower case letters are a DNA nucleoside. Subscript s represents a phosphorothioate internucleoside linkage (region A). LNA cytosines are optionally 5-methyl cytosine. The 2PO linker (region B) is 5' to the sequence region A, and comprises of two DNA nucleosides linked by phosphodiester linkage, with the internucleoside linkage between the 3' DNA nucleoside of region A and the 5' LNA nucleoside of region A also being phosphodiester. A linkage group (Y) may be used to link the conjugate group, when present, to region B, or A (SEQ ID NO 13 and 11). C57BL6/J mice were injected either iv or sc with a single dose saline or 0,25 mg/kg unconjugated LNA-antisense oligonucleotide (SEQ ID NO12) or equimolar amounts of LNA antisense oligonucleotides conjugated to GalNAc1, GalNAc2, or cholesterol(2PO) and sacrificed at days 1-7 according to the table below (experimental design).

RNA was isolated from liver and kidney and subjected to qPCR with ApoB specific primers and probe to analyse for ApoB mRNA knockdown. The oligonucleotide content was measured using ELISA method and total cholesterol in serum was measured.

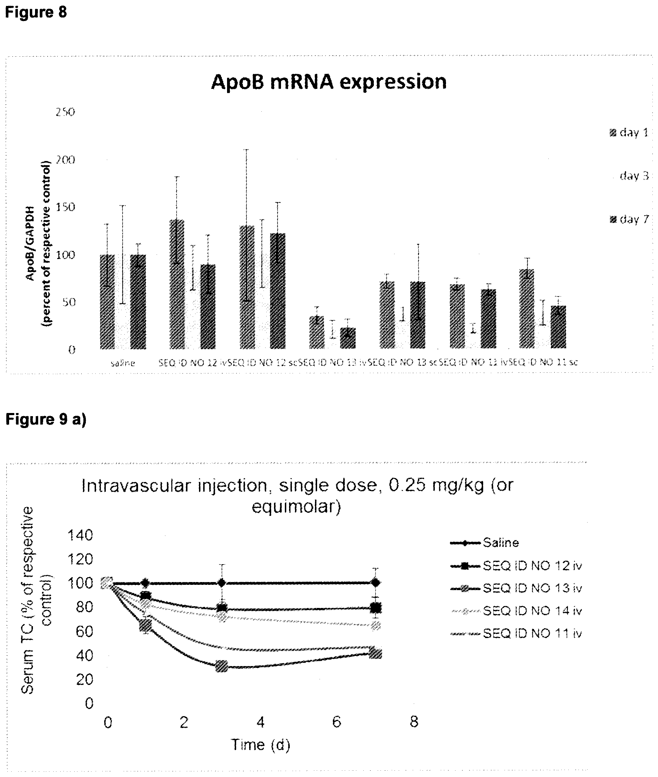

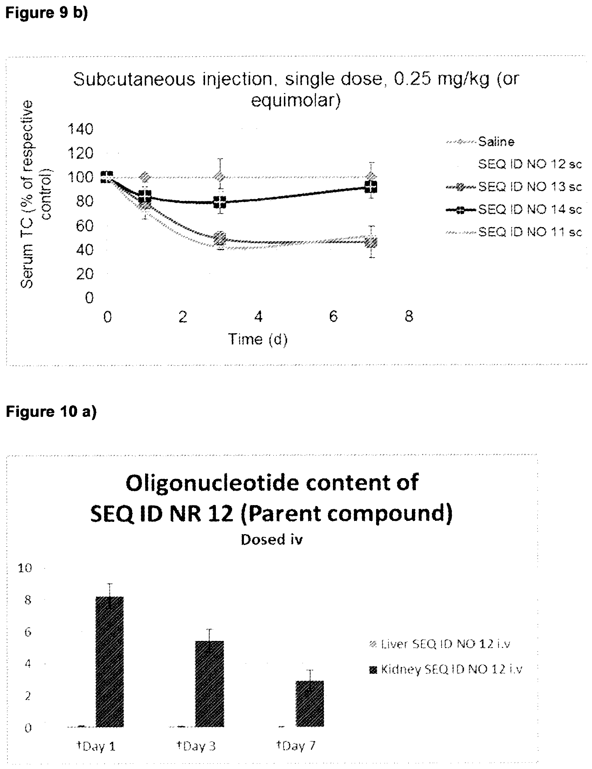

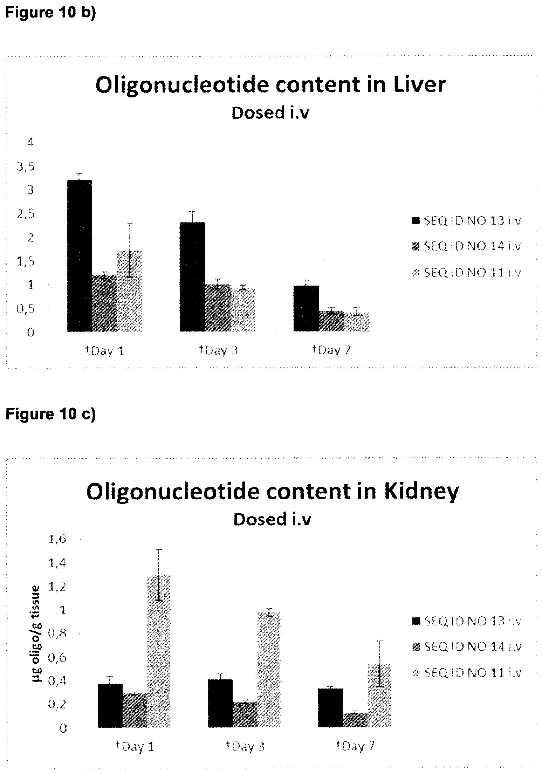

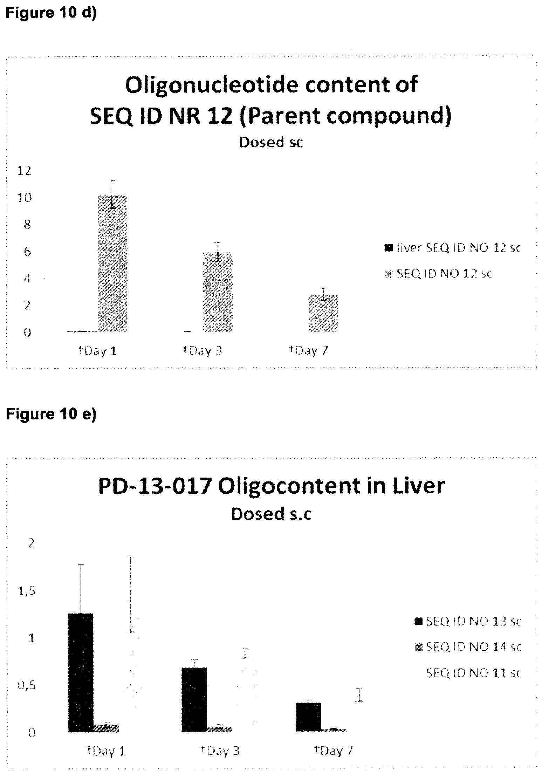

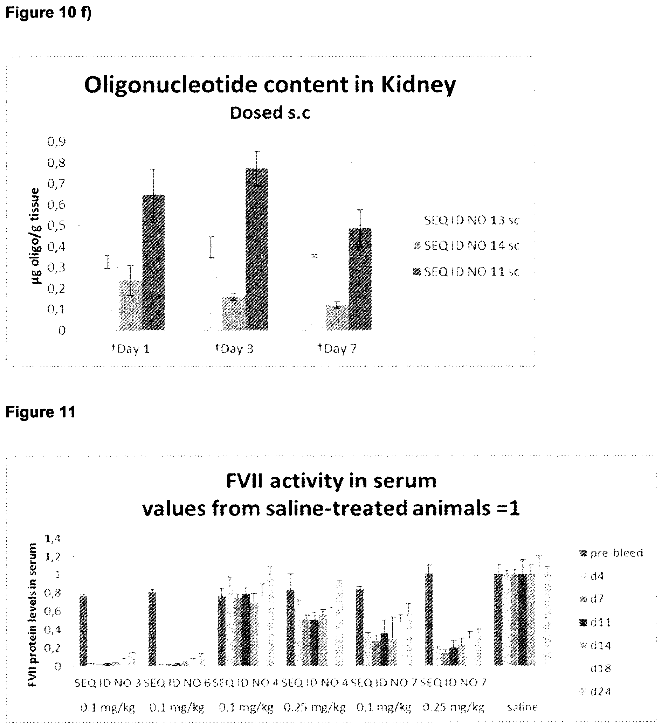

**Conclusions:** GalNAc1 and GalNAc2 conjugated to an ApoB LNA antisense oligonucleotide (SEQ ID NO 11 and 13) showed knock down of ApoB mRNA better than the unconjugated ApoB LNA (Fig. 8). For GalNAc 1 conjugate (SEQ ID NO 13) is seems that iv dosing is better than sc dosing which is surprising since the opposite has been reported for another GalNAc clusters (Alnylam, 9th Annual Meeting of the Oligonucleotide Therapeutics Society) . The total cholesterol data show how the GalNAc cluster conjugates (SEQ ID NO 11 and 13) gives better effect that the unconjugated and the cholesterol conjugated compounds (SEQ ID NO 12 and 14) both at iv and sc administration (Fig 9, a and b). The tissue content of the oligonucleotides (Fig 10, a-f) shows how the conjugates enhances the uptake in liver while giving less uptake in kidney compared to the parent compound. This holds for both iv and sc administration. When dosing iv the GalNAc 1 (SEQ ID NO 13) gives very much uptake in liver when compared to GalNAc 2 (SEQ ID NO 11) but since activity is good for both compounds the GalNAc 2 conjugate appears to induce a higher specific activity than GalNAc 1 conjugate indicating that GalNAc conjugates without the pharmacokinetic modulator may be particularly useful with LNA antisense oligonucleotides.

### Materials and Methods:

### Experimental design:

| **Gr'p no.** | **Animal id no.** | **No. of Animals** | **Animal strain/ gender/feed** | **Compound Dose level per day** | **Conc. at dose vol. 10 ml/kg** | **Adm. Route** | **Dose day** | **Sacr ifice day** |
|---|---|---|---|---|---|---|---|---|
| 1 | 1-3 | 3 | C57BL/6J/♀/Chow | Saline | - | i.v | 0 | 1 |
| 2 | 4-6 | 3 | C57BL/6J/♀/Chow | SEQ ID NO 12 0,25mg/kg | 0,025 mg/ml | i.v | 0 | 1 |
| 3 | 7-9 | 3 | C57BL/6J/♀/Chow | SEQ ID NO 12 0,25mg/kg | 0,025 mg/ml | s.c | 0 | 1 |
| 4 | 10-12 | 3 | C57BL/6J/♀/Chow | SEQ ID NO 13 0,36mg/kg | 0,036 mg/ml | i.v | 0 | 1 |
| 5 | 13-15 | 3 | C57BL/6J/♀/Chow | SEQ ID NO 13 0,36mg/kg | 0,036 mg/ml | s.c | 0 | 1 |
| 6 | 16-18 | 3 | C57BL/6J/♀/Chow | SEQ ID NO 14 0,32mg/kg | 0,032 mg/ml | i.v | 0 | 1 |
| 7 | 19-21 | 3 | C57BL/6J/♀/Chow | SEQ ID NO 14 0,32mg/kg | 0,032 mg/ml | s.c | 0 | 1 |
| 8 | 22-24 | 3 | C57BL/6J/♀/Chow | SEQ ID NO 11 0,34mg/kg | 0,034 mg/ml | i.v | 0 | 1 |
| 9 | 25-27 | 3 | C57BL/6J/♀/Chow | SEQ ID NO 11 0,34mg/kg | 0,034 mg/ml | s.c | 0 | 1 |
| 10 | 28-30 | 3 | C57BL/6J/♀/Chow | Saline | - | i.v | 0 | 3 |
| 11 | 31-33 | 3 | C57BL/6J/♀/Chow | SEQ ID NO 12 0,25mg/kg | 0,025 mg/ml | i.v | 0 | 3 |
| 12 | 34-36 | 3 | C57BL/6J/♀/Chow | SEQ ID NO 12 0,25mg/kg | 0,025 mg/ml | s.c | 0 | 3 |
| 13 | 37-39 | 3 | C57BL/6J/♀/Chow | SEQ ID NO 13 0,36mg/kg | 0,036 mg/ml | i.v | 0 | 3 |
| 14 | 40-42 | 3 | C57BL/6J/♀/Chow | SEQ ID NO 13 0,36mg/kg | 0,036 mg/ml | s.c | 0 | 3 |
| 15 | 43-45 | 3 | C57BL/6J/♀/Chow | SEQ ID NO 14 0,32mg/kg | 0,032 mg/ml | i.v | 0 | 3 |
| 16 | 46-48 | 3 | C57BL/6J/♀/Chow | SEQ ID NO 14 0,32mg/kg | 0,032 mg/ml | s.c | 0 | 3 |
| 17 | 49-51 | 3 | C57BL/6J/♀/Chow | SEQ ID NO 11 0,34mg/kg | 0,034 mg/ml | i.v | 0 | 3 |
| 18 | 52-54 | 3 | C57BL/6J/♀/Chow | SEQ ID NO 11 0,34mg/kg | 0,034 mg/ml | s.c | 0 | 3 |
| 19 | 55-57 | 3 | C57BL/6J/♀/Chow | Saline | - | i.v | 0 | 7 |
| 20 | 58-60 | 3 | C57BL/6J/♀/Chow | SEQ ID NO 12 0,25mg/kg | 0,025 mg/ml | i.v | 0 | 7 |
| 21 | 61-63 | 3 | C57BL/6J/♀/Chow | SEQ ID NO 12 0,25mg/kg | 0,025 mg/ml | s.c | 0 | 7 |
| 22 | 64-66 | 3 | C57BL/6J/♀/Chow | SEQ ID NO 13 0,36mg/kg | 0,036 mg/ml | i.v | 0 | 7 |
| 23 | 67-69 | 3 | C57BL/6J/♀/Chow | SEQ ID NO 13 0,36mg/kg | 0,036 mg/ml | s.c | 0 | 7 |
| 24 | 70-72 | 3 | C57BL/6J/♀/Chow | SEQ ID NO 14 0,32mg/kg | 0,032 mg/ml | i.v | 0 | 7 |
| 25 | 73-75 | 3 | C57BL/6J/♀/Chow | SEQ ID NO 14 0,32mg/kg | 0,032 mg/ml | s.c | 0 | 7 |
| 26 | 76-78 | 3 | C57BL/6J/♀/Chow | SEQ ID NO 10 0,34mg/kg | 0,034 mg/ml | i.v | 0 | 7 |
| 27 | 79-81 | 3 | C57BL/6J/♀/Chow | SEQ ID NO 11 0,34mg/kg | 0,034 mg/ml | s.c | 0 | 7 |

*Dose administration.* C57BL/6JBom female animals, app. 20 g at arrival, were dosed with 10 ml per kg BW (according to day 0 bodyweight) *i.v.* or s.c. of the compound formulated in saline or saline alone according to the table above.

*Sampling of liver and kidney tissue.* The animals were anaesthetised with 70% CO₂-30% O₂ and sacrificed by cervical dislocation according to the above table. One half of the large liver lobe and one kidney were minced and submerged in RNAlater. The other half of liver and the other kidney was frozen and used for tissue analysis.

Total RNA Isolation and First strand synthesis. Total RNA was extracted from maximum 30 mg of tissue homogenized by bead-milling in the presence of RLT-Lysis buffer using the Qiagen RNeasy kit (Qiagen cat. no. 74106) according to the manufacturer's instructions. First strand synthesis was performed using Reverse Transcriptase reagents from Ambion according to the manufacturer's instructions.

For each sample 0.5 µg total RNA was adjusted to (10.8 µl) with RNase free H₂O and mixed with 2 µl random decamers (50 µM) and 4 µl dNTP mix (2.5 mM each dNTP) and heated to 70 °C for 3 min after which the samples were rapidly cooled on ice. 2 µl 10x Buffer RT, 1 µl MMLV Reverse Transcriptase (100 U/µl) and 0.25 µl RNase inhibitor (10 U/µl) were added to each sample, followed by incubation at 42 °C for 60 min, heat inactivation of the enzyme at 95°C for 10 min and then the sample was cooled to 4 °C. cDNA samples were diluted 1: 5 and subjected to RT-QPCR using Taqman Fast Universal PCR Master Mix 2x (Applied Biosystems Cat #4364103) and Taqman gene expression assay (mApoB, Mn01545150_m1 and mGAPDH #4352339E) following the manufacturers protocol and processed in an Applied Biosystems RT-qPCR instrument (7500/7900 or ViiA7) in fast mode. Oligonucleotide content in liver and kidney was measured by sandwich ELISA method.

Serum cholesterol analysis: Immediately before sacrifice retro-orbital sinus blood was collected using S-monovette Serum-Gel vials (Sarstedt, Nümbrecht, Germany) for serum preparation. Serum was analyzed for total cholesterol using ABX Pentra Cholesterol CP (Triolab, Brondby, Denmark) according to the manufacturer's instructions.

### Example 6: In vivo inhibition of FVII (low doses, 24 days)

### Compounds used - see Example 1.

An *in vivo* mouse study was prepared using a total of 7 groups of mice (n=3). Each mouse was administered a single *i.v.* dose of LNA compound, at either 0,25mg/kg or 0,1mg/kg. A saline control group was included. The mice were pre-bled 1 day before administration, and subsequent bleeds were taken at days 4, 7, 11, 14, 18, and 24 during the study. The mice were sacrificed at days 24 and liver, kidney, and blood samples taken. See the table below (experimental design) table for study setup. FactorVII serum levels and mRNA levels were measured using standard assay techniques.

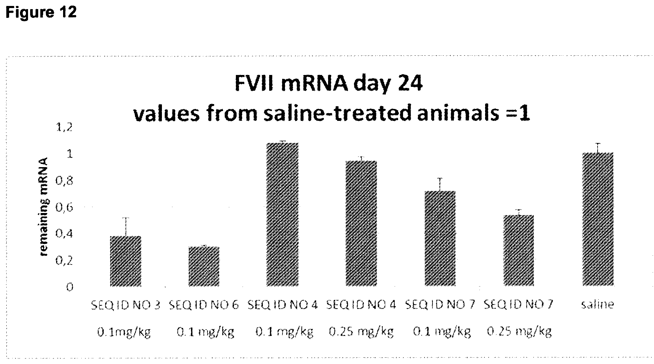

Conclusions: GalNAc conjugated to a FVII targeting LNA oligonucleotide (SEQ ID NO 3 and 6) show very good activity on FVII protein in serum during the 24 days (Figure 11) and mRNA at day 24 in liver (Figure 12) after single dose administration of only 0,1mg/kg. It is seen that the GalNAc cluster (SEQ ID NO 3 and 6) is more active for FVII protein and mRNA compared to the cholesterol conjugate (SEQ ID NO 4 and 7).

Materials and Methods: Male mice were administered iv and liver, kidney, and blood were sampled at sacrifice all according to the following scheme.

### Experimental design:

| compound | termination time point post dose | group size | dose (d0) mg/kg |
|---|---|---|---|
| Saline | d24 | 3 | none |
| SEQ ID NO 3 | d24 | 3 | 0,1 |
| SEQ ID NO 6 | d24 | 3 | 0,1 |
| SEQ ID NO 4 | d24 | 3 | 0,1 |
| SEQ ID NO 4 | d24 | 3 | 0,25 |
| SEQ ID NO 7 | d24 | 3 | 0,1 |
| SEQ ID NO 7 | d24 | 3 | 0,25 |

### Example 7: Non-Human Primate Study - GalNac conjugate study - PCSK9 and ApoB

### Compounds:

| SEQ ID NO | Seq (5'-3') (A) | Cleavable Linker (B) | Conjugate (C) |
|---|---|---|---|
| 11 | GₛCₛaₛtₛtₛgₛgₛtₛaₛtₛTₛCₛA | | GalNAc cluster Conj2a |
| 17 | GₛTₛtₛgₛaₛcₛaₛcₛtₛgₛTₛC | | GalNAc cluster Conj2a |
| 15 | AₛAₛTₛgₛcₛtₛaₛcₛaₛaₛaₛaₛcₛCₛCₛA | | GalNAc cluster Conj2a |
| 16 | TₛGₛcₛtₛgₛtₛgₛtₛgₛaₛgₛcₛtₛTₛGₛG | | GalNAc cluster Conj2a |
| 28 | AₛAₛTₛgₛcₛtₛaₛcₛaₛaₛaₛaₛCₛCₛCₛA | | GalNAc cluster Conj2a |
| 29 | GₛCₛtₛgₛtₛgₛtₛgₛaₛgₛcₛtₛtₛGₛG | | GalNAc cluster Conj2a |
| 30 | AₛAₛTₛgₛcₛtₛaₛcₛaₛaₛaₛaₛCₛCₛCₛA | 2PO-DNA (5'ca3') | cholesterol |
| 31 | TₛGₛcₛtₛgₛtₛgₛtₛgₛaₛgₛcₛtₛTₛGₛG | 2PO-DNA (5'ca3') | cholesterol |
| 14 | GₛCₛaₛtₛtₛgₛgₛtₛaₛtₛTₛCₛA | 2PO-DNA (5'ca3') | cholesterol |
| 32 | GₛTₛtₛgₛaₛcₛaₛcₛtₛgₛTₛC | 2PO-DNA (5'ca3') | cholesterol |

Capital letters are LNA nucleosides (such as beta-D-oxy LNA), lower case letters are DNA nucleosidez. Subscript s represents a phosphorothioate internucleoside linkages. LNA cytosines are optionally 5-methyl cytosine.

The primary objective for this study is to investigate selected lipid markers over 7 weeks after a single slow bolus injection of anti-PCSK9 and anti-ApoB LNA conjugated to GalNac cluster (Conj2a) to cynomolgus monkeys and assess the potential toxicity of compounds in monkey. The compounds which were prepared in sterile saline (0.9%) at an initial concentration of 0.625 and 2.5 mg/ml).

Male (PCSK9) or female monkeys (ApoB) monkeys of at least 24 months old are used, and given free access to tap water and 180g of MWM(E) SQC SHORT expanded diet (Dietex France, SDS, Saint Gratien, France) will be distributed daily per animal. The total quantity of food distributed in each cage will be calculated according to the number of animals in the cage on that day. In addition, fruit or vegetables will be given daily to each animal. The animals will be acclimated to the study conditions for a period of at least 14 days before the beginning of the treatment period. During this period, pre-treatment investigations will be performed. The animals are dosed i.v. at a dose if, for example, 0.25 mg/kg or 1 mg/kg. The dose volume will be 0.4 mL/kg. 2 animals are used per group. After three weeks, the data will be analyzed and a second group of animals using a higher or lower dosing regimen may be initiated - preliminary dose setting is 0.5 mg/kg and 1 mg/kg, or lower than that based on the first data set.

The dose formulations will be administered once on Day 1. Animals will be observed for a period of 7 weeks following treatment, and will be released from the study on Day 51. Day 1 corresponds to the first day of the treatment period. Clinical observations and body weight and food intake (per group) will be recorded prior to and during the study.

Blood is sampled and analysis at the following time points:

| Study Day | Parameters |
|---|---|
| -8 | RCP, L, Apo-B, PCSK9*, OA |
| -1 | L, Apo-B, PCSK9*, PK, OA |
| 1 | Dosing |
| 4 | LSB, L, Apo-B, PCSK9*, OA |
| 8 | LSB, L, Apo-B, PCSK9*, PK, OA |
| 15 | RCP, L, Apo-B, PCSK9*, PK, OA |
| 22 | LSB, L, Apo-B, PCSK9*, PK, OA |
| 29 | L, Apo-B, PCSK9*, PK, OA |
| 36 | LSB, L, Apo-B, PCSK9*, PK, OA |
| 43 | L, PK, Apo-B, PCSK9*, PK, OA |
| 50 | RCP, L, Apo-B, PCSK9*, PK, OA |

| | |
|---|---|
| RCP 0 routine clinical pathology, LSB = liver safety biochemistry, PK = pharmacokinetics, OA = other analysis, L = Lipids. | |

### Blood biochemistry

The following parameters will be determined for all surviving animals at the occasions indicated below:
- full biochemistry panel (complete list below) - on Days -8, 15 and 50,
- liver Safety (ASAT, ALP, ALAT, TBIL and GGT only) - on Days 4, 8, 22 and 36,
- lipid profile (Total cholesterol, HDL-C, LDL-C and Triglycerides) and Apo-B only - on Days -1, 4, 8, 22, 29, 36, and 43.

Blood (approximately 1.0 mL) is taken into lithium heparin tubes (using the ADVIA 1650 blood biochemistry analyzer): Apo-B, sodium, potassium, chloride, calcium, inorganic phosphorus, glucose, HDL-C, LDL-C, urea, creatinine, total bilirubin (TBIL), total cholesterol, triglycerides, alkaline phosphatase (ALP), alanine aminotransferase (ALAT), aspartate aminotransferase (ASAT),creatine kinase, gamma-glutamyl transferase (GGT), lactate dehydrogenase, total protein, albumin, albumin/globulin ratio.

Analysis of blood: Blood samples will be collected from Group 16 animals only on Days -8, - 1, 4, 8, 15, 22, 29, 36, 43 and 50.

Venous blood (approximately 2 mL) will be collected from an appropriate vein in each animal into a Serum Separating Tube (SST) and allowed to clot for at least 60 ± 30 minutes at room temperature. Blood will be centrifuged at 1000 g for 10 minutes under refrigerated conditions (set to maintain +4°C). The serum will be transferred into 3 individual tubes and stored at -80°C until analyzed at CitoxLAB France using an ELISA method (Circulex Human PCSK9 ELISA kit, CY-8079, validated for samples from cynomolgus monkey).

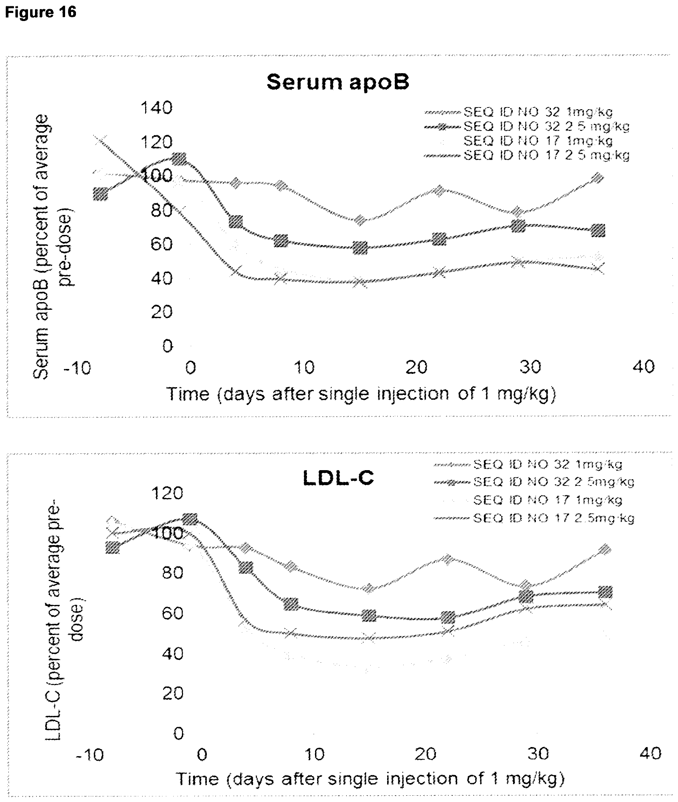

Other Analysis: WO2011009697 & WO2010142805 provides the methods for the following analysis: qPCR, PCSK9 / ApoB mRNA analysis, Other analysis includes PCSK9 / ApoB protein ELISA, serum Lp(a) analysis with ELISA (Mercodia No. 10-1106-01), tissue and plasma oligonucleotide analysis (drug content), Extraction of samples, standard - and QC-samples, Oligonucleotide content determination by ELISA. The data for the the ApoB conjugates are shown in Figure 16. Compounds 11 and 14, which retain the same oligonucleotide sequence did not exhibit significant pharmacology at the dose used, where as compounds SEQ ID NO 17 and 32, which also share the same oligo sequence were effective, with the GalNac conjugated compound (SEQ ID NO 17) being considerably more potent than the cholesterol conjugated compound. There was no indication of hepatotoxicity or nephrotoxicity with the ApoB targeting compounds.

The data for the PCSK9 tageting compounds is shown in the following table:

| Values for 2.5 mg/kg dose | | | Max PCSK9 reduction* | Max LDL-C reduction* |
|---|---|---|---|---|
| Compound **SEQ ID** | PCSK9 protein day 4 (percent of pre-dose) | PCSK9 protein day 29 (percent of pre-dose) | | |
| **30** | 86 | 71.5 | 69% (d15) | 87% (d29) |
| **31** | 81 | 71 | 71% (d29) | 84% (d22) |
| **28** | 57 | 42 | 42% (d29) | 71% (d15) |
| **16** | 80.5 | 56 | 55% (d29) | 84% (d15) |
| **29** | 51 | 53 | 48% (d4) | 94% (D8) |
| **25** | 55 | 60 | 55% (d4) | 89% (D4) |

| | | | | |
|---|---|---|---|---|
| * As compared to pre-dose baseline levels | | | | |

There was no indication of hepatotoxicity or nephrotoxicity with the PCSK9 targeting compounds. Notably, the PCSK9-GalNac compounds gave a rapid and highly effective down regulation of PCSK9 which was maintained over an extensive time period (entire length of the study), illustratating the GalNac conjugated compounds are more effective, both in terms of a rapid initial knock-down, and long duration, indicating that they may be dosed comparatively infrequently and at a lower dosage, as compared to both the unconjugated parent compounds, and compounds using alternative conjugation technology, such as cholesterol conjugation. A similar result was seen for one of the ApoB targeted compounds (see Figure 16) where the GaINac ompound was also found to be markedly more potent than the cholesterol conjugated compound, and gave a longer duration of action. SEQ ID NO 28 gave rapid and consistent down regulation of PCSK9 and LDL-C throughout the duration of the study (seen at day 34 at 2.5mg/kg dose, with notable PCSK9 down-regulation seen 48 days after the administratin of the single 2.5mg/kgdose.

### Example 8: Liver and Kidney toxicity Assessment in Rat.

Compounds of the invention can be evaluated for their toxicity profile in rodents, such as in mice or rats. By way of example the following protocol may be used: Wistar Han Crl:WI(Han) are used at an age of approximately 8 weeks old. At this age, the males should weigh approximately 250 g. All animals have free access to SSNIFF R/M-H pelleted maintenance diet (SSNIFF Spezialdiäten GmbH, Soest, Germany) and to tap water (filtered with a 0.22 µm filter) contained in bottles.The dose level of 10 and 40mg/kg/dose is used (subcutaneous administration) and dosed on days 1 and 8. The animals are euthanized on Day 15. Urine and blood samples are collected on day 7 and 14. A clinical pathology assessment is made on day 14. Body weight is determined prior to the study, on the first day of administration, and 1 week prior to necropsy. Food consumption per group will be assessed daily. Blood samples are taken via the tail vein after 6 hours of fasting. The following blood serum analysis is performed: erythrocyte count mean cell volume packed cell volume hemoglobin mean cell hemoglobin concentration mean cell hemoglobin thrombocyte count leucocyte count differential white cell count with cell morphology reticulocyte count, sodium potassium chloride calcium inorganic phosphorus glucose urea creatinine total bilirubin total cholesterol triglycerides alkaline phosphatase alanine aminotransferase aspartate aminotransferase total protein albumin albumin/globulin ratio. Urinalysis are performed α-GST, β-2 Microglobulin, Calbindin, Clusterin, Cystatin C, KIM-1, Osteopontin, TIMP-1, VEGF,and NGAL. Seven analytes (Calbindin, Clusterin, GST-α, KIM-1, Osteopontin, TIMP-1, VEGF) will be quantified under Panel 1 (MILLIPLEX® MAP Rat Kidney Toxicity Magnetic Bead Panel 1, RKTX1MAG-37K). Three analytes (β-2 Microglobulin, Cystatin C, Lipocalin-2/NGAL) will be quantified under Panel 2 (MILLIPLEX® MAP Rat Kidney Toxicity Magnetic Bead Panel 2, RKTX2MAG-37K). The assay for the determination of these biomarkers' concentration in rat urines is based on the Luminex xMAP® technology. Microspheres coated with anti- α-GST / β-2 microglobulin / calbindin / clusterin / cystacin C / KIM-1 / osteopontin / TIMP-1 / VEGF / NGAL antibodies are color-coded with two different fluorescent dyes.The following parameters are determined (Urine using the ADVIA 1650): Urine protein, urine creatinine. Quantitative parameters: volume, pH (using 10-Multistix SG test strips/Clinitek 500 urine analyzer), specific gravity (using a refractometer). Semiquantitative parameters (using 10-Multistix SG test strips/Clinitek 500 urine analyzer): proteins, glucose, ketones, bilirubin, nitrites, blood, urobilinogen, cytology of sediment (by microscopic examination).Qualitative parameters: Appearance, color. After sacrifice, the body weight and kidney, liver and spleen weight are determined and organ to body weight ratio calculated. Kidney and liver samples will be taken and either frozen or stored in formalin. Microscopic analysis is performed.

### Example 9 ApoB Targeting Compounds with FAM label conjugates

| # | Seq (5'-3') | Cleavable linker (B) | Conjugate (C) |
|---|---|---|---|
| 21 | GCattggtatTCA | 3PO-DNA (5'tca3') | FAM |
| 62 | GCattggtatTCA | 2PO-DNA (5'ca3') | FAM |
| 18 | GCattggtatTCA | 1 PO-DNA (5'a3') | FAM |
| 19 | GCattggtatTCA | 3PO-DNA (5'gac3') | FAM |
| 20 | GCattggtatTCA | no | FAM |

Capital letters are LNA nucleosides (such as beta-D-oxy LNA), lower case letters are DNA nucleosidez. Subscript s represents a phosphorothioate internucleoside linkages. LNA cytosines are optionally 5-methyl cytosine.

FAM-labeled ASOs with different DNA/PO-linkers were subjected to *in vitro* cleavage either in S1 nuclease extract - see table below. Liver or kidney homogenates or Serum (Table below).

FAM-labeled ASOs 100 µM with different DNA/PO-linkers were subjected to *in vitro* cleavage by S1 nuclease in nuclease buffer (60 U pr. 100 µL) for 20 and 120 minutes. The enzymatic activity was stopped by adding EDTA to the buffer solution. The solutions were then subjected to AIE HPLC analyses on a Dionex Ultimate 3000 using an Dionex DNApac p-100 column and a gradient ranging from 10mM - 1 M sodium perchlorate at pH 7.5. The content of cleaved and non cleaved oligonucleotide were determinded against a standard using both a fluoresense detector at 615 nm and a uv detector at 260 nm.

| SEQ ID NO | Linker sequence | % cleaved after 20min S1 | % cleaved after 120min S1 |
|---|---|---|---|
| 20 | -- | 2 | 5 |
| 18 | a | 29.1 | 100 |
| 62 | ca | 40.8 | 100 |
| 21 | tca | 74.2 | 100 |
| 19 | gac | 22.9 | n.d |

**Conclusion:** The PO linkers (or region B as referred to herein) results in the conjugate (or group C) being cleaved off, and both the length and/or the sequence composition of the linker can be used tomodulate susceptibility to nucleolytic cleavage of region B. The Sequence of DNA/PO-linkers can modulate the cleavage rate as seen after 20 min in Nuclease S1 extract (Table above). Sequence selection for region B (e.g.for the DNA/PO-linker) & can therefore also be used to modulate the level of cleavage in serum and in cells of target tissues.

Liver, kidney and serum (see table below) were spiked with oligonucleotide SEQ ID NO 21 to concentrations of 200 µg/g tissue. Liver and kidney samples collected from NMRI mice were homogenized in a homogenisation buffer (0,5% Igepal CA-630, 25 mM Tris pH 8.0, 100 mM NaCl, pH 8.0 (adjusted with 1 N NaOH). The homogenates were incubated for 24 hours at 37° and thereafter the homogenates were extracted with phenol - chloroform. The content of cleaved and non cleaved oligonucleotide in the extract from liver and kidney and from the serum were determinded against a standard using the above HPLC method.

| Seq ID | Linker Sequence | % cleaved after 24hrs liver homogenate | % cleaved after 24hrs kidney homogenate | % cleaved after 24hours in serum |
|---|---|---|---|---|
| 21 | tca | 83 | 95 | 0 |

**Conclusion:** The PO linkers (or region B as referred to herein) results in cleavage of the conjugate (or group C) from the oligonucleotide, in liver or kidney homogenate, but not in serum (Table above). Note: cleavage in the above assays refers to the cleavage of the cleavable linker, the oligomer or region A should remain functionally intact.

The susceptibility to cleavage in the assays shown in Example 9 can be used to determine whether a linker is biocleavable or physiologically labile.

### Example 10 GalNac Conjugates: Rat toxicity Study

### Methodology: See Example 8.

The rat safety study was performed at CiToxLabs, France. Male Wistar rats (n=4/group) were selected for the study as the Wistar Han rats in the used study set-up (dose range and time course) have previously been demonstrated to predict renal (and to some extent hepatic) toxicity in humans. The animals were injected s.c. Day 1 and Day 8 with conjugated LNA compounds (at 10 mg/kg), or corresponding unconjugated "parent compound" (at 40 mg/kg). Urine was collected Day 7 and Day 14 and kept on ice until analysis. Urine samples were centrifuged (approx. 380 g, 5 min, at +4°C) and a panel of urinary injury markers analyzed with a multiplex assay based on the Luminex xMAP® technology.

Out of the panel of urinary kidney injury markers in the study KIM-1 (kidney injury marker 1) demonstrated the largest dynamic range and most clear signal, as has recently been described for KIM-1 in a meta-analysis of urinary kidney injury markers (Vlasakova et al, Evaluation of the Relative Performance of Twelve Urinary Biomarkers for Renal Safety across Twenty Two Rat Sensitivity and Specificity Studies Toxicol. Sci. December 21, 2013). Compounds Used: SEQ ID NO 11 & 17 (target ApoB) and the following PCSK9 targeting compounds SEQ IDs:

| SEQ ID NO | Seq (5'-3') (A) | Conjugate (C) |
|---|---|---|
| 22 | TₛGₛCₛtₛaₛcₛaₛaₛaₛaₛcₛCₛCₛA | None |
| 23 | AₛAₛTₛgₛcₛtₛaₛcₛaₛaₛaₛaₛCₛCₛCₛA | None |
| 24 | AₛAₛTₛgₛcₛtₛaₛcₛaₛaₛaₛaₛcₛCₛCₛA | None |
| 25 | GₛCₛtₛgₛtₛgₛtₛgₛaₛgₛcₛtₛtₛGₛG | None |
| 26 | TₛGₛcₛtₛgₛtₛgₛtₛgₛaᵣgₛcₛtₛTₛGₛG | None |
| 27 | TₛGₛCₛtₛaₛcₛaₛaₛaₛaₛcₛCₛCₛA | GalNAc cluster Conj2a |
| 28 | AₛAₛTₛgₛcₛtₛaₛcₛaₛaₛaₛaₛCₛCₛCₛA | GalNAc cluster Conj2a |
| 15 | AₛAₛTₛgₛcₛtₛaₛcₛaₛaₛaₛaₛCₛCₛCₛA | GalNAc cluster Conj2a |
| 29 | GₛCₛtₛgₛtₛgₛtₛgₛaₛgₛcₛtₛtₛGₛG | GalNAc cluster Conj2a |
| 16 | TₛGₛcₛtₛgₛtₛgₛtₛgₛaₛcₛtₛTₛGₛG | GalNAc cluster Conj2a |

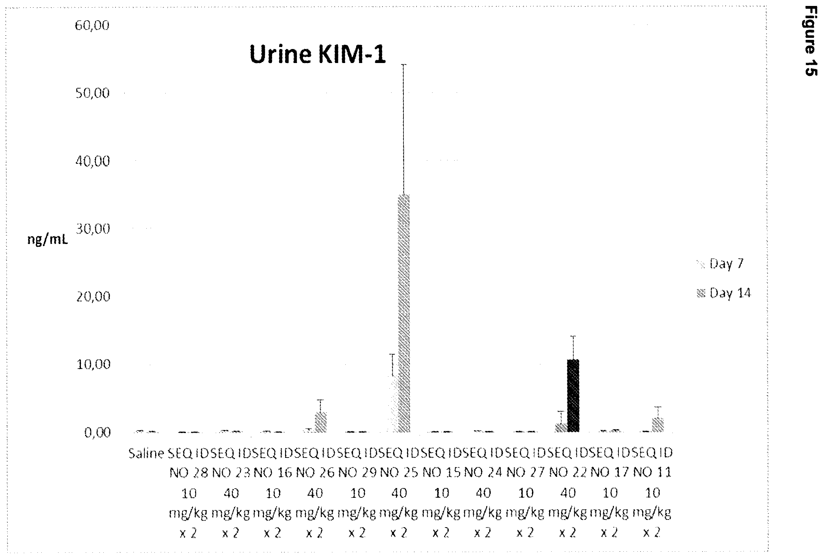

The results are shown in Figure 15, which illustrated that, the use of Galnac conjugates, the nephrotoxicity profile of a parent LNA compound can be profoundly improved. GalNac conjugated LNA compounds are therefore not only considerably more potent than their parent compounds, but also are associated with a marked reduction in the risk of nephrotoxicity.

### Example 11: LNA antimiRs GaINac Conjugates

### Compounds

Capital letters are LNA, such as beta-D-oxy LNA. Lower case letters are DNA. Subscript s is a phosphorothioate linkage. Other internucleoside linkages are phosphodiester (phosphate) linkages. Superscript m before a C represents LNA 5-methyl cytosine (optional). In some embodiments, the compounds may also be made with LNA cytosine. In some embodiments, the Conj1a group may be another GalNAc conjugate group, such as those disclosed herein, for example Conj2a.
miR-122 (Tiny) 5'-^{m}CₛAₛ^{m}CₛAₛ^{m}CₛTₛ^{m}Cₛ^{m}C-3' (SEQ ID NO 51)
GalNAc-tiny 5'-Conj1a^{m}CₛAₛ^{m}CₛAₛ^{m}CₛTₛ^{m}Cₛ^{m}C-3' (SEQ ID NO 52)
tiny-linker-tiny5'-^{m}CₛAₛ^{m}CₛAₛ^{m}CₛTₛ^{m}Cₛ^{m}Cca^{m}CₛAₛ^{m}CₛAₛ^{m}CₛTₛ^{m}Cₛ^{m}C-3' (SEQ ID NO 53)
GalNac-tiny-linker-tiny 5'-Conj1a^{m}CₛAₛ^{m}CₛAₛ^{m}CₛTₛ^{m}Cₛ^{m}Cca^{m}CₛAₛ^{m}CₛAₛ^{m}CₛTₛ^{m}Cₛ^{m}C-3' (SEQ ID NO 54)

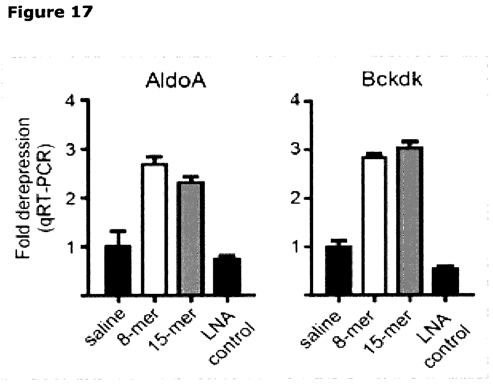

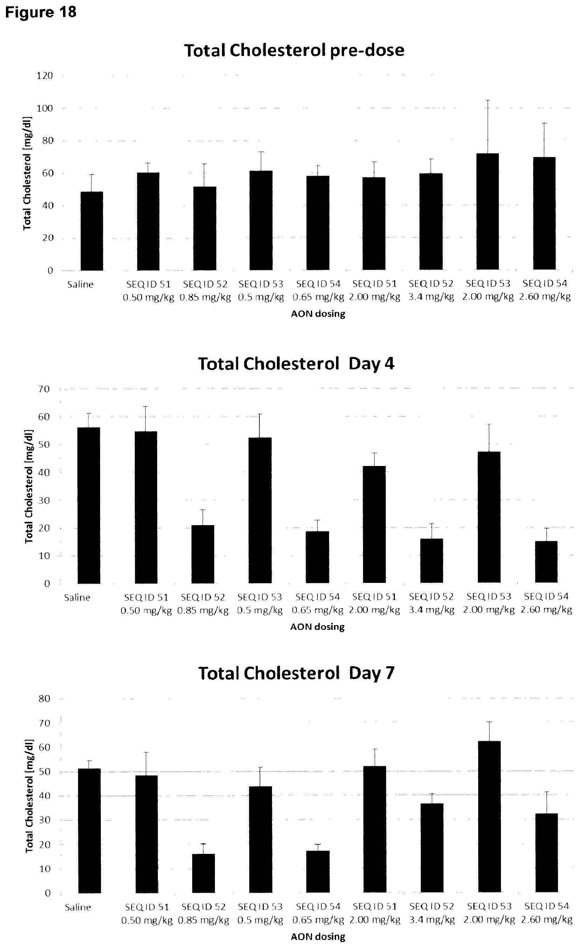

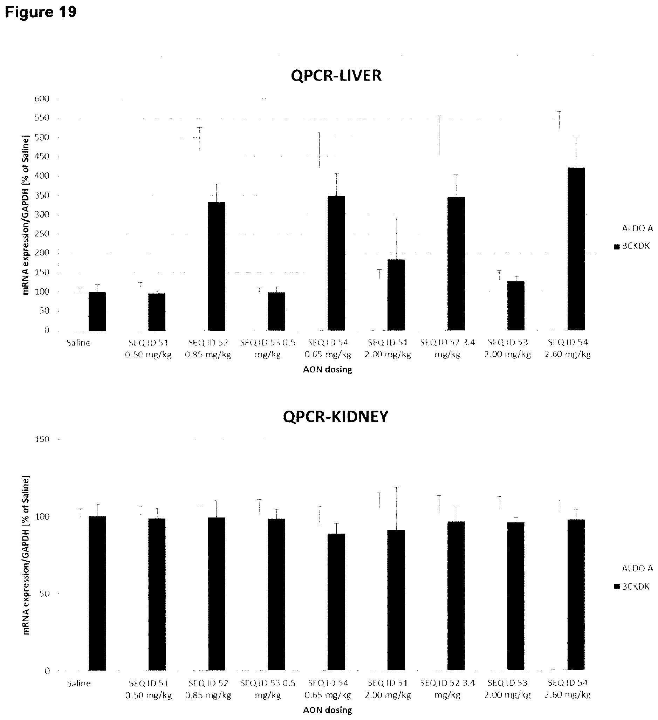

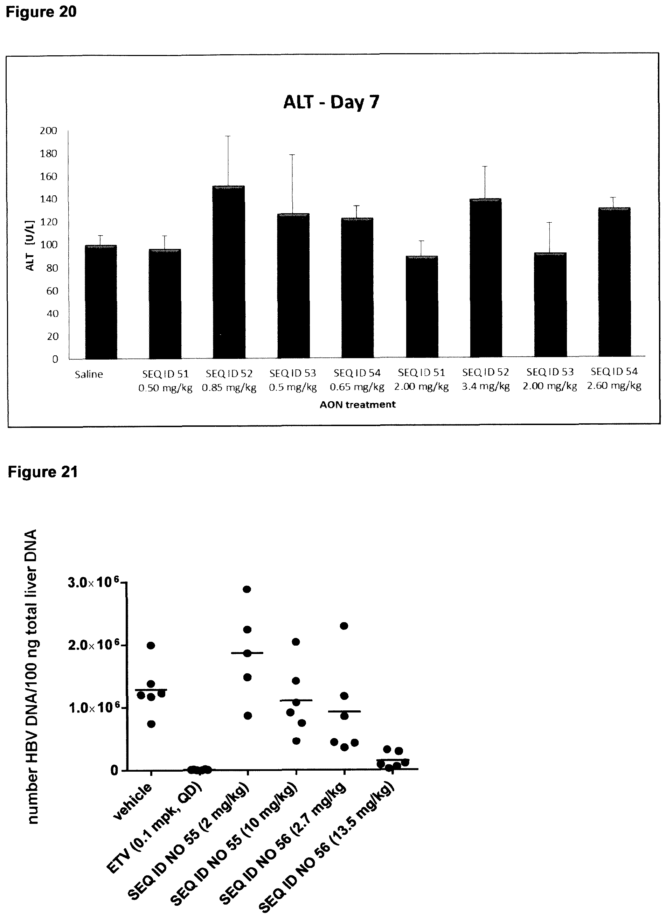

An *in vivo* mouse study was performed using a total of 9 groups of mice (n=5). Each mouse was dosed *i.v.* on days 0, 2 and 4 with either 0,5 mg/kg or 2 mg/kg or equimolar doses of the GalNAc conjugated LNA's compared to parent LNA compound. A saline control group was included (see study set up in Tab. 1). Serum samples were taken 4 days pre administration, interim at day 4 and at endpoint day 7. Liver and kidney samples were stored in RNA later. Validation of miR122 knock down of miR122 was done as described in Obad Nat Genet. 2011 Mar 20;43(4):371-8 (Fig.17). The cholesterol level in serum were analyzed as described in Elmen J, et al. LNA-mediated microRNA silencing in non-human primates. Nature. 2008;452:896-899. (Fig. 18) and mRNA levels of two miR122 down regulated genes (Aldo A and Bckdk) were analyzed using standard QPCR assay techniques (Fig.19). ALT was measured to assess tolerability of the compounds (Fig.20).

### Study Set-up

| group | compound | termination time point post dose | group size | dose (d0, d2, d4) mg/kg |
|---|---|---|---|---|
| | Saline | D7 | 5 | none |
| | SEQ ID 51 | D7 | 5 | 3x0,5 |
| | SEQ ID 52 | D7 | 5 | 3x0,85 |
| | SEQ ID 53 | D7 | 5 | 3x0,5 |
| | SEQ ID 54 | D7 | 5 | 3x0,65 |
| | SEQ ID 51 | D7 | 5 | 3x2 |
| | SEQ ID 52 | D7 | 5 | 3x3,4 |
| | SEQ ID 53 | D7 | 5 | 3x2 |
| | SEQ ID 54 | D7 | 5 | 3x2,6 |

Conclusions: Conjugation of GalNAc to anti-miR122 (SEQ ID 52 and 54) showed a remarkable improvement of miR122 knock down in the liver indicated by decreased total cholesterol levels (Fig.2) and up regulation of Aldo A and Bckdk mRNA already at in the low dose group (Fig.3, 3x 0,5 mg/kg). No effect of the anti- miR122 oligonucleotide was seen in the kidney. An increase in ALT was measured for SEQID 52 which showed a tendency to improve by conjugation of 2 oligonucleotides to one GalNAc (SEQID54).

### Example 12: GalNac Conjugates for HBV targeting oligonucleotides

Oligonucleotides were synthesized on uridine universal supports using the phosphoramidite approach on an Expedite 8900/MOSS synthesizer (Multiple Oligonucleotide Synthesis System) at 4 µmol scale. At the end of the synthesis, the oligonucleotides were cleaved from the solid support using aqueous ammonia for 1-2 hours at room temperature, and further deprotected for 16 hours at 65°C. The oligonucleotides were purified by reverse phase HPLC (RP-HPLC) and characterized by UPLC, and the molecular mass was further confirmed by ESI-MS. See below for more details.

### Elongation of the oligonucleotide

The coupling of β-cyanoethyl- phosphoramidites (DNA-A(Bz), DNA- G(ibu), DNA- C(Bz), DNA-T, LNA-5-methyl-C(Bz), LNA-A(Bz), LNA- G(dmf), LNA-T or C6-S-S linker) was performed by using a solution of 0.1 M of the 5'-O-DMT-protected amidite in acetonitrile and DCI (4,5-dicyanoimidazole) in acetonitrile (0.25 M) as activator. For the final cycle a commercially available C6-linked cholesterol phosphoramidite was used at 0.1M in DCM. Thiolation for introduction of phosphorthioate linkages is carried out by using xanthane hydride (0.01 M in acetonitrile/pyridine 9:1). Phosphordiester linkages are introduced using 0.02 M iodine in THF/Pyridine/water 7:2:1. The rest of the reagents are the ones typically used for oligonucleotide synthesis. For post solid phase synthesis conjugation a commercially available C6 aminolinker phorphoramidite was used in the last cycle of the solid phase synthesis and after deprotection and cleavage from the solid support the aminolinked deprotected oligonucleotide was isolated. The conjugates were introduced via activation of the functional group using standard synthesis methods.

### Purification by RP-HPLC:

The crude compounds were purified by preparative RP-HPLC on a Phenomenex Jupiter C18 10µ 150x10 mm column. 0.1 M ammonium acetate pH 8 and acetonitrile was used as buffers at a flowrate of 5 mL/min. The collected fractions were lyophilized to give the purified compound typically as a white solid.

### Abbreviations:

- DCI:: 4,5-Dicyanoimidazole
- DCM:: Dichloromethane
- DMF:: Dimethylformamide
- DMT:: 4,4'-Dimethoxytrityl
- THF:: Tetrahydrofurane
- Bz:: Benzoyl
- Ibu:: Isobutyryl
- RP-HPLC:: Reverse phase high performance liquid chromatography

The LNA gapmers were:
5'- GₛAₛGₛGₛcₛaₛtₛaₛgₛcₛaₛgₛ^{m}CₛAₛGₛG - 3' (SEQ ID NO 55)
5'- GₛAₛGₛGₛcₛaₛtₛaₛgₛcₛaₛgₛ^{m}CₛAₛGₛG - 3' with a 5' GalNac (Conj1a). (SEQ ID NO 56)

The above gapmer compound (non-conjugate version) has previously been highlighted in the art as particularly effective against HBV (WO2011/47312).

Capital letters are LNA nucleosides, such as beta-D-oxy-LNA, lower case letters are DNA nucleosides, subscript s is a phosphorothioate linkage. LNA cytosines may be 5-methyl cytosine.

An LNA oligomer can be assessed for antiviral effect in vivo in a mouse strain, such as BALB/c or C57B/6, where HBV replication can be established by hydrodynamic tail vein injection of a plasmid, such as pAAV/HBV1.3 , expressing a partially repeated HBV pregenomic RNA (J Virol. 1995 Oct;69(10):6158-69) or pAAV2/HBV1.3, a derivative thereof (WuxiAppTech, Wuxi, China). The injection of the plasmid in a high volume of saline, equal to 8-12% of the body mass of the mouse, will result in the accumulation and expression of the viral pregenomic RNA in the liver, subsequently leading to the establishment of an acute HBV infection. Treament of the animals with an LNA oligomer can be initiated 1 or more days before the injection of the plasmid or 1 day after the injection of the plasmid. Delivery of the LNA oligomer in saline can be done by intravenous or subcutaneous injection.

### Study Set-up

| group | compound | termination time point post dose | group size | dose (d0, d2, d4) mg/kg |
|---|---|---|---|---|
| | Saline | D8 | 6 | none |
| | entecavir | D8 | 6 | , 0.1 mg/kg, p.o., q.d, |
| | SEQ ID 55 | D8 | 6 | 1 x2 mg/kg |
| | SEQ ID 55 | D8 | 6 | 1x10mg/kg |
| | SEQ ID 56 | D8 | 6 | 1x2.7 mg/kg |
| | SEQ ID 56 | D8 | 6 | 1x13.5mg/kg |

An in vivo experiment was performed on SEQ ID 55 and SEQ ID 56, the latter being a GalNAC conjugate of the former (conj1a). The LNA compounds were tested for efficacy against HBV in the mouse hydrodynamic tailvein injection model using the Balb/C mouse strain. LNA compounds were be tested at equimolar doses; 2 and 10 mg/kg, single injection (SEQ ID 55) and at 2.7 and 13.5 mg/kg, single injection (SEQ ID 56). The LNA anti-HBV compounds were compared to saline and to entecavir, an inhibitor of the HBV reverse transcriptase. Entecavir was dosed p. o. daily at 0.1 mg/kg. Where applicable, the animals were treated with the LNA compound 24 hours before the hydrodynamic injection. Entecavir was given in daily doses as described starting 24 hours after the hydrodynamic injection. To initiate HBV replications in vivo, all the animals were injected via tail veins with 20 µg of pAAV2/ HBV plasmid DNA in saline. The injection was done in saline solution equivalent to 8% of a mouse body weight and the total dose was given within 5 seconds. Animals were observed for at least two hours to ensure their recovery from the hydrodynamic shock.

All the animals were monitored on a daily basis for body weight changes and clinical signs for the duration of the in vivo study. On days 1, 3, 5 and 7 post-hydrodynamic injection, blood samples were collected from all animals by submandibular bleeding, 4 hours after the administration of entecavir. On day 7, 4 hours after the last administration of entecavir, all the animals were bled via cardiac puncture upon euthanasia. Their livers were harvested and a sample snap-frozen. All the blood samples were collected in tubes containing heparin sodium

The portion of the left lobe of each liver was used to determine the level of HBV DNA replication by qPCR, as copies per unit weight of liver in which the quantity of total HBV DNA level is subtracted by the quantity of pAAV2/HBV DNA level. The plasma samples collected on day 7 was tested for ALT level, using a sandwich elisa assay (Cusabio; Cat. NO. CSB-E16539m). The LNA conjugate oligomers were well tolerated and no significant elevations of ALT were observed.

Conclusion: SEQ ID 55, which previously had been previously highlighted in the art as particularly effective against HBV (WO2011/47312) was found to have little effect on the replication of HBV in the liver at the dose levels used in this study. In contrast, SEQ ID 56, which consisted of the same oligonucleotide, only with a GalNAC conjugate attached, was seen to have a excellent dose-dependent antiviral effect on the HBV DNA level in the liver (Figure 22), with a 90% reduction in viral titer in the liver 8 days after delivery of a single dose and 7 days after the initiation of the infection. The compounds were well tolerated, with no evidence of dose-dependent increases in ALT levels of either compound. LNA antisense oligonucleotides are know to be efficiently targeted to the liver in the absence of conjugation - the comparative ineefectiveness of the parent compound and the highly potent effect of the conjugated compound is remarkable.

## Claims

1. An LNA antisense oligomer conjugate, comprising an LNA antisense oligomer for use in modulating a nucleic acid and a carbohydrate conjugate moiety which comprises an asialoglycoprotein receptor targeting moiety and a pharmacokinetic modulator selected from the group consisting of C8 - C36 saturated or un-saturated fatty acid, sterol, cholesterol, palmitoyl, hexadec-8-enoyl, oleyl, (9E, 12E)-octadeca-9,12-dienoyl, dioctanoyl, and C16-C20 acyl, wherein the conjugate moiety is covalently bound to the LNA antisense oligomer.

2. The LNA antisense oligomer conjugate according to claim 1, wherein the asialoglycoprotein receptor targeting moiety is selected from the group consisting of galactose, galactosamine, N-formyl-galactosamine, N-acetylgalactosamine, N-propionyl-galactosamine, N-n-butanoyl-galactosamine, and N-isobutanoylgalactose-amine.

3. The LNA antisense conjugate according to claim 1 or 2, wherein the asialoglycoprotein receptor targeting moiety is not Tyr-Glu-Glu-(aminohexyl GalNAc)3 or L3G4 or cholane-based galactose clusters.

4. The LNA antisense oligomer conjugate according to any one of claims 1 - 3, wherein the conjugate moiety comprises a galactose cluster.

5. The LNA antisense oligomer according to claim 4, wherein the galactose cluster consists of an N-acetylgalactosamine trimer.

6. The LNA antisense oligomer conjugate according to any one of claims 1 - 5, wherein the conjugate moiety is covalently linked to the oligomer via a physiologically cleavable linker.

7. The LNA antisense oligomer conjugate according to claim 6, wherein the physiologically cleavable linker is selected from the group consisting of, an acid labile linker, a disulphide linker, a region of phosphodiester linker nucleosides (region B).

8. The LNA antisense oligomer conjugate according to any one of claims 1 - 7, wherein the pharmacokinetic modulator is attached to the carbohydrate conjugate moiety via a linker, such as a physiologically cleavable linker.

9. The LNA antisense oligomer conjugate of any one of claim 1 - 8, where in the oligonucleotide has 7 - 26 such as 7 - 18, 7 - 10, 10 - 16, 12 - 14 contiguous nucleosides.

10. The LNA antisense oligomer conjugate of any one of claims 1 - 9, wherein the LNA antisense oligomer is an LNA gapmer, and LNA mixmer, an LNA totalmer, or an LNA tiny oligomer.

11. The LNA antisense oligomer conjugate of any one of claims 1 - 9, wherein the LNA antisense oligomer has at least 90% phosphorothioate internucleoside linkages.

12. The LNA antisense oligomer conjugate according to any one of claims 1 - 11, wherein the oligomer targets a liver-expressed nucleic acid, such as a RNA, such as a liver-expressed mRNA or microRNA or a viral nucleic acid.

13. The LNA antisense oligomer conjugate according to claim 12, wherein the liver-expressed RNA is a mRNA, selected from the group consisting of: (complement) FactorVII, complement Factor C6, Bcl2, TTR, PCSK9, ApoB, GCGR, CRP, DGAT2, GCCR, PTEN, PTP1B, SGLT2 and SOD1, or a viral RNA such as hepatitis C or hepatitis B.

14. The LNA antisense oligomer conjugate according to claim 12 or 13, wherein the oligomer is a gapmer or a shortmer oligomer.

15. The LNA antisense oligomer conjugate according to claim 12, wherein the oligomer targets a liver-expressed microRNA, such as miR-122.

16. The LNA antisense oligomer conjugate according to claim 12 or 15, wherein the oligomer is between 8 - 18 nucleotides in length.

17. The LNA oligomer conjugate according to any one of the preceding claims which targets a hepatitis B nucleic acid, such as a HBV DNA and/or RNA sequence.

18. The LNA antisense oligomer conjugate according to any one of the preceding claims, for use in medicine.

19. The LNA antisense oligomer conjugate according to any one of claims 1 - 17, for use in down-regulating a liver-expressed RNA.

20. The LNA antisense oligomer conjugate according to any one of claims 1 - 17, for use in treatment of a metabolic disease or disorder, such as a hepatic disease or disorder.

21. The LNA antisense oligomer conjugate according to claim 15 - 17, for use in treatment of hepatitis, such as hepatitis B or C.

22. A pharmaceutical composition comprising the LNA antisense oligomer conjugate according to any one of the proceeding claims, and a pharmaceutically acceptable diluent, carrier, salt or adjuvant.

23. The pharmaceutical composition according to claim 22 wherein the composition comprises a buffered saline solution and the LNA antisense oligomer conjugate.

24. An *in vitro* method of inhibiting the expression of a target gene in a cell, said method comprising administering the LNA antisense oligomer conjugate according to any one of the proceeding claims to a cell which is expressing said target gene, suitably in an amount effective to reduce the expression of the target gene in said cell.

## Patentansprüche

1. LNA-Antisense-Oligomerkonjugat, umfassend ein LNA-Antisense-Oligomer zur Verwendung beim Modulieren einer Nukleinsäure und einer Kohlenhydratkonjugatgruppierung, welche eine Asialoglykoproteinrezeptor-Targetinggruppierung und einen pharmakokinetischen Modulator umfasst, ausgewählt aus der Gruppe bestehend aus einer gesättigten oder ungesättigten C8-C36-Fettsäure, Sterin, Cholesterin, Palmitoyl, Hexadec-8-enoyl, Oleyl, (9E,12E)-Octadeca-9,12-dienoyl, Dioctanoyl und C16-C20-Acyl, wobei die Konjugatgruppierung kovalent an das LNA-Antisense-Oligomer gebunden ist.

2. LNA-Antisense-Oligomerkonjugat nach Anspruch 1, wobei die Asialoglykoproteinrezeptor-Targetinggruppierung aus der Gruppe bestehend aus Galactose, Galactosamin, N-Formylgalactosamin, N-Acetylgalactosamin, N-Propionylgalactosamin, N-n-Butanoylgalactosamin und N-Isobutanoylgalactoseamin ausgewählt ist.

3. LNA-Antisense-Konjugat nach Anspruch 1 oder 2, wobei es sich bei der Asialoglykoproteinrezeptor-Targetinggruppierung nicht um Tyr-Glu-Glu-(Aminohexyl-GalNAc)3 oder L3G4 oder cholanbasierte Galactose-Cluster handelt.

4. LNA-Antisense-Oligomerkonjugat nach einem der Ansprüche 1 bis 3, wobei die Konjugatgruppierung einen Galactose-Cluster umfasst.

5. LNA-Antisense-Oligomer nach Anspruch 4, wobei der Galactose-Cluster aus einem N-Acetylgalactosamintrimer besteht.

6. LNA-Antisense-Oligomerkonjugat nach einem der Ansprüche 1 bis 5, wobei die Konjugatgruppierung über einen physiologisch spaltbaren Linker kovalent mit dem Oligomer verbunden ist.

7. LNA-Antisense-Oligomerkonjugat nach Anspruch 6, wobei der physiologisch spaltbare Linker aus der Gruppe bestehend aus einem säurelabilen Linker, einem Disulfidlinker, einer Region von Phosphodiester-Linkernukleosiden (Region B) ausgewählt ist.

8. LNA-Antisense-Oligomerkonjugat nach einem der Ansprüche 1 bis 7, wobei der pharmakokinetische Modulator über einen Linker, zum Beispiel einen physiologisch spaltbaren Linker, mit der Kohlenhydratkonjugatgruppierung verbunden ist.

9. LNA-Antisense-Oligomerkonjugat nach einem der Ansprüche 1 bis 8, wobei das Oligonukleotid 7 bis 26, beispielsweise 7 bis 18, 7 bis 10, 10 bis 16, 12 bis 14 benachbarte Nukleoside aufweist.

10. LNA-Antisense-Oligomerkonjugat nach einem der Ansprüche 1 bis 9, wobei es sich bei dem LNA-Antisense-Oligomer um ein LNA-Gapmer und ein LNA-Mixmer, ein LNA-Totalmer oder ein sehr kleines LNA-Oligomer handelt.

11. LNA-Antisense-Oligomerkonjugat nach einem der Ansprüche 1 bis 9, wobei das LNA-Antisense-Oligomer mindestens 90 % Phosphorthioat-Internukleosid-Bindungen aufweist.

12. LNA-Antisense-Oligomerkonjugat nach einem der Ansprüche 1 bis 11, wobei das Oligomer gegen eine in der Leber exprimierte Nukleinsäure, zum Beispiel eine RNA, wie etwa eine in der Leber exprimierte mRNA oder MikroRNA oder eine virale Nukleinsäure, gerichtet ist.

13. LNA-Antisense-Oligomerkonjugat nach Anspruch 12, wobei es sich bei der in der Leber exprimierten RNA um eine mRNA handelt, ausgewählt aus der Gruppe bestehend aus: (Komplement-)Faktor VII, Komplement-Faktor C6, Bcl2, TTR, PCSK9, ApoB, GCGR, CRP, DGAT2, GCCR, PTEN, PTP1B, SGLT2 und SOD1, oder um eine virale RNA, wie beispielsweise Hepatitis C oder Hepatitis B.

14. LNA-Antisense-Oligomerkonjugat nach Anspruch 12 oder 13, wobei es sich bei dem Oligomer um ein Gapmer oder ein Shortmer-Oligomer handelt.

15. LNA-Antisense-Oligomerkonjugat nach Anspruch 12, wobei das Oligomer gegen eine in der Leber exprimierte MikroRNA wie etwa miR-122 gerichtet ist.

16. LNA-Antisense-Oligomerkonjugat nach Anspruch 12 oder 15, wobei das Oligomer eine Länge zwischen 8 und 18 Nukleotiden aufweist.

17. LNA-Oligomer-Konjugat nach einem der vorhergehenden Ansprüche, das gegen eine Hepatitis-B-Nukleinsäure, wie beispielsweise eine DNA- und/oder RNA-Sequenz von HBV, gerichtet ist.

18. LNA-Antisense-Oligomerkonjugat nach einem der vorhergehenden Ansprüche zur Verwendung in der Medizin.

19. LNA-Antisense-Oligomerkonjugat nach einem der Ansprüche 1 bis 17 zur Verwendung beim Herunterregulieren einer in der Leber exprimierten RNA.

20. LNA-Antisense-Oligomerkonjugat nach einem der Ansprüche 1 bis 17 zur Verwendung bei der Behandlung einer Stoffwechselkrankheit oder -störung, wie beispielsweise einer Leberkrankheit oder -störung.

21. LNA-Antisense-Oligomerkonjugat nach einem der Ansprüche 15 bis 17 zur Verwendung bei der Behandlung von Hepatitis, wie beispielsweise Hepatitis B oder C.

22. Pharmazeutische Zusammensetzung, umfassend das LNA-Antisense-Oligomerkonjugat nach einem der vorhergehenden Ansprüche und ein pharmazeutisch verträgliches Verdünnungsmittel, einen pharmazeutisch verträglichen Träger, ein pharmazeutisch verträgliches Salz oder Adjuvans.

23. Pharmazeutische Zusammensetzung nach Anspruch 22, wobei die Zusammensetzung eine gepufferte Salzlösung und das LNA-Antisense-Oligomerkonjugat umfasst.

24. *In-vitro-*Verfahren zum Hemmen der Expression eines Zielgens in einer Zelle, wobei das Verfahren das Verabreichen des LNA-Antisense-Oligomerkonjugats nach einem der vorhergehenden Ansprüche an eine Zelle, die das Zielgen exprimiert, in geeigneter Weise in einer Menge umfasst, die wirksam ist, um die Expression des Zielgens in der Zelle zu reduzieren.

## Revendications

1. Conjugué d'oligomère antisens de LNA, comprenant un oligomère antisens de LNA destiné à être utilisé pour moduler un acide nucléique et une fraction conjuguée glucidique qui comprend une fraction ciblant le récepteur de l'asialoglycoprotéine et un modulateur pharmacocinétique choisi dans le groupe constitué par un acide gras saturé ou insaturé en C8 à C36, un stérol, un cholestérol, un palmitoyle, un hexadéc-8-énoyle, un oléyle, un (9E, 12E)-octadéca-9,12-diénoyle, un dioctanoyle et un acyle en C16 à C20, la fraction conjuguée étant liée de manière covalente à l'oligomère antisens de LNA.

2. Conjugué d'oligomère antisens de LNA selon la revendication 1, dans lequel la fraction ciblant le récepteur de l'asialoglycoprotéine est choisie dans le groupe constitué par le galactose, la galactosamine, la N-formyl-galactosamine, la N-acétylgalactosamine, la N-propionyl-galactosamine, la N-n-butanoyl-galactosamine et la N-isobutanoylgalactosamine.

3. Conjugué d'oligomère antisens de LNA selon la revendication 1 ou 2, dans lequel la fraction ciblant le récepteur de l'asialoglycoprotéine n'est pas le Tyr-Glu-Glu-(aminohexyl-GalNAc)3 ou le L3G4 ou des agrégats de galactose à base de cholane.

4. Conjugué d'oligomère antisens de LNA selon l'une quelconque des revendications 1 à 3, dans lequel la fraction conjuguée comprend un agrégat de galactose.

5. Conjugué d'oligomère antisens de LNA selon la revendication 4, dans lequel l'agrégat de galactose est constitué d'un trimère de N-acétylgalactosamine.

6. Conjugué d'oligomère antisens de LNA selon l'une quelconque des revendications 1 à 5, dans lequel la fraction conjuguée est liée de manière covalente à l'oligomère par le biais d'un lieur physiologiquement clivable.

7. Conjugué d'oligomère antisens de LNA selon la revendication 6, dans lequel le lieur physiologiquement clivable est choisi dans le groupe constitué par un lieur labile acide, un lieur disulfure, une région de nucléosides lieurs phosphodiester (région B).

8. Conjugué d'oligomère antisens de LNA selon l'une quelconque des revendications 1 à 7, dans lequel le modulateur pharmacocinétique est attaché à la fraction conjuguée glucidique par le biais d'un lieur, tel qu'un lieur physiologiquement clivable.

9. Conjugué d'oligomère antisens de LNA selon l'une quelconque des revendications 1 à 8, dans lequel l'oligonucléotide comprend de 7 à 26 nucléosides contigus, notamment de 7 à 18, de 7 à 10, de 10 à 16, de 12 à 14 nucléosides contigus.

10. Conjugué d'oligomère antisens de LNA selon l'une quelconque des revendications 1 à 9, dans lequel l'oligomère antisens de LNA est un gapmer de LNA, un mixmer de LNA, un totalmer de LNA ou un minuscule oligomère de LNA.

11. Conjugué d'oligomère antisens de LNA selon l'une quelconque des revendications 1 à 9, dans lequel l'oligomère antisens de LNA comprend au moins 90 % de liaisons internucléosidiques phosphorothioate.

12. Conjugué d'oligomère antisens de LNA selon l'une quelconque des revendications 1 à 11, dans lequel l'oligomère cible un acide nucléique exprimé dans le foie, tel qu'un ARN, comme un ARMm ou un microARN exprimé dans le foie ou un acide nucléique viral.

13. Conjugué d'oligomère antisens de LNA selon la revendication 12, dans lequel l'ARN exprimé dans le foie est un ARMm choisi dans le groupe constitué par : le facteur VII (du complément), le facteur C6 du complément, Bcl2, TTR, PCSK9, ApoB, GCGR, CRP, DGAT2, GCCR, PTEN, PTP1B, SGLT2 et SOD1, ou un ARN viral tel que l'hépatite C ou l'hépatite B.

14. Conjugué d'oligomère antisens de LNA selon la revendication 12 ou 13, dans lequel l'oligomère est un gapmère ou un oligomère shortmère.

15. Conjugué d'oligomère antisens de LNA selon la revendication 12, dans lequel l'oligomère cible un microARN exprimé dans le foie, tel que miR-122.

16. Conjugué d'oligomère antisens de LNA selon la revendication 12 ou 15, dans lequel l'oligomère a une longueur comprise entre 8 et 18 nucléotides.

17. Conjugué d'oligomère de LNA selon l'une quelconque des revendications précédentes, qui cible un acide nucléique de l'hépatite B, tel qu'une séquence d'ADN et/ou d'ARN du VHB.

18. Conjugué d'oligomère antisens de LNA selon l'une quelconque des revendications précédentes, destiné à être utilisé en médecine.

19. Conjugué d'oligomère antisens de LNA selon l'une quelconque des revendications 1 à 17, destiné à être utilisé pour réguler à la baisse un ARN exprimé dans le foie.

20. Conjugué d'oligomère antisens de LNA selon l'une quelconque des revendications 1 à 17, destiné à être utilisé pour traiter une maladie ou un trouble métabolique, tel qu'une maladie ou un trouble hépatique.

21. Conjugué d'oligomère antisens de LNA selon les revendications 15 à 17, destiné à être utilisé pour traiter une hépatite, telle que l'hépatite B ou C.

22. Composition pharmaceutique comprenant le conjugué d'oligomère antisens de LNA selon l'une quelconque des revendications précédentes ainsi qu'un diluant, un excipient, un sel ou un adjuvant pharmaceutiquement acceptable.

23. Composition pharmaceutique selon la revendication 22, la composition comprenant une solution saline tamponnée et le conjugué d'oligomère antisens de LNA.

24. Méthode *in vitro* d'inhibition de l'expression d'un gène cible dans une cellule, ladite méthode comprenant l'administration du conjugué d'oligomère antisens de LNA selon l'une quelconque des revendications précédentes à une cellule qui exprime ledit gène cible, de manière appropriée en une quantité efficace pour réduire l'expression du gène cible dans ladite cellule.
